(19)

Europäisches
Patentamt

European
Patent Office

Office européen
des brevets

(11) **EP 3 733 714 A1**

(12) **EUROPEAN PATENT APPLICATION**

(43) Date of publication:
**04.11.2020 Bulletin 2020/45**

(21) Application number: **19172075.4**

(22) Date of filing: **30.04.2019**

(51) Int Cl.:
**C07K 16/32** (2006.01)    **C07K 16/46** (2006.01)
**A61K 39/00** (2006.01)    **A61P 35/00** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universität Zürich
8006 Zürich (CH)**

(72) Inventors:
• **KAST, Florian
8005 Zürich (CH)**

• **SCHWILL, Martin
8006 Zürich (CH)**
• **HONEGGER, Annemarie
8057 Zürich (CH)**
• **STÜBER, Jakob
81475 München (DE)**
• **TAMASKOVIC, Ratislav
4310 Rheinfelden (CH)**
• **PLÜCKTHUN, Andreas
8006 Zürich (CH)**

(74) Representative: **Schulz Junghans
Patentanwälte PartGmbB
Großbeerenstraße 71
10963 Berlin (DE)**

(54) **HER2-BINDING TETRAMERIC POLYPEPTIDES**

(57) The invention relates to a tetrameric polypeptide comprising a first polypeptide chain comprising a first VL antigen binding domain and a first CL constant domain, a second polypeptide chain comprising a first VH antigen binding domain, a first CH1 constant domain, a first CH2 constant domain and a first CH3 constant domain, a first ligand binding to a HER2 D4 epitope linked to the N-terminus of said first VL antigen binding domain or said first VH antigen binding domain by a first interdomain amino acid linker, a third polypeptide chain comprising a second VL antigen binding domain and a second CL constant domain, a fourth polypeptide chain comprising a second VH antigen binding domain, a second CH1 constant domain, a second CH2 constant domain and a second CH3 constant domain and a third ligand binding to a HER2 D4 epitope linked to the N-terminus of said second VL antigen binding domain or said second VH antigen binding domain by a second interdomain amino acid linker, wherein the VL antigen binding domains and the VH antigen binding domains together constitute a second ligand and a fourth ligand binding to a HER2 D1 epitope. The invention further relates to the tetrameric polypeptide for use in a method for the prevention or treatment of a malignant neoplastic disease, an isolated nucleic acid and a host cell for expression of the polypeptide and a method for obtaining the polypeptide.

Fig. 1

**Description**

The present invention relates to a tetrameric polypeptide having two binding sites to HER2 epitope D1 and two binding sites to HER2 epitope D4.

Background of the Invention

The members of the HER family of receptor tyrosine kinases are important mediators of cell growth, differentiation, migration and survival. The receptor family includes four distinct members including epidermal growth factor receptor (EGFR, ErbB1, or HER1), HER2 (ErbB2 or p185<neu>), HER3 (ErbB3) and HER4 (ErbB4). The members of the EGFR family are closely related single-chain modular glycoproteins with an extracellular ligand binding region, a single transmembrane domain and an intracellular tyrosine kinase, followed by specific phosphorylation sites which are important for the docking of downstream signaling proteins.

An excess of HER2 on the cell surface causes transformation of epithelial cells from multiple tissues. Amplification of the human homolog of the *neu* gene (also known as HER2) is observed in breast and ovarian cancers and correlates with a poor prognosis (US 4,968,603). Overexpression of HER2 has also been observed in other carcinomas including carcinomas of the stomach, endometrium, salivary gland, lung, kidney, colon, thyroid, pancreas and bladder.

While monoclonal antibody therapy directed against HER2 has been shown to provide improved treatment in e.g. metastatic breast cancers that overexpress HER2, there is still considerable room for improvement.

Based on the above-mentioned state of the art, the objective of the present invention is to provide means and methods to provide a HER2-targeting agent which is improved in view of the above-stated disadvantages of the prior art, in particular to provide a HER2-targeting agent displaying improved HER2 signalling inhibition, downregulation of expression, polymerization and clustering, inhibition of receptor diffusion, degradation, and/or inhibition of recycling, in the absence of additional small molecule toxins bound to the HER2 targeting agent. This objective is attained by the subject-matter of the independent claims of the present specification.

Detailed Description of the Invention

The invention relates to a tetrameric polypeptide comprising or consisting of

- a first polypeptide chain comprising in N to C orientation a first VL antigen binding domain and a first CL constant domain,

- a second polypeptide chain comprising in N to C orientation a first VH antigen binding domain, a first CH1 constant domain, a first CH2 constant domain and a first CH3 constant domain,

- a first ligand that specifically binds to a HER2 D4 epitope, wherein the first ligand is comprised in the first polypeptide chain and linked to the N-terminus of the first VL antigen binding domain by a first interdomain amino acid linker, or the first ligand is comprised in the second polypeptide chain and linked to the N-terminus of the first VH antigen binding domain by a first interdomain amino acid linker,

- wherein the first VL antigen binding domain of the first polypeptide chain and the first VH antigen binding domain of the second polypeptide chain together constitute a second ligand, particularly a Fab domain, that specifically binds to a HER2 D1 epitope,

- a third polypeptide chain comprising in N to C orientation a second VL antigen binding domain and a second CL constant domain,

- a fourth polypeptide chain comprising in N to C orientation a second VH antigen binding domain, a second CH1 constant domain, a second CH2 constant domain and a second CH3 constant domain,

- a third ligand that specifically binds to a HER2 D4 epitope, wherein the third ligand is comprised in the third polypeptide chain and linked to the N-terminus of the second VL antigen binding domain by a second interdomain amino acid linker, or the third ligand is comprised in the fourth polypeptide chain and linked to the N-terminus of the second VH antigen binding domain by a second interdomain amino acid linker,

- wherein the second VL antigen binding domain of the third polypeptide chain and the second VH antigen binding domain of the fourth polypeptide chain together constitute a fourth ligand, particularly an Fab domain, that specifically binds to a HER2 D1 epitope.

The tetrameric polypeptide according to the invention thus comprises two times two different HER2-binding paratopes, in other words, is tetravalent. The polypeptide is biparatopic, because it contains binding sites to two distinct HER2 epitopes, namely D1 and D4 on a single molecule.

The VL antigen binding domain and the CL constant domain of the first and third polypeptide chain are domains of an immunoglobulin G light chain, and the VH antigen binding domain and the CH1, CH2 and CH3 constant domains of the second and fourth polypeptide are domains of an immunoglobulin G heavy chain. In other words, the first and the third polypeptide chains each comprise an immunoglobulin G light chain, and the second and the fourth polypeptide chains each comprise an immunoglobulin G heavy chain. The immunoglobulin light and heavy chains of the tetrameric polypeptide according to the invention form the second and fourth ligands specifically binding to HER2 D1 epitope.

In addition, polypeptides comprising a first and third ligand which specifically binds to HER2 D4 epitope are fused to the N-terminus of the immunoglobulin G heavy chains or immunoglobulin G light chains by an interdomain amino acid linker resulting in a tetrameric polypeptide having four HER2 binding sites, two of which bind to the D4 epitope and two of which bind to the D1 epitope.

Surprisingly, the tetrameric polypeptide according to the invention displays superior HER2 inactivation compared to conventional antibodies and other antibody-like molecules, such as divalent biparatopic polypeptides (having a total of two binding regions) and has additional effects on cell growth, apoptosis, HER2 internalization and HER2 degradation. Therefore, the tetrameric polypeptides according to the present invention are promising candidates for therapy of HER2-expressing cancer.

Without wishing to be bound be theory, it is believed that the CH1, CH2, CH3 and CL domains contribute, particularly the CH2 and CH3 domains, to the additional effects of the tetrameric polypeptides of the invention, particularly inhibition of cell growth and proliferation, induction of apoptosis and other forms of cell death, HER2 internalization, HER2 recycling inhibition and HER2 degradation, HER2 crosslinking, reduction of HER2 surface mobility, HER2 expression downregulation, inhibition of HER2 dimerization and signalling by positioning the variable domains at a particular angle and distance.

In certain embodiments, the first polypeptide chain and the third polypeptide chain, particularly the immunoglobulin light chain of the first polypeptide chain and the immunoglobulin light chain of the third polypeptide chain, are characterized by a sequence identity with each other of 70% or more, particularly 80% or more, more particularly 90% or more, even more particularly 95% or more, wherein most particularly the first polypeptide chain and the third polypeptide chain are identical. This means that the first polypeptide chain is characterized by a sequence identity with the third polypeptide chain in the above-stated range.

In certain embodiments, the second polypeptide chain and the fourth polypeptide chain, particularly the immunoglobulin heavy chain of the second polypeptide chain and the immunoglobulin heavy chain of the fourth polypeptide chain, are characterized by a sequence identity with each other of 70% or more, particularly 80% or more, more particularly 90% or more, even more particularly 95% or more, wherein most particularly the second polypeptide chain and the fourth polypeptide chain are identical. This means that the second polypeptide chain is characterized by a sequence identity with the fourth polypeptide chain in the above-stated range.

In certain embodiments, the first ligand and the third ligand are characterized by a sequence identity with each other of 70% or more, particularly 80% or more, more particularly 90% or more, even more particularly 95% or more, wherein most particularly the first ligand and the third ligand are identical. In other words, the first ligand is substantially the same as the third ligand. This means that the first ligand is characterized by a sequence identity with the third ligand in the above-stated range.

In certain embodiments, the second ligand and the fourth ligand comprise a sequence identity with each other of 70% or more, particularly 80% or more, more particularly 90% or more, even more particularly 95% or more, wherein most particularly the first ligand and the third ligand are identical. In other words, the second ligand is substantially the same as the fourth ligand. This means that the second ligand is characterized by a sequence identity with the fourth ligand in the above-stated range.

In certain embodiments, the first CH2 constant domain and the first CH3 constant domain of the second polypeptide chain interact with, particularly are covalently linked to, the second CH2 constant domain and the second CH3 constant domain of the fourth polypeptide chain, such that a tetrameric polypeptide is formed. In particular, the CH2 and CH3 constant domains of the second and fourth polypeptide chain dimerize. In particular, combined with the interaction between the first and the second polypeptide chains and the third and the fourth polypeptide chains, particularly by means of the dimerization between the respective VL and VH antigen binding domains and the CL and CH1 constant domains, this results in tetramer formation of the first, second, third and fourth polypeptide. Therefore, in respect of multimer formation, the tetrameric polypeptide of the present invention is similar to an antibody.

In certain embodiments, the second polypeptide chain comprises a first hinge region between the first CH1 constant domain and the first CH2 constant domain, and the fourth polypeptide chain comprises a second hinge region between the second CH1 constant domain and the second CH2 constant domain, wherein the first hinge region and the second hinge region mediate complex formation between the second polypeptide chain and the fourth polypeptide chain, particularly by at least one disulphide bond, more particularly by a first disulphide bond and a second disulphide bond, such that a tetrameric polypeptide is formed. Complex formation between the CH1 and CH2 constant domains may thus occur by the hinge region, i.e. by disulphide bond formation between cysteine residues, just as in antibodies.

In certain embodiments, the CH2 constant domain and/or the CH3 constant domain of the second polypeptide chain is truncated at its C-terminus.

In certain embodiments, the CH2 constant domain and/or the CH3 constant domain of the fourth polypeptide chain is truncated at its C-terminus.

In certain embodiments, the first ligand comprises or consists of a single-chain variable fragment polypeptide chain comprising an scFv heavy chain, an scFv linker chain, and an scFv light chain. In certain embodiments, the scFv heavy chain is the VH domain of 4D5 (particularly SEQ ID No. 80), wherein the scFv light chain is the VL domain of 4D5 (particularly SEQ ID No. 81).

In certain embodiments, the third ligand comprises or consists of a single-chain variable fragment polypeptide chain comprising in N to C orientation an scFv heavy chain, an scFv linker chain, and an scFv light chain. In certain embodiments, the scFv heavy chain is the VH domain of 4D5 (particularly SEQ ID No. 80), and wherein the scFv light chain is the VL domain of 4D5 (particularly SEQ ID No. 81).

In certain embodiments, the single-chain variable fragment polypeptide chain of the first or third ligand comprises in N to C orientation an scFv heavy chain, an scFv linker chain, and an scFv light chain. In certain embodiments, the single-chain variable fragment polypeptide chain comprises in N to C orientation an scFv light chain, an scFv linker chain, and an scFv heavy chain. In other words, the the scFv heavy chain and the scFv light chain may be provided in any orientation on the single-chain variable fragment polypeptide chain.

In certain embodiments,

- the scFv heavy chain of the first ligand and/or the third ligand comprises or consists of a peptide sequence characterized by a sequence identity of 70 % or more, particularly 80 % or more, more particularly 90 % or more, even more particularly 95 % or more, with a peptide sequence selected from SEQ ID No. 15, SEQ ID No. 21, SEQ ID No. 22, SEQ ID No. 23, SEQ ID No. 44, SEQ ID No. 53, SEQ ID No. 54 and SEQ ID No. 80, wherein most particularly the scFv heavy chain of the first ligand comprises or consists of a peptide sequence identical to a peptide sequence selected from SEQ ID No. 15, SEQ ID No. 21, SEQ ID No. 22, SEQ ID No. 23, SEQ ID No. 44, SEQ ID No. 53, SEQ ID No. 54 and SEQ ID No. 80, and

- the scFv light chain of the first ligand and/or the third ligand comprises or consists of a peptide sequence characterized by a sequence identity of 70 % or more, particularly 80 % or more, more particularly 90 % or more, even more particularly 95 % or more, with a peptide sequence selected from SEQ ID No. 14, SEQ ID No. 24, SEQ ID No. 25, SEQ ID No. 26, SEQ ID No. 43 and SEQ ID No. 81, wherein most particularly the scFv light chain of the first ligand comprises or consists of a peptide sequence identical to a peptide sequence selected from SEQ ID No. 14, SEQ ID No. 24, SEQ ID No. 25, SEQ ID No. 26, SEQ ID No. 43 and SEQ ID No. 81.

That is the scFv light and heavy chains may completely consist of the above-defined peptide sequence or the scFv light and heavy chains may comprise the above-defined peptide sequence, wherein the scFv light and heavy chains may contain additional peptide sequences.

In certain embodiments, the scFv light chain of the first ligand comprises or consists of the VL antigen binding domain of antibody 4D5 and the scFv heavy chain of the first ligand comprises or consists of the VH antigen binding domain of antibody 4D5.

In certain embodiments, the scFv light chain of the third ligand comprises or consists of the VL antigen binding domain of antibody 4D5 and the scFv heavy chain of the third ligand comprises or consists of the VH antigen binding domain of antibody 4D5.

In the context of the present specification, the term 4D5 refers to the humanized monoclonal antibody trastuzumab, also known as Herceptin, and also referred to herein as "TZB" which is directed against the membrane-proximal domain IV of HER2 (Cho et al., 2003).

In certain embodiments, the scFv linker chain comprises a peptide sequence characterized by a sequence identity of 70 % or more, particularly 80 % or more, more particularly 90 % or more, even more particularly 95 % or more, with SEQ ID No. 16, wherein most particularly the scFv linker chain comprises a peptide sequence identical to SEQ ID No. 16.

In certain embodiments,

- the first polypeptide chain and/or the third polypeptide chain comprises a peptide sequence characterized by a sequence identity of 70 % or more, particularly 80 % or more, more particularly 90 % or more, even more particularly 95 % or more, with a peptide sequence selected from SEQ ID No. 18, SEQ ID No. 30, SEQ ID No. 31, SEQ ID No. 32, SEQ ID No. 39, SEQ ID No. 41, SEQ ID No. 50 and SEQ ID 76, wherein most particularly the first polypeptide chain comprises a peptide sequence identical to a peptide sequence selected from SEQ ID No. 18, SEQ ID No. 30, SEQ ID No. 31, SEQ ID No. 32, SEQ ID No. 39, SEQ ID No. 41, SEQ ID No. 50 and SEQ ID 76, and

- the second polypeptide chain and/or the fourth polypeptide chain comprises a peptide sequence characterized by a sequence identity of 70 % or more, particularly 80 % or more, more particularly 90 % or more, even more particularly 95 % or more, with a peptide sequence selected from SEQ ID No. 19, SEQ ID No. 20, SEQ ID No. 27, SEQ ID No. 28, SEQ ID No. 29, SEQ ID No. 40, SEQ ID No. 42, SEQ ID No. 51, SEQ ID No. 52 and SEQ ID 77, wherein most particularly the second polypeptide chain comprises a peptide sequence identical to a peptide sequence selected from SEQ ID No. 19, SEQ ID No. 20, SEQ ID No. 27, SEQ ID No. 28, SEQ ID No. 29, SEQ ID No. 40, SEQ ID No. 42, SEQ ID No. 51, SEQ ID No. 52 and SEQ ID 77.

Therefore, the VL and VH antigen binding domain of the first, second, third and fourth polypeptide chains may be substantially identical to the VL and VH antigen binding domains of the scFv fragment termed A21 (Hu S. et al., 2008, Proteins, 70, 938-949) which specifically binds to domain I of HER2.
In certain embodiments,

- the first polypeptide chain and/or the third polypeptide chain comprises a peptide sequence characterized by a sequence identity of 70 % or more, particularly 80 % or more, more particularly 90 % or more, even more particularly 95 % or more, with SEQ ID No. 36, SEQ ID No. 37, SEQ ID No. 38 and SEQ ID No. 78, wherein most particularly the first polypeptide chain comprises a peptide sequence identical to SEQ ID No. 36, SEQ ID No. 37, SEQ ID No. 38 and SEQ ID No. 78, and

- the second polypeptide chain and/or the fourth polypeptide chain comprises a peptide sequence characterized by a sequence identity of 70 % or more, particularly 80 % or more, more particularly 90 % or more, even more particularly 95 % or more, with SEQ ID No. 33, SEQ ID No. 34, SEQ ID No. 35 and SEQ ID No. 79, wherein most particularly the second polypeptide chain comprises a peptide sequence identical to SEQ ID No. 33, SEQ ID No. 34, SEQ ID No. 35 and SEQ ID No. 79.

Therefore, the immunoglobulin light and heavy chains of the first, second, third and fourth polypeptide chains may comprise IgG domains (VL, CL, VH, CH1, CH2 and/or CH3) substantially identical to the corresponding domains of the ErbB2 antibody termed 7C2 (US 7,371,376) which specifically binds to domain I of HER2.
In certain particular embodiments, the tetrameric polypeptide according to the invention is essentially an immunoglobulin G type antibody (particularly a human or humanized monoclonal IgG antibody) having two identical heavy chains and two identical light chains, wherein the antigen specific variable heavy and light chains together form a ligand (the second and fourth ligand) specifically reactive to the D1 domain of Her2, and each of the light chains, or each of the heavy chains, contain an N-terminally linked polypeptide comprising an scFv polypeptide chain constituted of a heavy and light variable region linked by an scFv linker chain, and the scFv polypeptide chain is linked to the N terminus of the immunoglobulin heavy or light chain via an interdomain amino acid linker consisting of 1 to 20 amino acids.
In certain embodiments,

- the first polypeptide chain and/or the third polypeptide chain is characterized by a sequence identity of 70 % or more, particularly 80 % or more, more particularly 90 % or more, even more particularly 95 % or more, with SEQ ID No. 1, wherein most particularly the first polypeptide chain is identical to SEQ ID No. 1, and

- the second polypeptide chain and/or the fourth polypeptide chain is characterized by a sequence identity of 70 % or more, particularly 80 % or more, more particularly 90 % or more, even more particularly 95 % or more, with SEQ ID No. 2, wherein most particularly the second polypeptide chain is identical to SEQ ID No. 2.

The resulting tetrameric polypeptide is referred to as "441" (for identity). In this construct, IgG light chains (of the first and third polypeptide chain) comprising the VL antigen binding domain of antibody A21 are N-terminally fused to an scFv fragment comprising the VL and VH antigen binding domains of 4D5 (trastuzumab or HERCEPTIN, HER2 D4 binder) and combined with IgG heavy chains (the second and fourth polypeptide chains) comprising the VH antigen binding domain of antibody A21. The VL and VH antigen binding domains of A21 together constitute a HER2 D1 binder).
In certain embodiments,

- the first polypeptide chain and/or the third polypeptide chain is characterized by a sequence identity of 70 % or more, particularly 80 % or more, more particularly 90 % or more, even more particularly 95 % or more, with SEQ ID No. 3, wherein most particularly the first polypeptide chain is identical to SEQ ID No. 3, and

- the second polypeptide chain and/or the fourth polypeptide chain is characterized by a sequence identity of 70 % or more, particularly 80 % or more, more particularly 90 % or more, even more particularly 95 % or more, with SEQ ID No. 4, wherein most particularly the second polypeptide chain is identical to SEQ ID No. 4.

The resulting tetrameric polypeptide is referred to as "47C2" (for identity). In this construct, IgG light chains (of the first and third polypeptide chain) comprising the VL antigen binding domain of antibody 7C2 are N-terminally fused to an scFv fragment comprising the VL and VH antigen binding domains of 4D5 (trastuzumab or HERCEPTIN, HER2 D4 binder) and combined with IgG heavy chains (the second and fourth polypeptide chains) comprising the VH antigen binding domain of antibody 7C2. The VL and VH antigen binding domains of 7C2 together constitute a HER2 D1 binder).
In certain embodiments,

- the first polypeptide chain and/or the third polypeptide chain is characterized by a sequence identity of 70 % or more, particularly 80 % or more, more particularly 90 % or more, even more particularly 95 % or more, with SEQ ID No. 11, wherein most particularly the first polypeptide chain is identical to SEQ ID No. 11, and

- the second polypeptide chain and/or the fourth polypeptide chain is characterized by a sequence identity of 70 % or more, particularly 80 % or more, more particularly 90 % or more, even more particularly 95 % or more, with SEQ ID No. 12, wherein most particularly the second polypeptide chain is identical to SEQ ID No. 12.

The resulting tetrameric polypeptide is referred to as "241" (for identity). In this construct, IgG heavy chains (of the second and fourth polypeptide chain) comprising the VH antigen binding domain of antibody A21 are N-terminally fused to an scFv fragment comprising the VL and VH antigen binding domains of 4D5 (trastuzumab or HERCEPTIN, HER2 D4 binder) and combined with IgG light chains (the first and third polypeptide chains) comprising the VL antigen binding domain of antibody A21. The VL and VH antigen binding domains of A21 together constitute a HER2 D1 binder).
In certain embodiments, the VH antigen binding domain is selected from the VH antigen binding domain of A21 (particularly SEQ ID No. 40, 42, 51, 52 or 77) and the VH antigen binding domain of 7C2 (particularly SEQ ID No. 79), and wherein the VL antigen binding domain is selected from the VL antigen binding domain of A21 (particularly SEQ ID No. 39, 41, 50 or 76) and the VL antigen binding domain of 7C2 (particularly SEQ ID No. 78).
In certain embodiments, the first and third polypeptide chains are identical to SEQ ID No. 1 or a functional equivalent peptide sequence characterized by a sequence identity of at least 70%, 75%, 80%, 85%, 90%, 95%, 96%, 97%, 98% or 99% to SEQ ID No. 1, and the second and fourth polypeptide chains are identical to SEQ ID No. 2 or a functional equivalent peptide sequence characterized by a sequence identity of at least 70%, 75%, 80%, 85%, 90%, 95%, 96%, 97%, 98% or 99% (construct 441 in case of sequence identity) to SEQ ID No. 2.
In certain embodiments, the first and third polypeptide chains are identical to SEQ ID No. 3 or a functional equivalent peptide sequence characterized by a sequence identity of at least 70%, 75%, 80%, 85%, 90%, 95%, 96%, 97%, 98% or 99% to SEQ ID No. 3, and the second and fourth polypeptide chains are identical to SEQ ID No. 4 or a functional equivalent peptide sequence characterized by a sequence identity of at least 70%, 75%, 80%, 85%, 90%, 95%, 96%, 97%, 98% or 99% (construct 47C2 in case of sequence identity) to SEQ ID No. 4.
In certain embodiments, the first and third polypeptide chains are identical to SEQ ID No. 11 or a functional equivalent peptide sequence characterized by a sequence identity of at least 70%, 75%, 80%, 85%, 90%, 95%, 96%, 97%, 98% or 99% to SEQ ID No. 11, and the second and fourth polypeptide chains are identical to SEQ ID No. 12 or a functional equivalent peptide sequence characterized by a sequence identity of at least 70%, 75%, 80%, 85%, 90%, 95%, 96%, 97%, 98% or 99% (construct 241 in case of sequence identity) to SEQ ID No. 12.
The interdomain amino acid linker is not restricted in amino acid composition, but amino acids shown to contribute to linker flexibility are chosen in particular embodiments contemplated herein. The inventors have shown linkers to work that consist of G, S and/or T residues, for example repeats of $(GG_mS)$ and $(GG_mT)$ with m selected from 1 to 3, and the entire linker length not exceeding 20, 25, or even 30. Interdomain linkers as short as one or two amino acids have been shown to work. The first and the third polypeptide may comprise the same interdomain amino acid linker or different interdomain amino acid linkers.
In certain embodiments, the interdomain amino acid linker consists of 1, 2, 3, 4, 5, 6, 7, 8, 9, 10, 11, 12, 13, 14, 15, 16, 17, 18, 19, 20, 21, 22, 23, 24 or 25 amino acids.
With regard to the length and sequence composition of the interdomain amino acid linker, the inventors' results indicate that any linker having an equivalent length of a maximum of 25 amino acids that is not expected, because of structure prediction, to interfere with the solubility of the resulting protein, is expected to function.

The polypeptide according to any one of the preceding claims, wherein the interdomain amino acid linker comprises or consists of amino acids G, A, J, S, T, P, C, V, M and E, particularly wherein the interdomain amino acid linker comprises or consists of amino acids G, S, A and T.

In particular embodiments, the interdomain amino acid linker is $(GG\Sigma)_n$ with n being an integer and $n \geq 4$ (particularly n is 4, 5, 6, 7 or 8), and with $\Sigma$ selected from S and T.

In particular embodiments, the interdomain amino acid linker is $(GGS)_n$ with n being an integer and $n \geq 4$ (particularly n is 4, 5, 6, 7 or 8).

In particular embodiments, the interdomain amino acid linker is $(GGT)_n$ with n being an integer and $n \geq 4$ (particularly n is 4, 5, 6, 7 or 8).

In particular embodiments, the interdomain amino acid linker is $(G\Sigma G)_n$ with n being an integer and $n \geq 4$ (particularly n is 4, 5, 6, 7 or 8), and with $\Sigma$ selected from S and T.

In particular embodiments, the interdomain amino acid linker is $(\Gamma\Gamma\Sigma)_n$ with n being an integer and $n \geq 4$ (particularly n is 4, 5, 6, 7 or 8), and with each $\Gamma$ independently from any other $\Gamma$ being selected being from A, G and V, and $\Sigma$ being selected from S and T.

Important considerations at the time of choosing the linker sequence have been solubility and flexibility. The skilled person will readily be able to vary this sequence in composition and length based on the teaching herein and the knowledge available on linker design, as exemplified by Chen et al., 2013, Advanced Drug Delivery Reviews, 65, 1357-1369 and Evers et al., 2006, Biochemistry, 45, 13183-13192.

In certain embodiments, the interdomain amino acid linker is characterized by an amino acid sequence $(GGGGS)_n$, with n being 1, 2, 3, 4 or 5.

In certain embodiments, the interdomain amino acid linker comprises or is a sequence characterized by one of SEQ ID No. 17, SEQ ID No. 55 to SQ ID No. 69 and SEQ ID No. 82 to SEQ ID 91.

In certain embodiments, the interdomain amino acid linker comprises or consists of a peptide sequence selected from one of SEQ ID No. 17, SEQ ID No. 55 to SEQ ID No. 69 and SEQ ID No. 82 to SEQ ID No. 91 or a functional equivalent peptide sequence characterized by a sequence identity of at least 70% to a peptide sequence selected from one of SEQ ID No. 17, SEQ ID No. 55 to SEQ ID No. 69 and SEQ ID No. 82 to SEQ ID No. 91.

A second aspect of the invention relates to the polypeptide according to the first aspect for use in a method for the prevention or treatment of a malignant neoplastic disease associated with expression of HER2 (a HER2-positive cancer).

A third aspect of the invention relates to an isolated nucleic acid encoding at least one of the first polypeptide chain, the second polypeptide chain, the third polypeptide chain and the fourth polypeptide chain of the tetrameric polypeptide according to the first aspect of the invention. In particular, the isolated nucleic acid may be comprised in a plasmid for expression in a bacterial or a eukaryotic host cell. The nucleic acid sequences encoding the first, the second, the third and the fourth polypeptide may be provided on the same plasmid or on separate plasmids, i. e for co-expression in the same host.

A fourth aspect of the invention relates to a host cell which is adapted to produce at least one of the first polypeptide chain, the second polypeptide chain, the third polypeptide chain and the fourth polypeptide chain of the tetrameric polypeptide according to the first aspect of the invention. In particular, the host cell is a bacterial cell or a eukaryotic cell. More particularly, the host cell is a Chinese Hamster Ovary (CHO) cell.

In certain embodiments, the host cell comprises the isolated nucleic acid according to the third aspect of the invention, such that the host cell is able to produce at least one of the first polypeptide chain, the second polypeptide chain, the third polypeptide chain and the fourth polypeptide chain of the polypeptide according to the first aspect of the invention. In particular, the first, second, third and fourth polypeptide may be co-produced in the same cell or produced separately and combined in vitro.

A fifth aspect of the invention relates to a method for obtaining the polypeptide according to the first aspect of the invention, wherein the method comprises culturing the host cell according to the fourth aspect of the invention, so that at least one of the first polypeptide chain, the second polypeptide chain, the third polypeptide chain and the fourth polypeptide chain of the polypeptide according to the first aspect of the invention is produced.

Wherever alternatives for single separable features such as, for example, an isotype protein or coding sequence, ligand type or medical indication are laid out herein as "embodiments", it is to be understood that such alternatives may be combined freely to form discrete embodiments of the invention disclosed herein. Thus, any of the alternative embodiments for a detectable label may be combined with any of the alternative embodiments of ligand and these combinations may be combined with any medical indication or diagnostic method mentioned herein.

The invention is further illustrated by the following examples and figures, from which further embodiments and advantages can be drawn. These examples are meant to illustrate the invention but not to limit its scope.

*Description of the Figures*

Fig. 1      shows schemes of biparatopic anti-HER2 binding agents.

Fig. 2      shows further schemes of biparatopic anti-HER2 binding agents

Fig. 3      shows a vector map of a plasmid for co-expression of the light and heavy chain of construct 441 in CHO cells (Pymex10 based vector with double expression cassette [CMV GOI polyA]).

Fig. 4      shows a vector map of a plasmid for co-expression of the light and heavy chain of construct 47C2 in CHO cells (Pymex10 based vector with double expression cassette [CMV GOI polyA]).

Fig. 5      shows a vector map of a plasmid for expression of construct 841 in CHO cells.

Fig. 6      shows an elution profile of construct 441 from Protein A affinity chromatography.

Fig. 7      shows an elution profile of construct 441 from cation exchange chromatography.

Fig. 8      shows an elution profile of construct 441 from size exclusion chromatography.

Fig. 9      shows a Coumassie-stained SDS-PAGE gel of fractions from purification of construct 441.

Fig. 10      shows the viability testing of CHOs during the expression of construct 441 (scFV-IgG). Expression optimization of construct 441 in CHOs cells for indicated time. Cells were cultured in CHOgro medium from MIrus (MIR 6260) and additionally fed with free cysteine (reduced form) (2), glutathione (3), fetal calf serum (4) or all additives respectively (5). CHO cells were analyzed on CASY cell counter (Schärfe System).

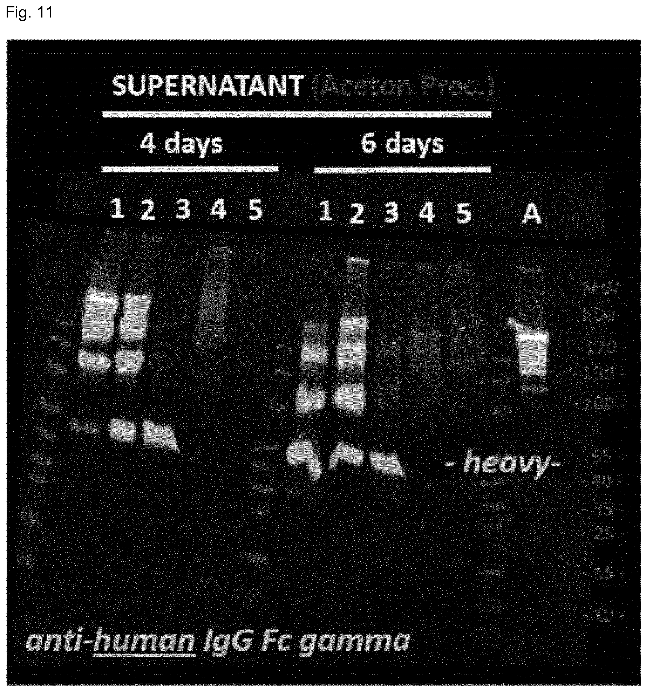

Fig. 11      shows a Western blot of construct 441 expression, secreted to the medium of CHO cells after indicated times. Cells were cultured in CHOgro medium from MIrus (MIR 6260) (1) and additionally fed with free cysteine (reduced form) (2), glutathione (3), fetal calf serum (4) or all additives together (5), respectively. Protein was precipitated from medium by acetone precipitation and re-solubilized in SDS PAGE buffer. Proteins were resolved on 4-12 % gradient gel and the western blot was analyzed on an Odyssey system (LI-COR). Purified intact full length construct 441 is shown as control (A) and runs above the 170 kDa marker. Molecular weight marker Page ruler from Thermo Scientific is shown in red.

Fig. 12      shows cell proliferation assays (XTT) with BT474 cells after 4 days of treatment. Trastuzumab (TZB), biparatopic DARPin (6L1G) and different fusion variants of the biparatopic construct. LF IgG HL (murine parent of construct 441), HF IgG HL (murine parent of construct 241) show similar anti-proliferative activity compared to the biparatopic DARPin 6L1G, which is superior to trastuzumab (TZB). HF IgG LH (murine variant, no seq.) and LF IgG LH (murine variant, no seq.) show reduced anti-proliferative activity compared to biparatopic DARPin and higher $IC_{50}$ concentrations.

Fig. 13      shows cell proliferation assays (XTT) with BT474 cells after 4 days of treatment to test the effect of the linker length. Biparatopic DARPin (6L1G) and different fusion linker variants of the biparatopic construct (murine parent construct of 441) are compared. The 2-AA linker (GS) shows highest anti-proliferative activity. The 4-, 7- and 12-AA linkers show similar activity. The 22-AA linker variant shows reduced activity.

Fig. 14      shows cell proliferation assays (XTT) with BT474 cells after 4 days of treatment. Biparatopic DARPin (6G; 6L1G), biparatopic construct 441 (441), biparatopic construct 411 (humanized kappa1 VH1) and biparatopic construct 443 (humanized kappa4 VH3). All show similar plateau levels of anti-proliferative activity, except 443, which shows reduced activity.

Fig. 15      shows cell proliferation assays (XTT) with BT474 cells after 4 days of treatment with different humanized versions of A21 IgG, when fused to TZB scFv. The strategy of humanization is described above. Different variants use humanized kappa1 VH3 or a humanized kappa1 VH core graft.

Fig. 16    shows XTT cell proliferation assay with BT474 cells after 4 day of treatment. Tetravalent IgG (HF IgG HL and LF IgG HL murine) versus bivalent Fab fusions (HF Fab HL and LF Fab HL murine). All constructs show similar plateau and IC50 values.

Fig. 17    shows XTT cell proliferation assay with SKBR3 cells after 4 day of treatment. Biparatopic DARPin (6G) biparatopic construct (441 tf), trastuzumab (TZB).

Fig. 18    shows cell proliferation assays (XTT) with CALU-3 cells after 4 days of treatment. Biparatopic DARPin (6G), biparatopic construct (construct 441 (441tf), trastuzumab (TZB).

Fig. 19    shows cell proliferation assays (XTT) with BT474 cells after 4 days of treatment, testing effect of domain 1 binding unit. Biparatopic construct with A21 (construct 441tf) or 7C2 fusions show different IC50 and plateau level.

Fig. 20    shows cell proliferation assays (XTT) with BT474 cells after 4 days of treatment, testing the effect of domain 1 binding unit. Biparatopic construct with A21 (construct 441) or with 39S (39s HF IgG H)L

Fig. 21    shows XTT cell proliferation assays with HCC1419 cells after 4 days of treatment. Biparatopic DARPin (6G; 6L1G), biparatopic construct 441 (441tf) and bivalent LF-oaFabFc (A21-TZB-4oa). 441 and 6G show similar inhibition of cell proliferation after 4 days. LF-oaFabFc show slightly reduced inhibition of cell proliferation compared to 441.

Fig. 22    shows XTT cell proliferation assay with BT474 and HCC1419 cells after 4 day of treatment. All human.

Fig. 23    shows XTT cell proliferation assay with BT474 and HCC1419 cells after 4 day of treatment. All human.

Fig. 24    shows a) in the upper panel XTT cell proliferation assays with BT474 (left) and HCC1419 (right) cells after 4 day of treatment; and in the lower panel XTT cell proliferation assays with BT474 (left) and HCC1419 (right) cells after 4 day of treatment (variants with higher affinity (NGS and GGG)); b) repeated experiments with a new expression of NGS.

Fig. 25    shows XTT cell proliferation assays with BT474 (left) and HCC1419 (right) cells after 4 day of treatment.

Fig. 26    shows XTT cell proliferation assays with HCC1419 cells grown as 3D spheroids.

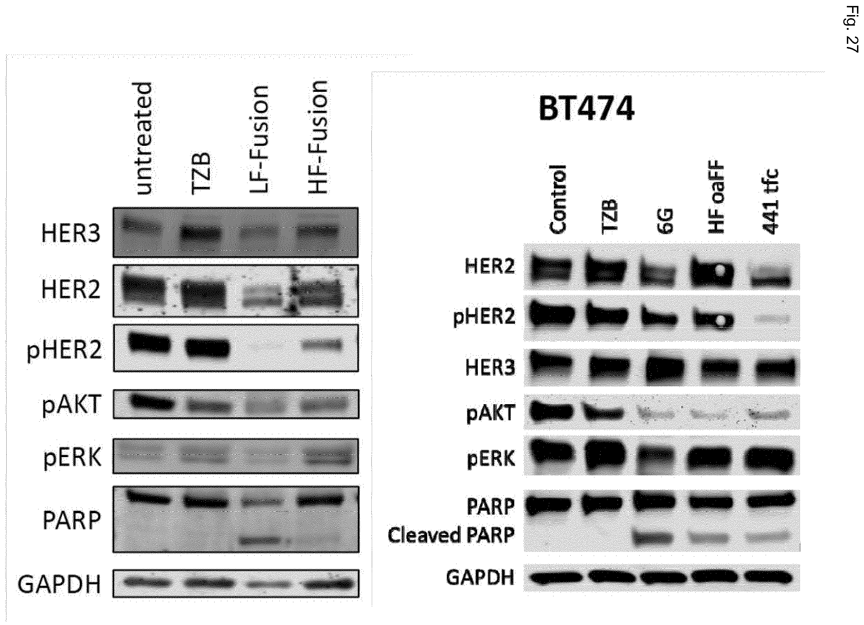

Fig. 27    shows Western Blots 24 hours post treatment (BT474) with indicated agents (murine).

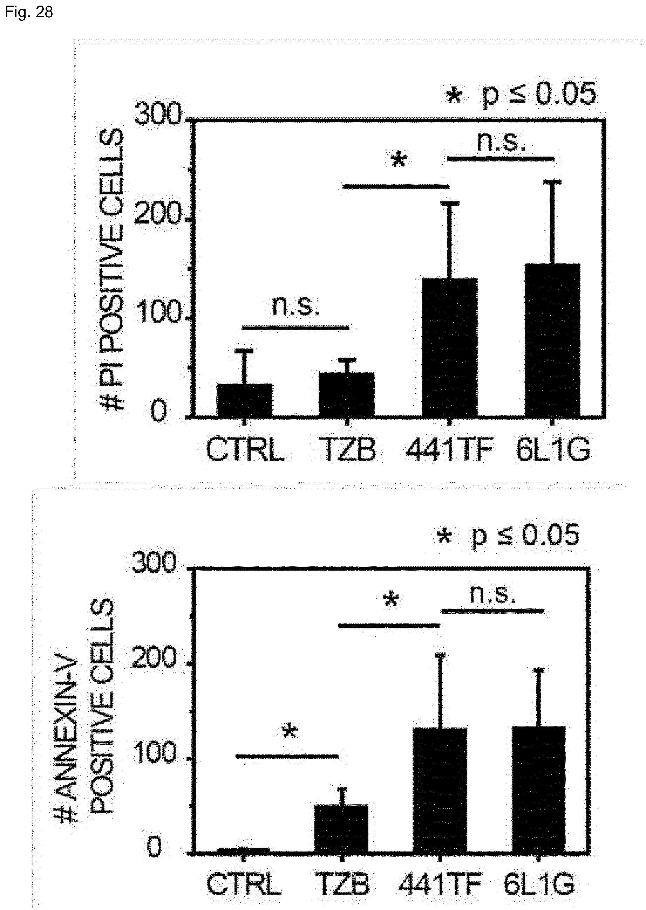

Fig. 28    shows in the upper panel Induction of apoptosis in BT474 cells after 3 days of treatment. Average number of propidium iodide (PI) positive cells was determined for 4 replicates, counted by cell profiler and was analyzed with Student's t-test. Biparatopic construct (441, 441tf) induced significantly more cell death than trastuzumab (TZB). 441 and biparatopic DARPin (6L1G) show similar level of cell death; and in the lower panel Induction of apoptosis in BT474 cells after 3 days of treatment. Average number of annexin-V positive cells was determined for 3-4 replicates, counted by cell profiler and was analyzed with Student's t-test. Biparatopic construct 441 induced significantly more apoptosis than trastuzumab (TZB). Construct 441 and 6L1G show similar level of apoptosis.

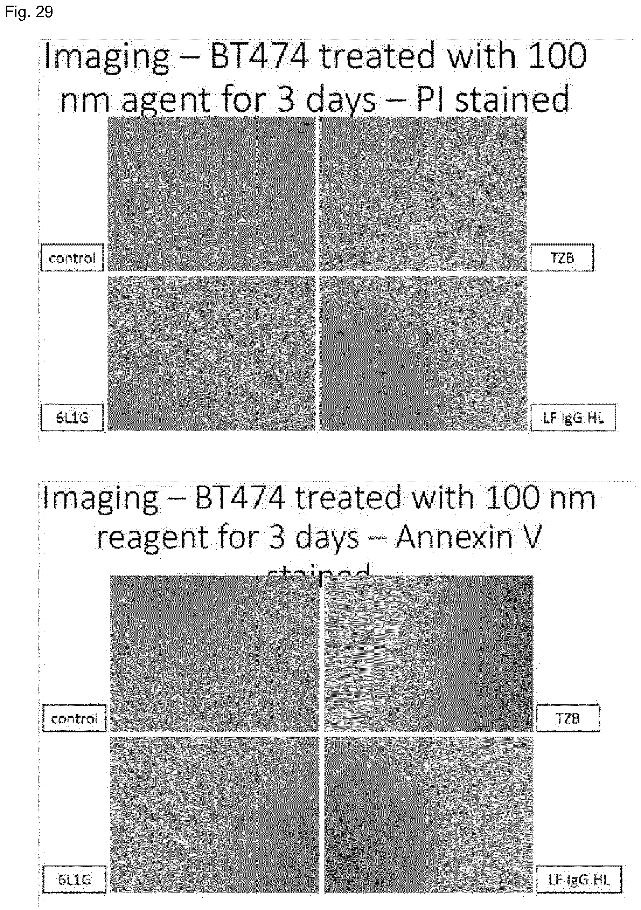

Fig. 29    shows images of BT474 cells treated with the indicated agents for 3 days.

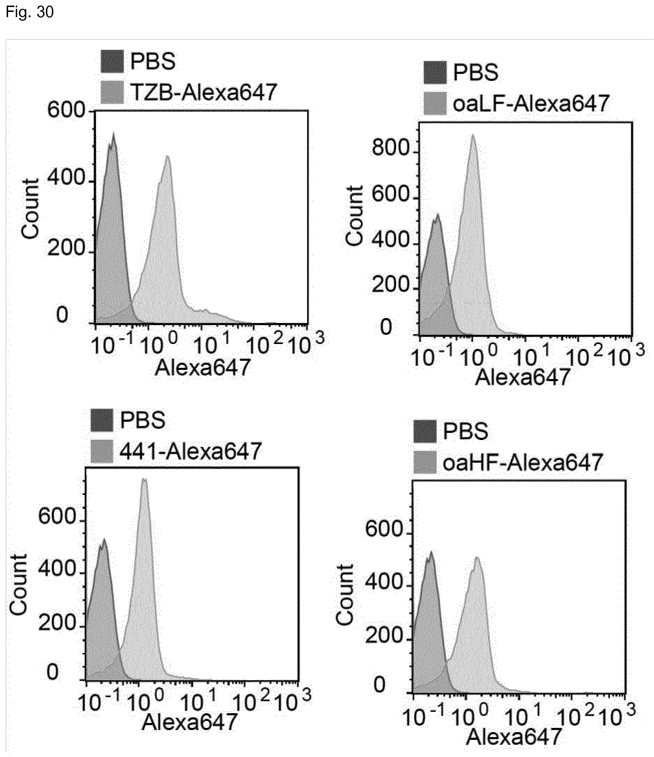

Fig. 30    shows Alexa647-labeled trastuzumab (TZB), biparatopic construct 441 and biparatopic one armed constructs oaLF and oaHF were incubated for 1 h at 100 nM concentration with 3 million BT474 cells in 3 ml PBS containing NaN$_3$ (0.1%) and BSA (1 %) at 4°C. Note that BT474 cells were pre-treated with 0.1 % NaN$_3$ in PBS with 1% BSA to block internalization before binding. Cells were analyzed afterwards on CyFlow Space instrument (Partec). All binding agents show specific binding to the surface of HER2-positive BT474 cells.

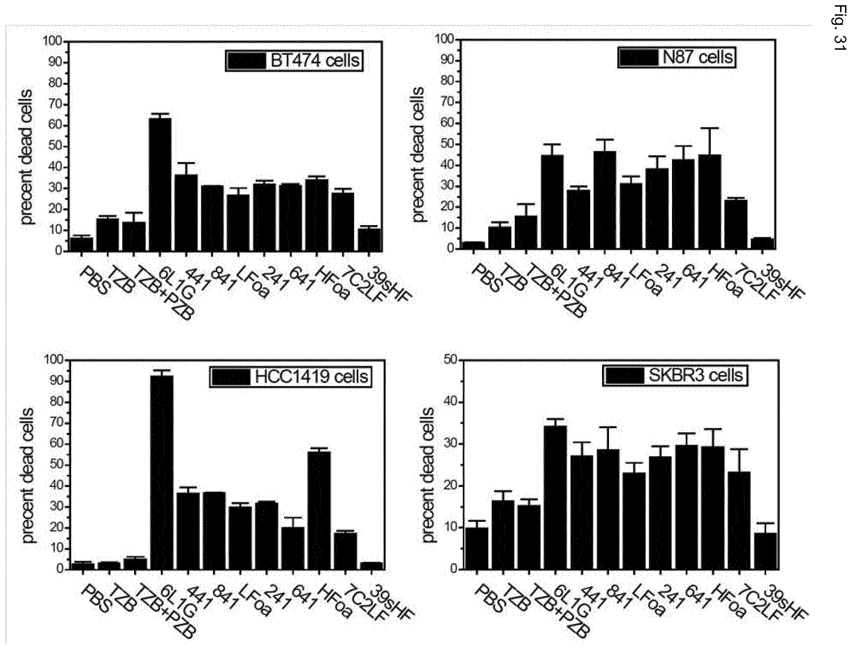

Fig. 31    shows the induction of cell death after treatment with 100 nM of indicated agents. BT474, N87, HCC1419 and SKBR3 cells were seeded 24 h before treatment in 96 black clear-well microscopy plates (Nunc), continuously treated for 3 days and stained with HOECHST-33342 (Invitrogen) for total cells and with propidium iodide (Sigma) for membrane-permeable dead cells. Cells were analyzed on a Lionheart FX Automated Microscope (BioTek Instruments) and the number of propidium iodide and HOECHST-33342 positive cells was quantified with Gen5 software (BioTek Instruments). The ratio of propidium iodide and HOECHST-33342 positive cells was calculated for 3 biological replicates and the mean and SD is shown in the corresponding column plots. Biparatopic binding agents (6L1G, 441, 841, LFoa, 241, 641, HFoa, 7C2LF) binding to domain 1 and 4 of HER2 induce continuously more dead cells than trastuzumab (TZB) or the combination of trastuzumab and pertuzumab (TZB+PZB) in HER2-positive cancer cells.

Fig. 32    shows the half-life of construct 441 in the serum of NSG mice. Drawn sera of mice with previous 441 injections (3 mg/kg) were analyzed by sandwich ELISA. 441 showed an alpha phase of around 4.3 hours, followed by a beta phase of more than 45 hours.

Fig. 33    shows in-vivo activity of 441 on N87 xenografts in SCID beige mice. After N87 tumors had reached 350 mm$^3$ in size, mice were treated with four injections of 441 (10 mg/kg) during 11 days. 441 lead to significant growth arrest compared to untreated mice.

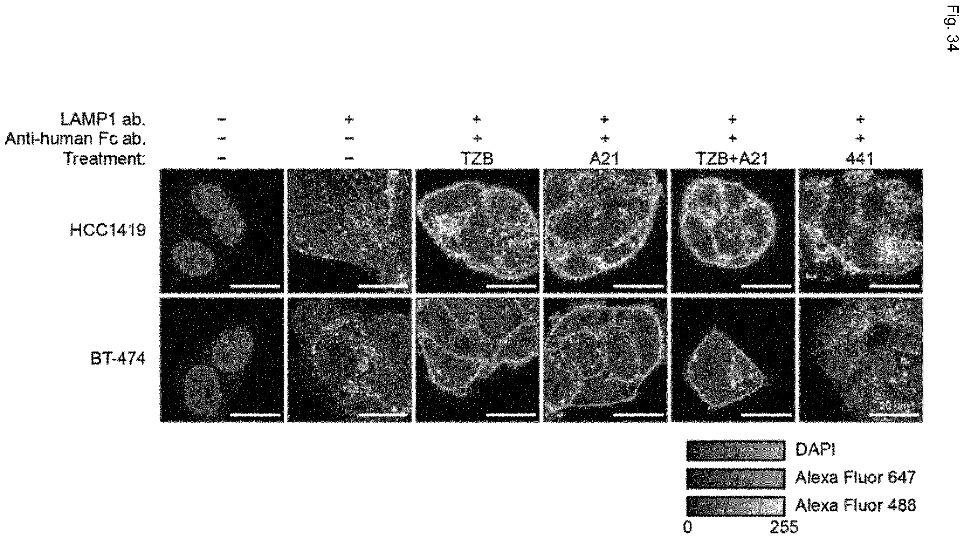

Fig. 34    shows representative microscopy images of BT-474 cells after treatment for 2 h with trastuzumab (TZB), huA21G (A21), their combination, or 441, and non-treated cells, either without or with addition of an anti-human primary antibody, as controls. Nuclei were stained using 2-(4-amidinophenyl)-1$H$-indol-6-carboximidamide (DAPI), antibodies were detected with an anti-human Fc antibody from goat, and lysosomal compartments using an anti-LAMP1 antibody.

Fig. 35    shows the result of a time-course treatment and subsequent surface protein internalization and degradation assay for the constructs 441 and 841, hA21G, trastuzumab (TZB), the combination of trastuzumab and hA21G (TZB + hA21G), pertuzumab (PZB), the combination of trastuzumab and pertuzumab (TZB + PZB), and the inhibitor of HSP90, geldanamycin (GA).

Example 1: Biparatopic Anti-HER2 Binding Agents

The inventors have generated biparatopic IgG derivatives. In contrast to other available biparatopic HER2-targeting antibodies, e.g. the antibody-drug conjugate (ADC) from Medimmune MEDI4276 (Li et al., 2016), these IgGs show very strong anti-tumor activity as "naked" binding proteins, i.e., without attached drug (Kast et al., in preparation). Thus, it is believed that these novel biparatopic anti-HER2 IgGs combine the mechanisms of action of trastuzumab plus pertuzumab plus the action of small molecule kinases inhibitors against HER2 in one single molecule. In addition, potential off-target effects of the biparatopic anti-HER2 IgGs are expected to remain far below those of ADC fusions, such as T-DM1 or MEDI4276, as they can only act on HER2-addicted cells, while ADCs can via their toxin act in many healthy tissue. This opens up the therapeutic windows for new combination therapies. Furthermore, pan-ErbB inhibition by polymerization of HER2 receptors may passively block compensatory activation of other receptor tyrosine kinases (RTKs). The biparatopic anti-HER2 binding agents interfere with the free lateral movement of HER2 receptors on the cell surface of HER2-amplified cancer, yet without inducing signaling competent complexes, which may block the activation of other RTKs. Consequently, biparatopic anti-HER2 binding agents may show strong synergies with small molecule inhibitors, which tend to induce expression of compensatory RTKs that eventually drives escape from therapy. Therefore, biparatopic anti-HER2 IgGs bear a very high potential to elicit strong anti-tumor synergies in combination with small-molecule inhibitors on a broad panel of HER2-amplified cancers. The potential for synergies with small-molecule inhibitors is superior to current single-specificity antibodies or antibody combinations.

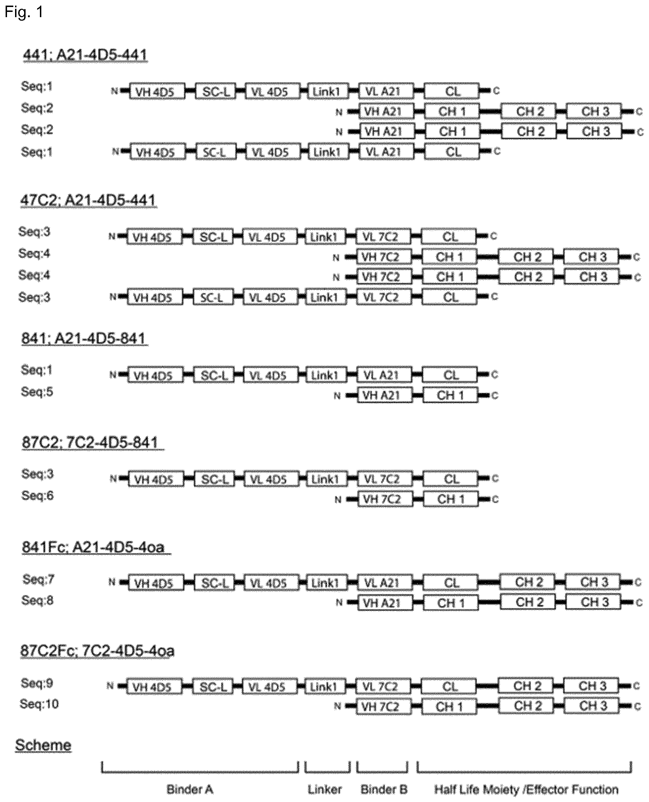

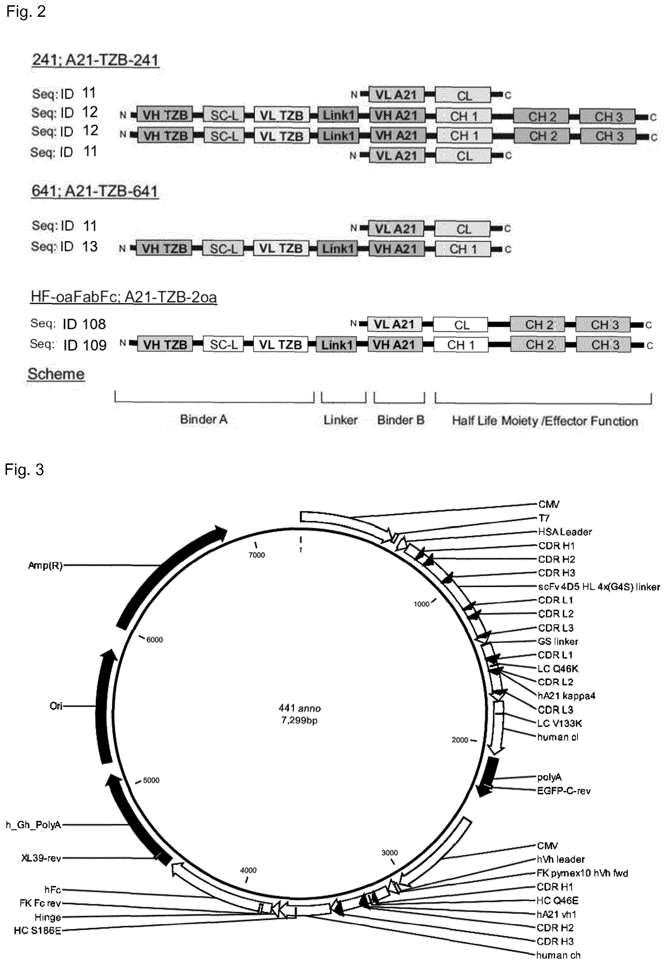

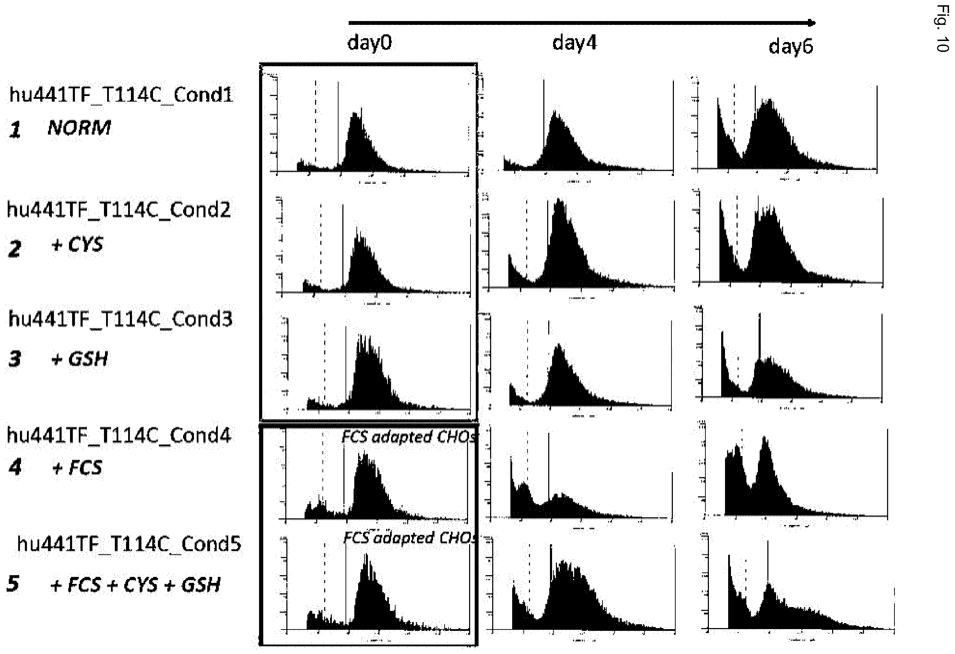

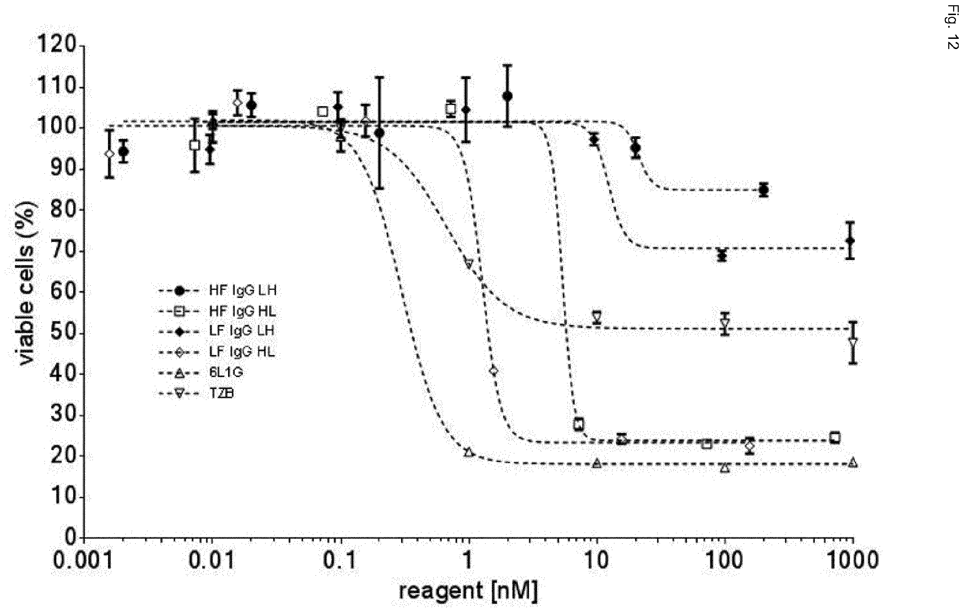

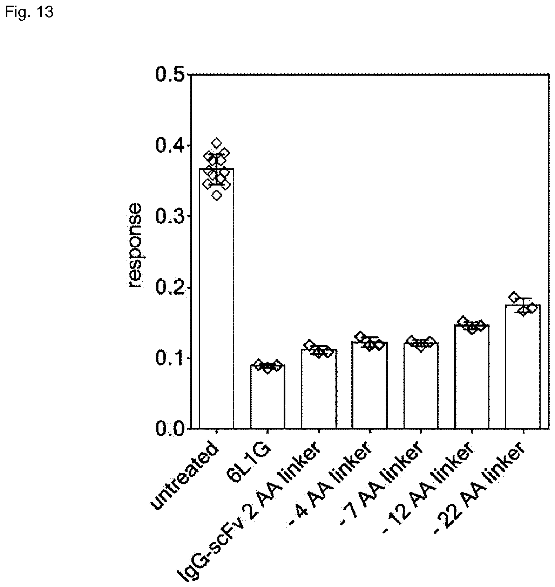

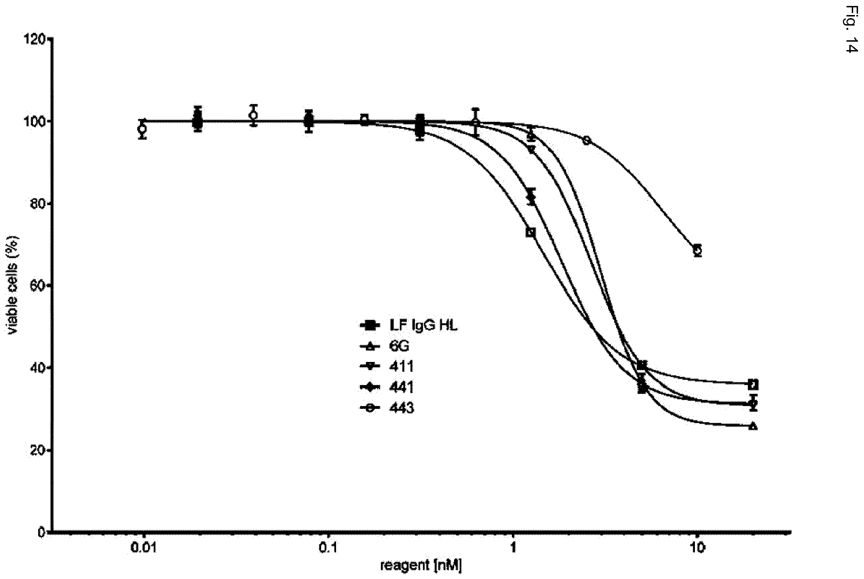

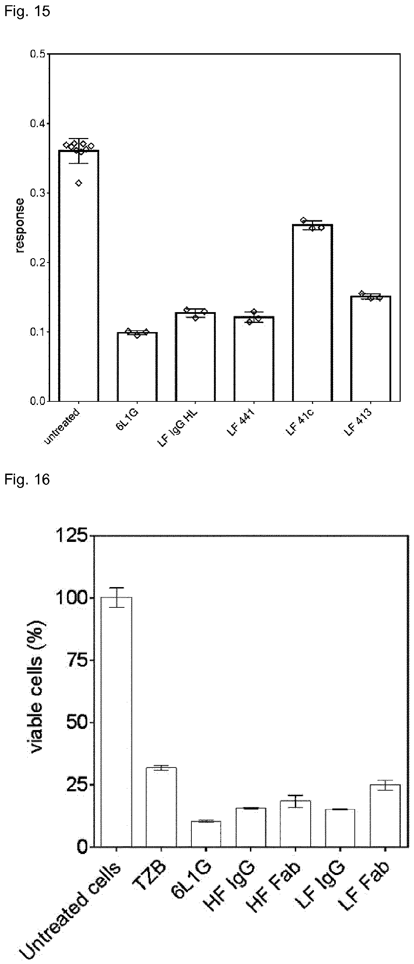

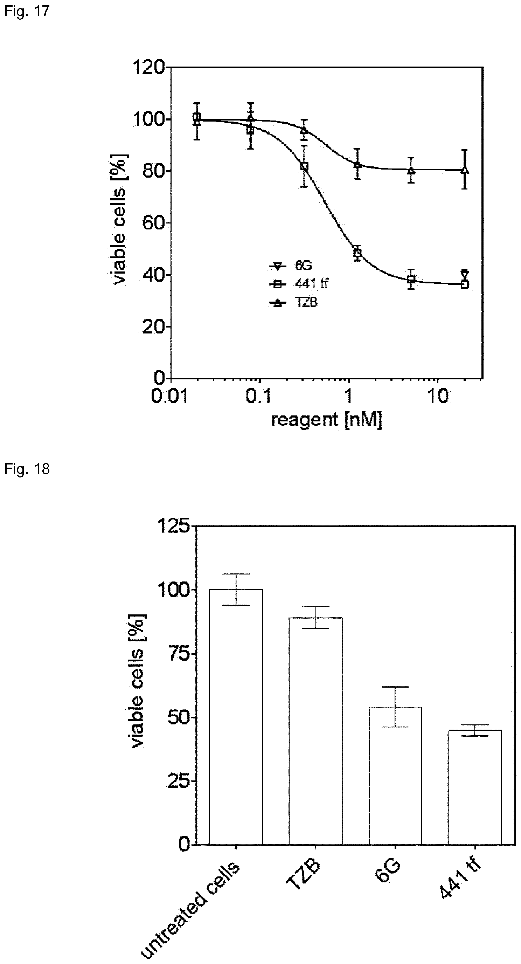

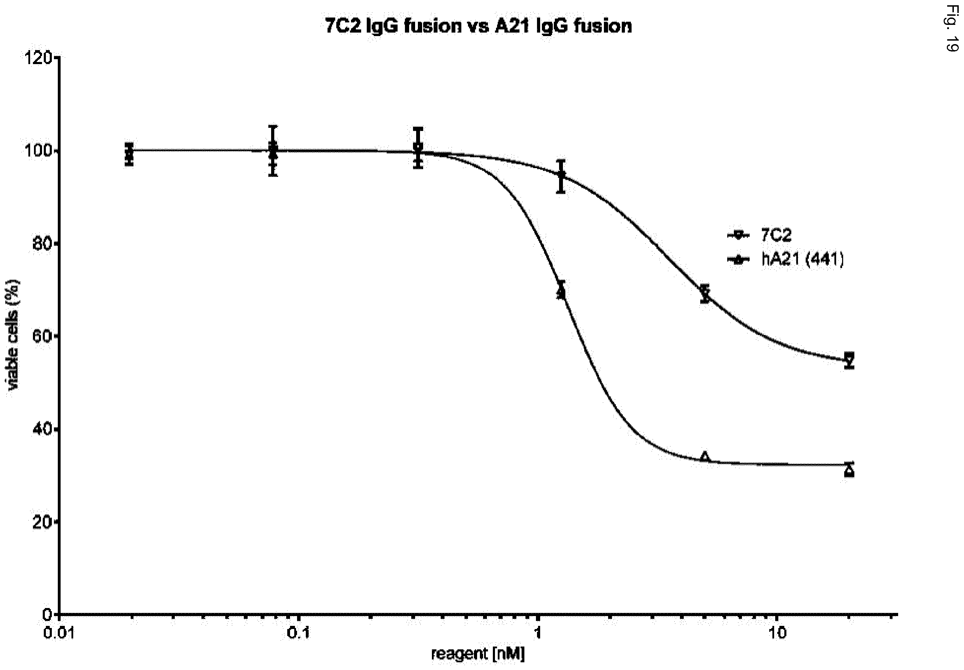

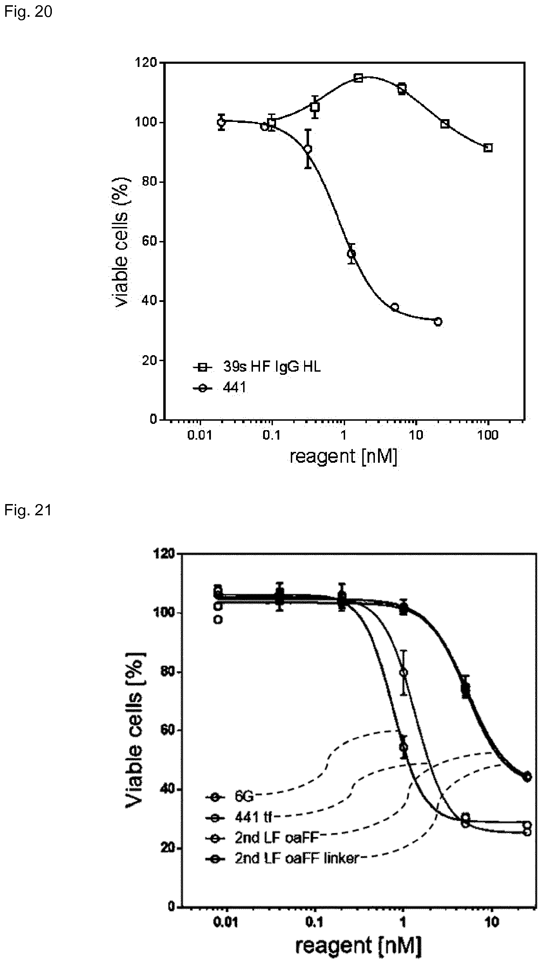

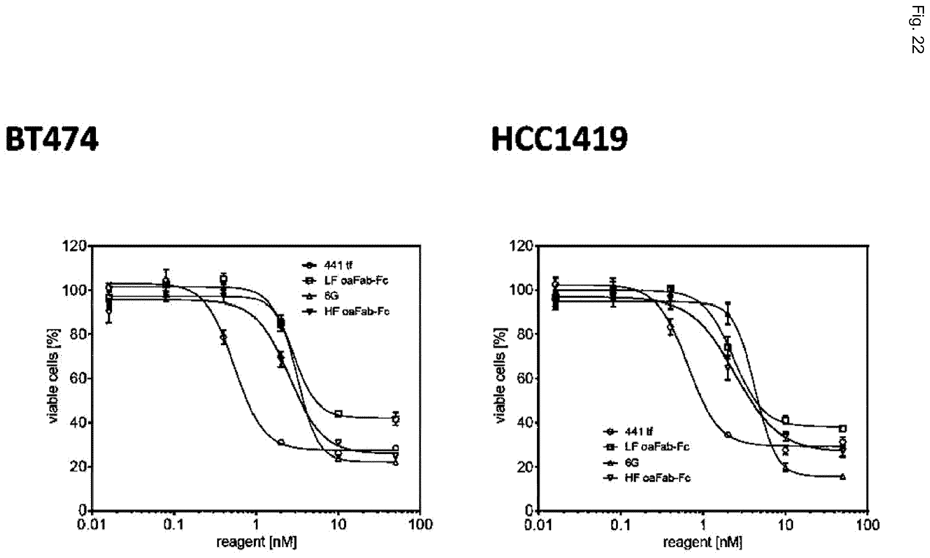

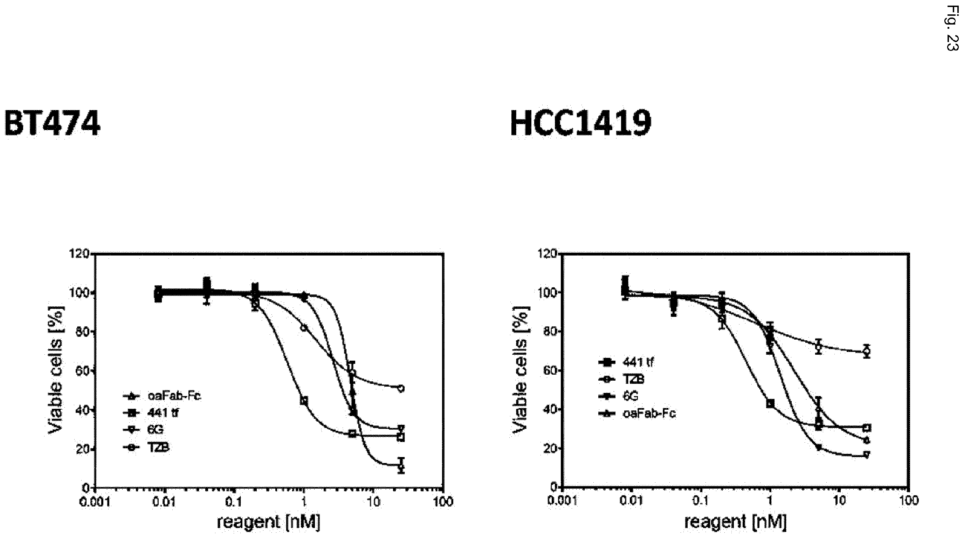

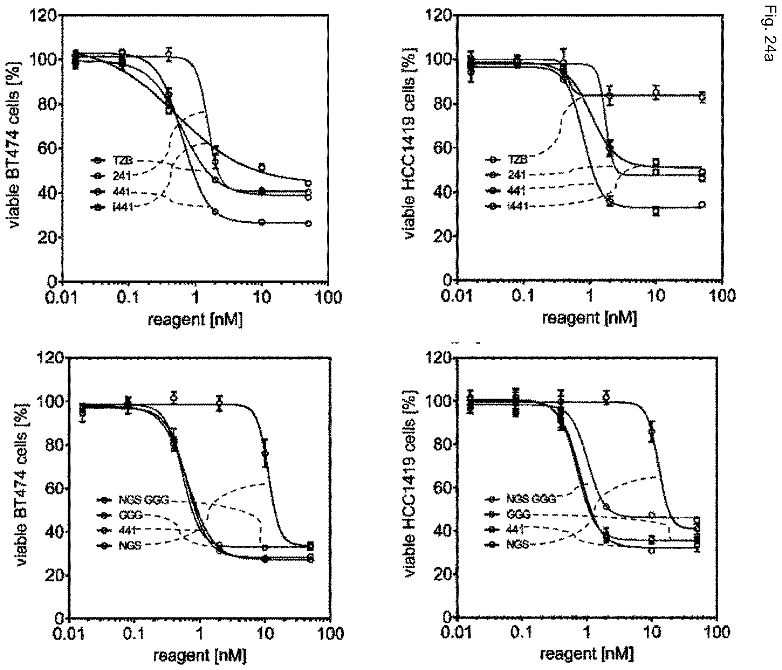

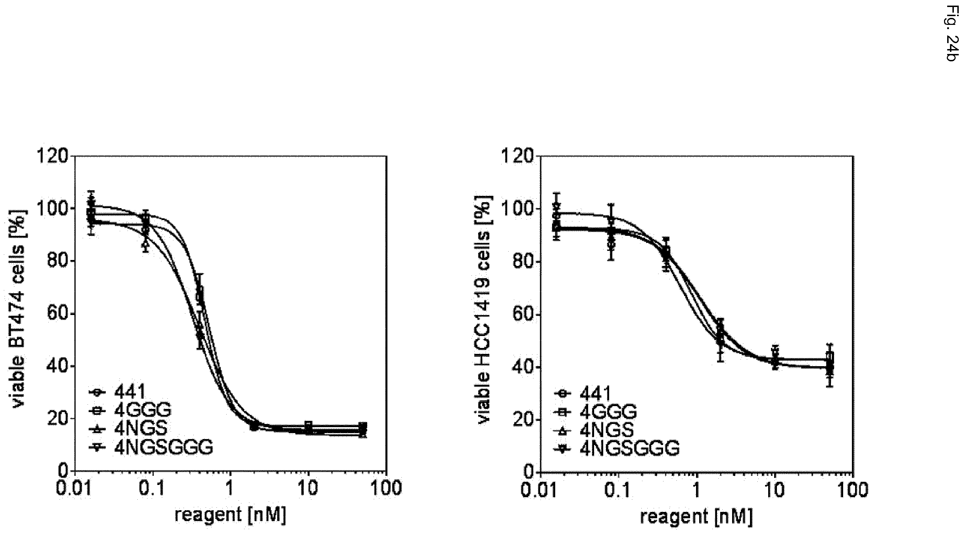

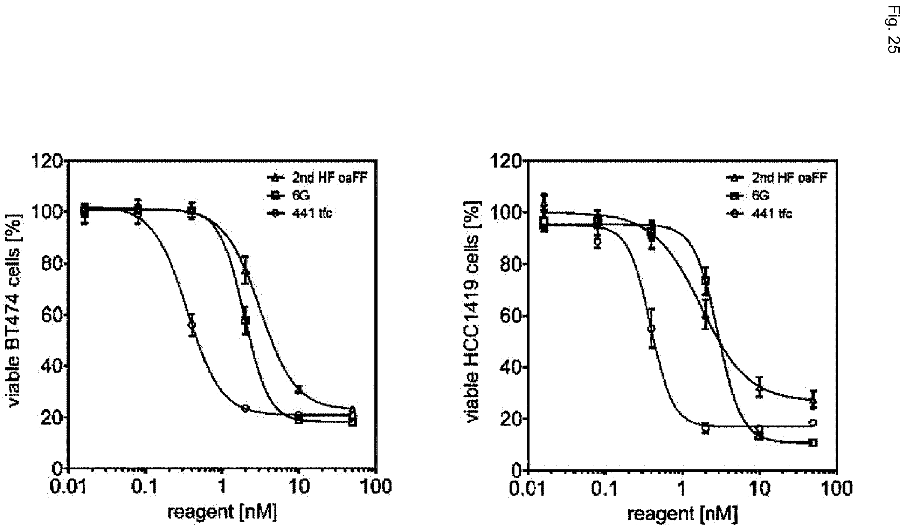

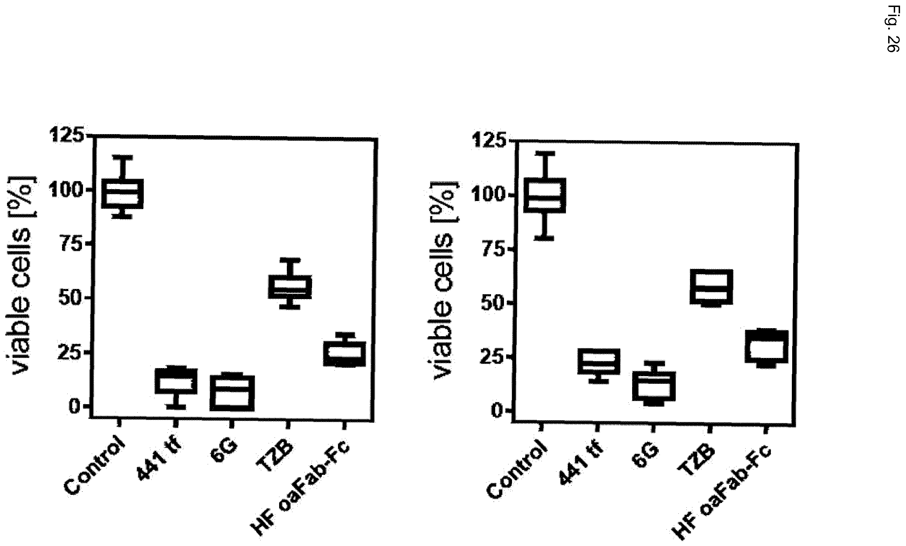

Illustrative schemes of preferred biparatopic IgG constructs are shown in Fig. 1 and 2
Data regarding preparation, and biological activity of the biparatopic IgG constructs are shown in Figs 3 to 26.

*Protocol for Production of Biparatopic IgGs in CHOs cells*

*Vector design*

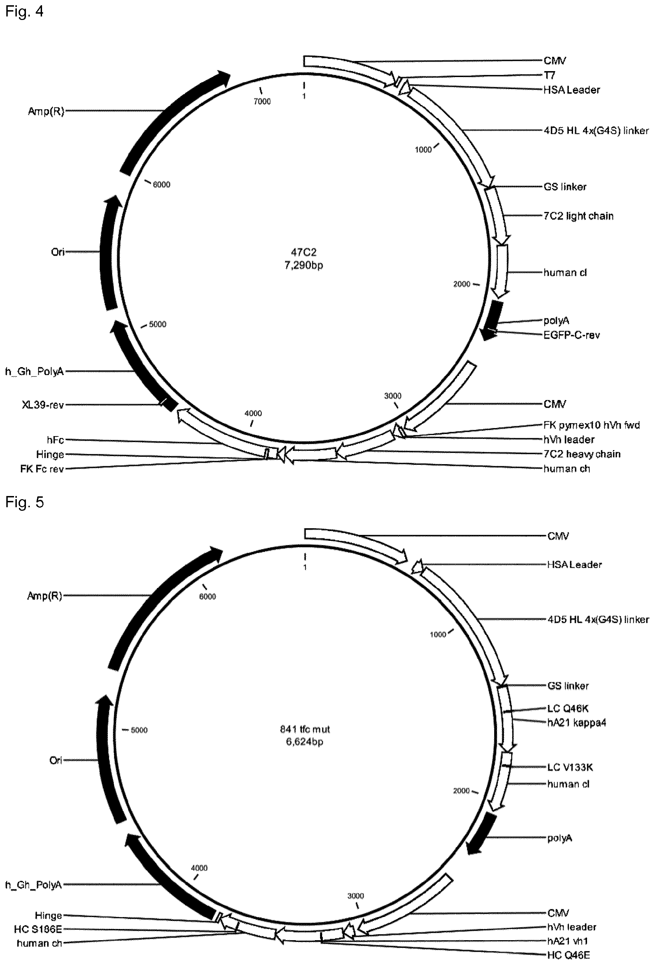

Bicistronic plasmids containing two expression cassettes or two vector systems were constructed for co-transfections. Derivatives of plasmid pYMex10 (Morphosys) were used for the bicistronic strategy. In the resulting plasmid constructs, the coding sequences of the polypeptide chains of the multimeric constructs were each under the control of an individual CMV promotor and terminated by a polyA tail signal as for example taken from bovine growth hormone or simian virus 40 (see Fig. 3 and 4). pcDNA 3.1 (Thermo) derived vectors were used for co-transfections. Here, the individual polypeptide chains are on a separate plasmid reducing the risk of recombination of homologous elements and further allow to adjust molar ratios of plasmids for the transfections to improve the yield. Exchange of genes of interest is possible by standard cloning techniques. Examples of the resulting constructs are depicted in Fig. 5.

*Expression in CHO-S*

Exponentially growing CHO-S cells (Thermo) were seeded in CHOgro (Mirrus) at a density of four millions per ml in TPP600 bioreactors. Per ml of culture 3 $\mu$g of linear polyethylenimine (MW 25,000, PolySciences Inc) and 1.25 $\mu$g of highly pure plasmid DNA were added with in-between mixing. Eventually, cultures were supplemented with valproic acid to a final concentration of 1 mM. Proteins were expressed at 31° or 37°C, 8% $CO_2$ and 180 rpm with a 50 mm throw in Kuhner ISF1-X shaker for up to 12 days.

*Purification of molecules*

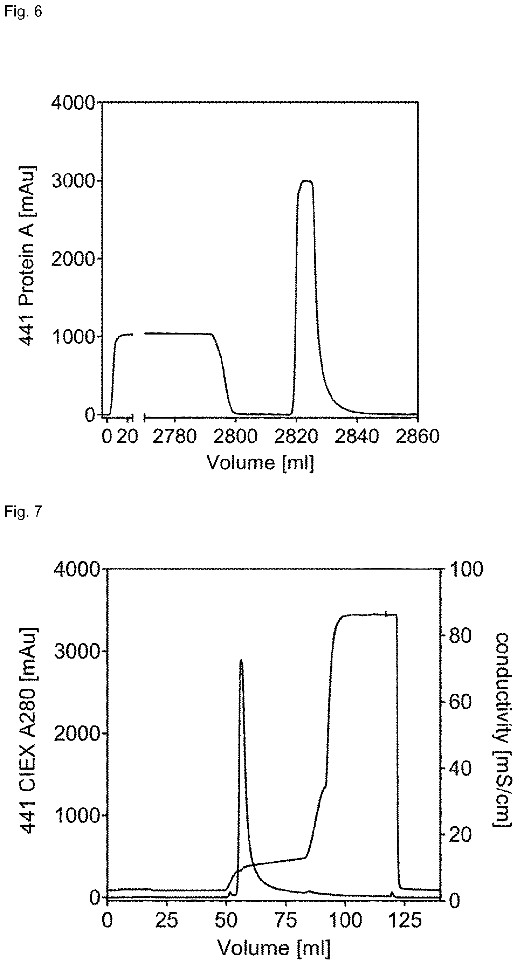

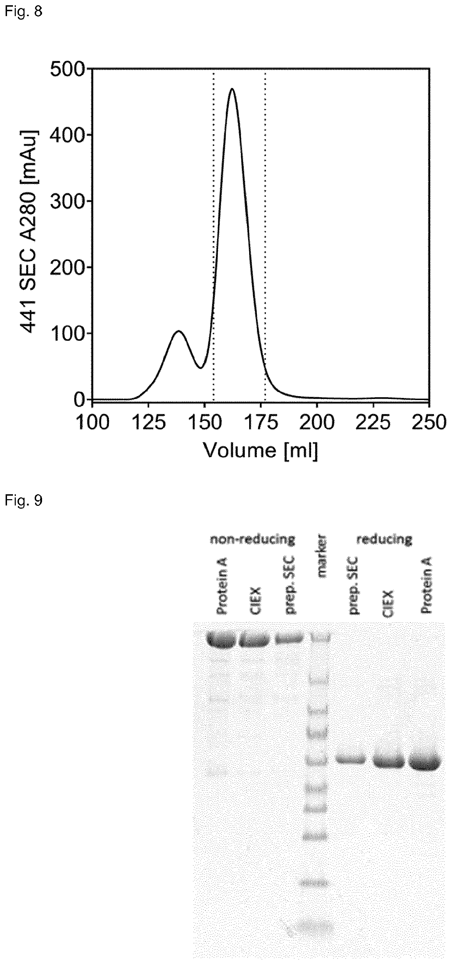

Expression cultures were harvested by centrifugation at 1400 rpm and 4°C for 30 min. Supernatants were furthermore cleared by 0.22 $\mu$m filtration and adjusted to pH of 7. All subsequent purification steps were performed at 4°C. Supernatants were applied to PBS equilibrated rProtein A columns (GE) operated on a AEKTA Pure system. After PBS wash, bound protein was eluted by 0.1 M Glycine pH 2.75 (Chromatogram Fig. 6). Fractions of interest were pooled and buffer exchanged to 20 mM Bis/Tris methane 20 mM NaCl pH 6.75. Filtered protein solutions were loaded on Resource S (GE) columns and eluted with a gradient to 100% 20 mM Bis/Tris methane 1000 mM NaCl pH 6.75 (see Fig. 7). Desired fractions were pooled and if required polished on SEC (Superdex200 columns of adequate size; GE) for high purity (Fig. 8). Monomeric fractions were pooled, filtered, and purity analyzed on SDS page (Fig. 9). Eventually, pure protein was used for biochemical and cell assays as well as in-vivo studies.

*HER2-related effects of tetravalent biparatopic IgGs*

The tetrameric (tetravalent and biparatopic) polypeptide constructs 441 and 47C2 were tested in various assays, which are all well-known to the skilled person, and compared to the tetravalent IgG-fusion MEDI4276 (without a toxin), the dimeric bivalent biparatopic polypeptide constructs 841, 87C2 and Fc fusions thereof, the bivalent biparatopic DARPin construct 6L1G (see patent application WO 2014/060365 A1), single IgGs (TZB, PZB, A21 and 7C2) and combinations thereof.

Growth inhibition was tested in an XTT cell viability assay using live-cell high-content microscopy, Hoechst staining and cell count. As shown in Table 1, constructs 441, 47C2, 841, 87C2 and the Fc fusions of 841 and 87C2, 6L1G and the combination of TZB with A21 resulted in full growth inhibition, whereas single antibodies and TZB combined with PZB lead to partial inhibition. Surprisingly, in the absence of a cytotoxic drug, the antibody MEDI4276 stimulated growth of XTT cells.

The effect of the constructs on apoptosis/cell death were analyzed by live-cell high-content microscopy with annexin-V and PI staining or by detecting cleaved PARP in cell lysates by Western blot for analysis of PARP cleavage.

Constructs 441, 47C2, 841, 87C2 and their Fc fusions, 6L1G had an effect on apoptosis, whereas MEDI4276, single antibodies did not influence apoptosis, and TZB and A21 had a partial effect. The combination TZB+PZB is able to induce apoptosis in a very small fraction of cells or in fragile cell lines.

HER2 crosslinking on the cell surface, also termed "lockdown", was measured by fluorescence recovery after photobleaching (FRAP) and single cell localization microscopy. A reduction of the FRAP signal indicates lower mobility of cells and therefore crosslinking in response to the polypeptide constructs.

441, 47C2 and 6L1G resulted in lockdown of receptors, and partial crosslinking effect was measured for 841, 87C2 and their Fc fusions as well as the antibody combinations TZB+PZB and TZB+A21. Single antibodies had no effect on crosslinking.

Furthermore, HER2 internalization into cells was analyzed by a surface protein internalization and degradation assay, confocal microscopy and flow cytometry as described in detail in example 2.

The tetravalent biparatopic constructs 441, 47C2 and MEDI4276 displayed a strong effect on HER2 internalization, whereas a recycling inhibition was detected for the combinations TZB+PZB and TZB+A21). The remaining constructs showed no effect.

HER2 degradation was tested by a surface protein internalization and degradation assay and Western blot detection of total HER2. Here, 441 and 47C2 lead to rapid strong degradation. MEDI4276 had an effect on degradation, but less strong compared to 441 and 47C2. In contrast, the antibody combinations resulted in slow degradation and the remaining constructs had no effect.

**Table 1: Results of assays measuring HER2-related effects of polypeptide constructs**

| Construct | Growth inhibition | Apoptosis/cell death | "Lockdown" (crosslinking on cells) | HER2 Internalization | HER2 Degradation |
|---|---|---|---|---|---|
| 441/47C2 | Yes | Yes | Yes | Strong | Rapid, Strong |
| MEDI4276 (without toxin) | No (stimulating !) | No | Not determined but expected | Same as 441 | Less than 441 |
| 841/87C2 and Fc fusions thereof | Yes | Yes | Partial | No | No |
| 6L1G | Yes | Yes | Yes | No | No |
| Single antibodies | Partial | No | No | No | No |
| TZB+PZB | Partial | No (yes) | Crosslinking | Recycling inhibition (very weak) | Very slow |
| TZB+A21 | Yes | Partial | Crosslinking | Recycling inhibition | Slow |

Table 2 shows the results of HER2 binding studies performed with the same constructs as the experiments described above. Binding was determined by flow cytometry and additionally by size-exclusion chromatography/multi angle light scattering (SEC-MALS) in case of complex formation between the biparatopic IgG constructs and HER2.

All constructs bound the extracellular domains 1, 2 and/or 4 as expected (see Table 2). 441, 47C2 and the combination TZB + A21 displayed an extremely slow off rate which was slower compared to the remaining constructs.

Interestingly, all biparatopic constructs resulted in a HER2 binding stoichiometry of close to 1:1. Single antibodies and antibody combinations only lead approximately to a 1:2 stoichiometry.

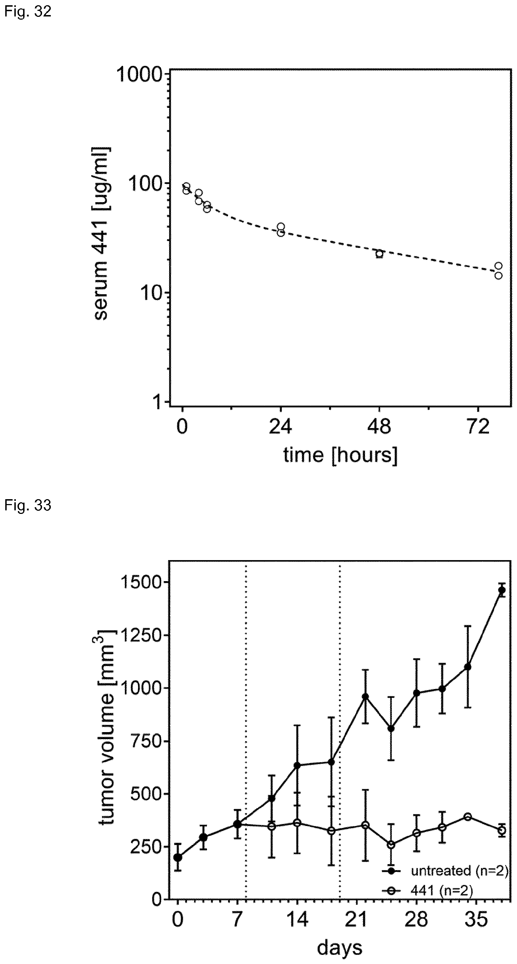

Serum half-life of construct 441 was further tested by intra venous injection in NSG mice and time resolved detection of biparatopic IgG in blood samples by ELISA. To determine the half-life of 441, 3 mg/kg of purified construct were intravenously injected in NSG mice. At indicated time points mice were blead, whole blood allowed to clot and sera gained by taking supernatants of centrifuged samples. A standard capture ELISA was used to evaluate serum levels of 441 (Fig. 32). Anti-human Fc antibody from mouse was directly coated on maxisorb plates. Bound 441 and sera spiked 441 standards were revealed by anti-kappa chain antibodies from goat conjugated to alkaline phosphatase. Data was fitted with a two-phase decay model and resulting half-life was calculated to be 4.3+45.3 hours ($\alpha$ and $\beta$ phase).

**Table 2: HER2 binding properties and other parameters of polypeptide constructs**

| Construct | Binds to | Off Rate | Bound HER2 molecules on average in solution | Total # Paratope per molecule | Serum Half life | Molecular weight kDa | Complex size on cells (single molecule) |
|---|---|---|---|---|---|---|---|
| 441/47C2 | HER2 ECD1/4 | extremely slow 2.7 x 10^-4 | Close to 1:1 | 4 | 45 hours | 200 | Large |
| MEDI4276 (without toxin) | HER2 ECD2/4 | Not determined But expected extremely slow | Close to 1:1 | 4 | rapid clearance (app. 1-3 day) | 200 | Not determined |
| 841/87C2 and Fc fusions thereof | HER2 ECD1/4 | Very slow | Close to 1:1 | 2 | Not determined | 72 (+FC = 122) | Not determined |
| 6L1G | HER2 ECD1/4 | Very slow | Close to 1:1 | 2 | 5 min (12 hours PEGylated) | 32 | Medium |

(continued)

| Construct | Binds to | Off Rate | Bound HER2 molecules on average in solution | Total # Paratope per molecule | Serum Half life | Molecular weight kDa | Complex size on cells (single molecule) |
|---|---|---|---|---|---|---|---|
| Single antibodies | HER2 ECD1/2/4 | Very slow | Close to 1:2 | 2 | 12 days | 145 | Small |
| TZB+PZB | HER2 ECD2/4 | Very slow | Close to 1:2 | 2 | 12 days | 145 | Not determined |
| TZB+A21 | HER2 ECD1/4 | extremely slow 3.66 x 10^-4 | Close to 1:2 | 2 | 12 days | 145 | Not determined |

Surprisingly, the tetrameric tetravalent biparatopic constructs 441 and 47C2 lead to strong inhibition of cell proliferation, induced cell death by apoptosis and led to crosslinking of HER2 on the cell surface and induced strong HER2 internalization and strong HER2 degradation in addition to their excellent binding properties to HER2. 441 and 47C2 were superior to or scored equally well as all other constructs in all categories.

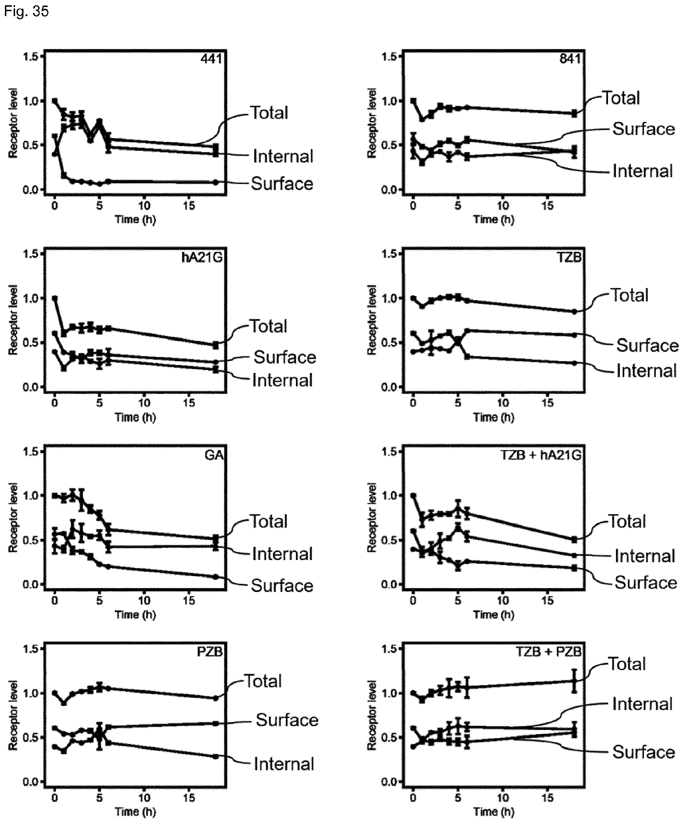

TZB+PZB and TZB+A21 resulted in a decrease of total HER2 (very weak in case of TZB+PZB) which was attributed to recycling inhibition, a mechanism by which HER2 is degraded without prior intracellular accumulation (see Fig. 35).

To determine the effect of construct 441 on tumor growth, SCID beige (Charles River) mice were inoculated on the right flank with five million N87 cells in 50% matrigel (Corning). After tumors had reached around 350 mm$^3$ mice were treated with flat injections of 200 $\mu$g of 441 for four times with a three to four day interval. Treated mice responded to 441 with growth arrest and tumors of control mice showed unhindered progress (Fig. 33).

Example 2: Enhanced Internalization. Lysosomal Trafficking, and Degradation of HER2 by revealed molecule 441

*Microscopy with BT-474 and HCC1419 breast cancer cells*

For microscopy of fixed samples, cells were seeded at a density of $4 \cdot 10^4$ cm$^{-2}$ in $\mu$-slides (Ibidi, cat. no. 80824) in complete medium. On the next day, cells were treated with the respective molecules. After 2 h, cells were once washed with Dulbecco's phosphate buffered saline (DPBS), and fixed by addition of 4% (w/v) paraformaldehyde dissolved in DPBS and incubation at room temperature for 10 min. Next, cells were washed twice with PBSBA+T (DPBS supplemented with 1% (w/v) bovine serum albumin (BSA), 0.1% (w/v) sodium azide, and 0.5% (w/v) Tween-20). Afterwards, cells were incubated in anti-LAMP antibody (Cell Signaling Technology, cat. no. D401S) dissolved at 1:150 (v/v) in PBSBA+T, further supplemented with 100 ng ml$^{-1}$ 2-(4-amidinophenyl)-1*H*-indol-6-carboximidamide (DAPI) for 30 min at room temperature. Cells were then washed twice with PBSBA+T, and subsequently anti-mouse, conjugated to Alexa Fluor 488 (Thermo Fisher Scientific, cat. no. A11001) and anti-human, conjugated to Alexa Fluor 647 (Thermo Fisher Scientific, cat. no. A-21445) antibodies from goat, dissolved in PBSBA+T, were added and incubated for 30 min at room temperature. Next, cells were washed twice with PBSBA+T, fixed once more by addition of 4% (w/v) paraformaldehyde dissolved in DPBS and incubation at room temperature for 10 min, finally washed once with PBSA, and stored in PBSA (DPBS supplemented with 0.1% (w/v) sodium azide) at 4 °C until measurement. Imaging was performed on a SP5 confocal laser scanning microscope (Leica). The images show that for both cell lines, only 441 was able to induce its rapid internalization, and shows strong colocalization with lysosomal (LAMP1-positive) compartments.

*Surface protein internalization and degradation assay*

To quantify internalization and degradation of HER2 upon treatments, we performed a quantitative surface protein internalization and degradation assay was performed. In brief, a stable FIp-In TREx HEK293 cell line (Thermo Fisher Scientific, cat. no. K650001) was generated according to the instructions of the manufacturer, in which a HaloTag-HER2 receptor fusion can be overexpressed upon induction. For the assay, cells were seeded two days before the first treatment, and one day before treatment, doxycycline was added to induce stable overexpression for 24 h. Treatments (100 nM) were added at indicated time points, referring to the time of cell labeling.

After completion of the treatment time intervals, cells were labeled in a two-step procedure. A HaloTag ligand containing to Alexa Fluor 660 (HTL-AF660, Promega, cat. no. G8472), which is completely cell-impermeable and therefore stains surface receptors only, was coupled in a first labeling step. A cell-permeable HaloTag ligand containing tetramethyl rhodamine (HTL-TMR, Promega, cat. no. G8252), was, in the second step, applied to stain all receptor fusion, which resides in intracellular compartments. Thus, signals originating from surface and internal receptor are detected in separate channels on a flow cytometer. Therefore, information regarding the localization, and using the rescaling procedure described below, about the quantitative distribution, can be obtained. A commercially available dead-cell stain was used for exclusion of permeabilized (dead) cells from analysis, for which all receptor would appear to be on the surface. Fluorescence intensities in each channel for 2'000-10'000 cells was recorded using a LSR II Fortessa (BD), and single, non-permeabilized cells gated. Mean fluorescence intensities of these populations were obtained using FlowJo 10.4 (FlowJo).

*Data processing*

To correct for different detection efficiencies of the flow cytometry instrument in the channels for AF660 and TMR, the data were scaled, yielding relative abundances. A control sample *(utr.,s.)*, in which the first (HTL-AF660-labeling) step is omitted, is required to this end. In this sample, all HaloTag molecules will react with HTL-TMR in this "single" (s.) labeling procedure, irrespective of their localization. Using the mean fluorescence intensities (*MFI*) of the singlet, non-permeabilized cell population, the normalized (feature-scaled) signal $S_{\text{TMR}}$ in the TMR channel for a samples is obtained by normalizing to a single-labeled, untreated control sample *(utr.,s.)* and background subtraction:

$$S_{\text{TMR}}(\text{sample}) = \frac{MFI_{\text{TMR}}(\text{sample}) - MFI_{\text{TMR}}(\text{utr.,unlab.})}{MFI_{\text{TMR}}(\text{utr., s.}) - MFI_{\text{TMR}}(\text{utr.,unlab.})} \#(\text{eq. S1}) \qquad ,$$

where *utr.,unlab.* represents an untreated, unlabeled control (showing only autofluorescence).

The first, surface-labeling step with cell-impermeable dye is virtually saturating in the complete two-step (double) labeling procedure. The normalized surface signal $S_{\text{AF660}}$ can thus be defined in:

$$S_{\text{AF660}}(\text{sample}) = \frac{MFI_{\text{AF660}}(\text{sample}) - MFI_{\text{AF660}}(\text{utr.,unlab.})}{MFI_{\text{AF660}}(\text{utr., d.}) - MFI_{\text{AF660}}(\text{utr.,unlab.})} \#(\text{eq. S2}).$$

The signal of a sample can be related to the surface signal from a double-labeled control (*utr.,d.*) and does not require a separate single-stained sample, however, because internal receptor is not accessible to HTL-AF660 and thus cannot be stained.

$\Delta S_{\text{TMR}}$, the difference from the single to the double labeling procedure in the TMR channel, now exactly corresponds to the number of molecules, which were blocked by the first, surface-specific step. The signal in the AF660 channel in the same double labeling experiment also is a direct correlate of this number of molecules:

$$\Delta S_{\text{TMR}} = S_{\text{TMR}}(\text{utr.,s.}) - S_{\text{TMR}}(\text{utr., d.}) = S_{\text{AF660,scaled}}(\text{utr.,d.}) \#(\text{eq. S3}).$$

A correction factor $C_A$ can thus be defined, which relates the measured intensity $S_{\text{AF660}}(\text{utr.,d.})$ (recorded in the AF660 channel) to $S_{\text{AF660,scaled}}(\text{utr.,d.})$ (in the scale of the TMR channel):

$$S_{\text{AF660,scaled}}(\text{utr.,d.}) = S_{\text{AF660}}(\text{utr.,d.}) \times C_A \#(\text{eq. S4}).$$

Using signals from single-labeled and double-labeled, untreated cells, calculation of $C_A$ is possible as follows:

$$C_A = \frac{S_{\text{TMR}}(\text{utr.,s.}) - S_{\text{TMR}}(\text{utr.,d.})}{S_{\text{AF660}}(\text{utr., d.})} = \frac{100\% - S_{\text{TMR}}(\text{utr.,d.})}{S_{\text{AF660}}(\text{utr., d.})} \#(\text{eq. S5}) \qquad ,$$

taking into consideration that the TMR signal of single-labeled, untreated cells was, using eq. S1, scaled to 100% before. Correction of the signals recorded in the AF660 channel, can, for treated samples, now be done according to:

$$S_{\text{AF660,scaled}}(\text{tre.,d.}) = C_{\text{A}} \times S_{\text{AF660}}(\text{tre.,d.}) \#(\text{eq. S6})$$

$S_{\text{TMR}}(\text{tre.,d.})$ and $S_{\text{AF660,scaled}}(\text{tre.,d.})$ then truly represent the abundance of internal and surface protein, respectively, and the sum $S_{\text{AF660,scaled}}(\text{tre.,d-})+S_{\text{TMR}}(\text{tre.,d.})$ represents the amount of total protein, for a double-labeled, treated sample, always relative to an untreated control sample.

Data scaling as described above was performed and the results plotting using MATLAB R2017b (MathWorks), R 3.5.1, Prism 6.07 (GraphPad), and Excel2016 (Microsoft). The results are shown in Fig. 35. In contrast to all other constructs, construct 441 showed almost HER2 internalization after less than 5 minutes.

Claims

1. A tetrameric polypeptide comprising or consisting of

a. a first polypeptide chain comprising a first VL antigen binding domain and a first CL constant domain,
b. a second polypeptide chain comprising a first VH antigen binding domain, a first CH1 constant domain, a first CH2 constant domain and a first CH3 constant domain,
c. a first ligand that specifically binds to a HER2 D4 epitope, wherein said first ligand is comprised in said first polypeptide chain and linked to the N-terminus of said first VL antigen binding domain by a first interdomain amino acid linker, or said first ligand is comprised in said second polypeptide chain and linked to the N-terminus of said first VH antigen binding domain by a first interdomain amino acid linker,
d. wherein the first VL antigen binding domain of the first polypeptide chain and the first VH antigen binding domain of the second polypeptide chain together constitute a second ligand, particularly a Fab domain, that specifically binds to a HER2 D1 epitope,
e. a third polypeptide chain comprising a second VL antigen binding domain and a second CL constant domain,
f. a fourth polypeptide chain comprising a second VH antigen binding domain, a second CH1 constant domain, a second CH2 constant domain and a second CH3 constant domain,
g. a third ligand that specifically binds to a HER2 D4 epitope, wherein said third ligand is comprised in said third polypeptide chain and linked to the N-terminus of said second VL antigen binding domain by a second interdomain amino acid linker, or said third ligand is comprised in said fourth polypeptide chain and linked to the N-terminus of said second VH antigen binding domain by a second interdomain amino acid linker,
h. wherein the second VL antigen binding domain of the third polypeptide chain and the second VH antigen binding domain of the fourth polypeptide chain together constitute a fourth ligand, particularly an Fab domain, that specifically binds to a HER2 D1 epitope.

2. The polypeptide according to claim 1, wherein

- said first polypeptide chain and said third polypeptide chain and/or said second polypeptide chain and said fourth polypeptide chain are **characterized by** a sequence identity with each other of 70% or more, particularly 80% or more, more particularly 90% or more, even more particularly 95% or more, wherein most particularly said first polypeptide chain and said third polypeptide chain and/or said second polypeptide chain and said fourth polypeptide chain are identical and/or wherein
- said first ligand and said third ligand are **characterized by** a sequence identity with each other of 70% or more, particularly 80% or more, more particularly 90% or more, even more particularly 95% or more, wherein most particularly said first ligand and said third ligand are identical.

3. The tetrameric polypeptide according to any one of the preceding claims, wherein said first CH2 constant domain and said first CH3 constant domain of said second polypeptide chain interact with, particularly are covalently linked with, said second CH2 constant domain and said second CH3 constant domain of said fourth polypeptide chain, such that a tetrameric polypeptide is formed.

4. The tetrameric polypeptide according to any one of the preceding claims, wherein said second polypeptide chain comprises a first hinge region between said first CH1 constant domain and said first CH2 constant domain, and said fourth polypeptide chain comprises a second hinge region between said second CH1 constant domain and said second CH2 constant domain, wherein said first hinge region and said second hinge region mediate complex formation between said second polypeptide chain and said fourth polypeptide chain, particularly by at least one disulphide bond, such that a tetrameric polypeptide is formed.

5. The polypeptide according to any one of the preceding claims, wherein said first ligand and/or said third ligand comprises or consists of a single-chain variable fragment polypeptide chain comprising an scFv heavy chain, an scFv linker chain, and an scFv light chain, wherein particularly

a. said scFv heavy chain of said first ligand and/or said third ligand comprises a peptide sequence **characterized by** a sequence identity of 70 % or more, more particularly 80 % or more, even more particularly 90 % or more, even more particularly 95 % or more, with a peptide sequence selected from SEQ ID No. 15, SEQ ID No. 21, SEQ ID No. 22, SEQ ID No. 23, SEQ ID No. 44, SEQ ID No. 53, SEQ ID No. 54 and SEQ ID No. 80, wherein most particularly said scFv heavy chain of said first ligand and/or said third ligand comprises a peptide sequence identical to a peptide sequence selected from SEQ ID No. 15, SEQ ID No. 21, SEQ ID No. 22, SEQ ID No. 23, SEQ ID No. 44, SEQ ID No. 53, SEQ ID No. 54 and SEQ ID No. 80, and

b. said scFv light chain of said first ligand and/or said third ligand comprises a peptide sequence **characterized by** a sequence identity of 70 % or more, more particularly 80 % or more, even more particularly 90 % or more, even more particularly 95 % or more, with a peptide sequence selected from SEQ ID No. 14, SEQ ID No. 24, SEQ ID No. 25, SEQ ID No. 26, SEQ ID No. 43 and SEQ ID No. 81, wherein most particularly said scFv light chain of said first ligand and/or said third ligand comprises a peptide sequence identical to a peptide sequence selected from SEQ ID No. 14, SEQ ID No. 24, SEQ ID No. 25, SEQ ID No. 26, SEQ ID No. 43 and SEQ ID No. 81.

6. The polypeptide according to claim 5, wherein said scFv linker chain comprises a peptide sequence **characterized by** a sequence identity of 70 % or more, particularly 80 % or more, more particularly 90 % or more, even more particularly 95 % or more, with SEQ ID No. 16, wherein most particularly said scFv linker chain comprises a peptide sequence identical to SEQ ID No. 16.

7. The polypeptide according to any one of the preceding claims, wherein

a. said first polypeptide chain and/or said third polypeptide chain comprises a peptide sequence **characterized by** a sequence identity of 70 % or more, particularly 80 % or more, more particularly 90 % or more, even more particularly 95 % or more, with a peptide sequence selected from SEQ ID No. 18, SEQ ID No. 30, SEQ ID No. 31, SEQ ID No. 32, SEQ ID No. 39, SEQ ID No. 41, SEQ ID No. 50 and SEQ ID 76, wherein most particularly said first polypeptide chain and/or said third polypeptide chain comprises a peptide sequence identical to a peptide sequence selected from SEQ ID No. 18, SEQ ID No. 30, SEQ ID No. 31, SEQ ID No. 32, SEQ ID No. 39, SEQ ID No. 41, SEQ ID No. 50 and SEQ ID No. 76, and

b. said second polypeptide chain and/or said fourth polypeptide chain comprises a peptide sequence **characterized by** a sequence identity of 70 % or more, particularly 80 % or more, more particularly 90 % or more, even more particularly 95 % or more, with a peptide sequence selected from SEQ ID No. 19, SEQ ID No. 20, SEQ ID No. 27, SEQ ID No. 28, SEQ ID No. 29, SEQ ID No. 40, SEQ ID No. 42, SEQ ID No. 51, SEQ ID No. 52 and SEQ ID 77, wherein most particularly said second polypeptide chain and/or said fourth polypeptide chain comprises a peptide sequence identical to a peptide sequence selected from SEQ ID No. 19, SEQ ID No. 20, SEQ ID No. 27, SEQ ID No. 28, SEQ ID No. 29, SEQ ID No. 40, SEQ ID No. 42, SEQ ID No. 51, SEQ ID No. 52 and SEQ ID 77.

8. The polypeptide according to any one of the preceding claims, wherein

a. said first polypeptide chain and/or said third polypeptide chain comprises a peptide sequence **characterized by** a sequence identity of 70 % or more, particularly 80 % or more, more particularly 90 % or more, even more particularly 95 % or more, with SEQ ID No. 36, SEQ ID No. 37, SEQ ID No. 38 and SEQ ID No. 78, wherein most particularly said first polypeptide chain and/or said third polypeptide chain comprises a peptide sequence identical to SEQ ID No. 36, SEQ ID No. 37, SEQ ID No. 38 and SEQ ID No. 78, and

b. said second polypeptide chain and/or said fourth polypeptide chain comprises a peptide sequence **characterized by** a sequence identity of 70 % or more, particularly 80 % or more, more particularly 90 % or more, even more particularly 95 % or more, with SEQ ID No. 33, SEQ ID No. 34, SEQ ID No. 35 and SEQ ID No. 79, wherein most particularly said second polypeptide chain and/or said fourth polypeptide chain comprises a peptide sequence identical to SEQ ID No. 33, SEQ ID No. 34, SEQ ID No. 35 and SEQ ID No. 79.

9. The polypeptide according to any one of the preceding claims, wherein

a. said first polypeptide chain and/or said third polypeptide chain is **characterized by** a sequence identity of 70 % or more, particularly 80 % or more, more particularly 90 % or more, even more particularly 95 % or more, with SEQ ID No. 1, wherein most particularly said first polypeptide chain and/or said third polypeptide chain is identical to SEQ ID No. 1, and

b. said second polypeptide chain and/or said fourth polypeptide chain is **characterized by** a sequence identity of 70 % or more, particularly 80 % or more, more particularly 90 % or more, even more particularly 95 % or more, with SEQ ID No. 2, wherein most particularly said second polypeptide chain and/or said fourth polypeptide chain is identical to SEQ ID No. 2.

10. The polypeptide according to any one of the preceding claims, wherein

a. said first polypeptide chain and/or said third polypeptide chain is **characterized by** a sequence identity of 70 % or more, particularly 80 % or more, more particularly 90 % or more, even more particularly 95 % or more, with SEQ ID No. 3, wherein most particularly said first polypeptide chain and/or said third polypeptide chain is identical to SEQ ID No. 3, and

b. said second polypeptide chain and/or said fourth polypeptide chain is **characterized by** a sequence identity of 70 % or more, particularly 80 % or more, more particularly 90 % or more, even more particularly 95 % or more, with SEQ ID No. 4, wherein most particularly said second polypeptide chain and/or said fourth polypeptide chain is identical to SEQ ID No. 4.

11. The polypeptide according to any one of the preceding claims, wherein said first interdomain amino acid linker and/or said second interdomain amino acid linker consists of 1, 2, 3, 4, 5, 6, 7, 8, 9, 10, 11, 12, 13, 14, 15, 16, 17, 18, 19 or 20 amino acids, and wherein said first interdomain amino acid linker and/or said second interdomain amino acid linker comprises or consists of amino acids G, A, J, S, T, P, C, V, M and E, particularly wherein said first interdomain amino acid linker and/or said second interdomain amino acid linker comprises or consists of amino acids G, S, A and T, more particularly wherein said first interdomain amino acid linker and/or said second interdomain amino acid linker is **characterized by** an amino acid sequence (GGGGS)n, with n being 1, 2, 3, 4 or 5 or said first interdomain amino acid linker and/or said second interdomain amino acid linker comprises or consists of a peptide sequence selected from one of SEQ ID No. 17, SEQ ID 55 to SQ ID 69 and SEQ ID 82 to SEQ ID 91 or a functional equivalent peptide sequence **characterized by** a sequence identity of at least 70%.

12. The polypeptide according to any one of the claims 1 to 11 for use in a method for the prevention or treatment of a malignant neoplastic disease associated with expression of HER2.

13. An isolated nucleic acid encoding at least one of the first polypeptide chain, the second polypeptide chain, the third polypeptide chain and the fourth polypeptide chain of the polypeptide according to any one of the claims 1 to 11.

14. A host cell which is adapted to produce at least one of the first polypeptide chain, the second polypeptide chain, the third polypeptide chain and the fourth polypeptide chain of the polypeptide according to any one of the claims 1 to 11, wherein particularly the host cell comprises the isolated nucleic acid according to claim 13, such that the host cell is able to produce at least one of the first polypeptide chain, the second polypeptide chain, the third polypeptide chain and the fourth polypeptide chain of the polypeptide according to any one of the claims 1 to 11.

15. A method for obtaining the polypeptide according to any one of the claims 1 to 11, wherein the method comprises culturing the host cell according to claim 14, so that at least one of the first polypeptide chain, the second polypeptide chain, the third polypeptide chain and the fourth polypeptide chain of the polypeptide according to any one of the claims 1 to 11 is produced.

Fig. 1

Fig. 2

<u>241; A21-TZB-241</u>

<u>641; A21-TZB-641</u>

<u>HF-oaFabFc; A21-TZB-2oa</u>

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24a

Fig. 24b

Fig. 25

Fig. 26

Fig. 27

Fig. 28

Fig. 29

Imaging – BT474 treated with 100 nm agent for 3 days – PI stained

control | TZB
6L1G | LF IgG HL

Imaging – BT474 treated with 100 nm reagent for 3 days – Annexin V stained

control | TZB
6L1G | LF IgG HL

Fig. 30

Fig. 31

Fig. 32

Fig. 33

Fig. 34

Fig. 35

Europäisches
Patentamt

European
Patent Office

Office européen
des brevets

# EUROPEAN SEARCH REPORT

Application Number

EP 19 17 2075

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | VAHEH OGANESYAN ET AL: "Structural insights into the mechanism of action of a biparatopic anti-HER2 antibody", JOURNAL OF BIOLOGICAL CHEMISTRY, vol. 293, no. 22, 18 April 2018 (2018-04-18), pages 8439-8448, XP55633117, ISSN: 0021-9258, DOI: 10.1074/jbc.M117.818013 * figure 1 * | 1-15 | INV. C07K16/32 C07K16/46 A61K39/00 A61P35/00 |
| Y | LI JOHN Y ET AL: "A Biparatopic HER2-Targeting Antibody-Drug Conjugate Induces Tumor Regression in Primary Models Refractory to or Ineligible for HER2-Targeted Therapy", CANCER CELL, CELL PRESS, US, vol. 29, no. 1, 11 January 2016 (2016-01-11), pages 117-129, XP029383988, ISSN: 1535-6108, DOI: 10.1016/J.CCELL.2015.12.008 * figure 1 * | 1-15 | |
| Y | WO 2014/060365 A1 (UNIVERSITÄT ZÜRICH PROREKTORAT MNW [CH]) 24 April 2014 (2014-04-24) * figure 19 * | 1-15 | TECHNICAL FIELDS SEARCHED (IPC)<br><br>C07K<br>A61K<br>A61P |
| A | WO 2015/091738 A1 (HOFFMANN LA ROCHE [CH]; HOFFMANN LA ROCHE [US]) 25 June 2015 (2015-06-25) * claim all; figure 1b * | 1-15 | |
| A | WO 2012/143523 A1 (GENMAB AS [DK]; DE GOEIJ BART [NL] ET AL.) 26 October 2012 (2012-10-26) * claim all; table 15 * | 1-15 | |

-/--

The present search report has been drawn up for all claims

| Place of search | Date of completion of the search | Examiner |
|---|---|---|
| Munich | 22 October 2019 | Fellows, Edward |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 19 17 2075

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/070565 A1 (MEDIMMUNE LLC [US]) 16 May 2013 (2013-05-16) * figure 1 * ----- | 1-15 | |
| A | WO 2015/157592 A1 (MEDIMMUNE LLC [US]) 15 October 2015 (2015-10-15) * paragraph [0177]; figure 7 * ----- | 1-15 | |

**TECHNICAL FIELDS SEARCHED (IPC)**

The present search report has been drawn up for all claims

| Place of search | Date of completion of the search | Examiner |
|---|---|---|
| Munich | 22 October 2019 | Fellows, Edward |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 2 of 2

## ANNEX TO THE EUROPEAN SEARCH REPORT
## ON EUROPEAN PATENT APPLICATION NO.

EP 19 17 2075

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2019

| Patent document cited in search report | | Publication date | Patent family member(s) | | Publication date |
|---|---|---|---|---|---|
| WO 2014060365 | A1 | 24-04-2014 | AU | 2013331763 A1 | 19-03-2015 |
| | | | CA | 2883264 A1 | 24-04-2014 |
| | | | EP | 2906591 A1 | 19-08-2015 |
| | | | JP | 2015532306 A | 09-11-2015 |
| | | | JP | 2019031559 A | 28-02-2019 |
| | | | US | 2015284463 A1 | 08-10-2015 |
| | | | WO | 2014060365 A1 | 24-04-2014 |
| WO 2015091738 | A1 | 25-06-2015 | BR | 112016010706 A2 | 05-12-2017 |
| | | | CA | 2925677 A1 | 25-06-2015 |
| | | | CN | 105829347 A | 03-08-2016 |
| | | | EP | 3083696 A1 | 26-10-2016 |
| | | | EP | 3327038 A2 | 30-05-2018 |
| | | | HK | 1223115 A1 | 21-07-2017 |
| | | | JP | 6510532 B2 | 08-05-2019 |
| | | | JP | 2017501706 A | 19-01-2017 |
| | | | JP | 2019141066 A | 29-08-2019 |
| | | | KR | 20160099087 A | 19-08-2016 |
| | | | RU | 2016129517 A | 25-01-2018 |
| | | | US | 2017029529 A1 | 02-02-2017 |
| | | | WO | 2015091738 A1 | 25-06-2015 |
| WO 2012143523 | A1 | 26-10-2012 | CA | 2832387 A1 | 26-10-2012 |
| | | | CN | 103796678 A | 14-05-2014 |
| | | | JP | 6177231 B2 | 09-08-2017 |
| | | | JP | 2014517823 A | 24-07-2014 |
| | | | US | 2017369590 A1 | 28-12-2017 |
| | | | WO | 2012143523 A1 | 26-10-2012 |
| WO 2013070565 | A1 | 16-05-2013 | AU | 2012336069 A1 | 22-05-2014 |
| | | | CA | 2854806 A1 | 16-05-2013 |
| | | | CN | 103906533 A | 02-07-2014 |
| | | | EP | 2776061 A1 | 17-09-2014 |
| | | | HK | 1202065 A1 | 18-09-2015 |
| | | | JP | 2014533249 A | 11-12-2014 |
| | | | US | 2014302038 A1 | 09-10-2014 |
| | | | US | 2017114151 A1 | 27-04-2017 |
| | | | WO | 2013070565 A1 | 16-05-2013 |
| WO 2015157592 | A1 | 15-10-2015 | AU | 2015243377 A1 | 15-09-2016 |
| | | | BR | 112016022910 A2 | 17-10-2017 |
| | | | CA | 2943299 A1 | 15-10-2015 |
| | | | CL | 2016002547 A1 | 23-06-2017 |
| | | | CN | 106232139 A | 14-12-2016 |
| | | | EP | 3129055 A1 | 15-02-2017 |
| | | | HK | 1232127 A1 | 05-01-2018 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 19 17 2075

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2019

| Patent document cited in search report | Publication date | Patent family member(s) | Publication date |
|---|---|---|---|
| | | JP 2017512765 A | 25-05-2017 |
| | | KR 20160143808 A | 14-12-2016 |
| | | SG 11201608192S A | 28-10-2016 |
| | | TW 201542594 A | 16-11-2015 |
| | | US 2017291955 A1 | 12-10-2017 |
| | | WO 2015157592 A1 | 15-10-2015 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4968603 A
- US 7371376 B
- WO 2014060365 A1

**Non-patent literature cited in the description**

- **HU S. et al.** *Proteins,* 2008, vol. 70, 938-949
- **CHEN et al.** *Advanced Drug Delivery Reviews,* 2013, vol. 65, 1357-1369
- **EVERS et al.** *Biochemistry,* 2006, vol. 45, 13183-13192

SEQUENCE LISTING

<110>  Universität Zürich

<120>  HER2-binding tetrameric polypeptides

<130>  uz385ep-a

<160>  93

<170>  PatentIn version 3.5

<210>  1
<211>  469
<212>  PRT
<213>  Artificial Sequence

<220>
<223>  Antibody fusion construct

<400>  1

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
            35                  40                  45

Ala Arg Ile Tyr Pro Thr Asn Gly Tyr Thr Arg Tyr Ala Asp Ser Val
        50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Gly Gly Gly Gly Ser Gly Gly Gly
            115                 120                 125

Gly Ser Gly Gly Gly Gly Ser Gly Gly Gly Gly Ser Asp Ile Gln Met
        130                 135                 140

Thr Gln Ser Pro Ser Ser Leu Ser Ala Ser Val Gly Asp Arg Val Thr
145                 150                 155                 160

Ile Thr Cys Arg Ala Ser Gln Asp Val Asn Thr Ala Val Ala Trp Tyr

                    165                    170                    175

Gln Gln Lys Pro Gly Lys Ala Pro Lys Leu Leu Ile Tyr Ser Ala Ser
            180                185                190

Phe Leu Tyr Ser Gly Val Pro Ser Arg Phe Ser Gly Ser Arg Ser Gly
            195                200                205

Thr Asp Phe Thr Leu Thr Ile Ser Ser Leu Gln Pro Glu Asp Phe Ala
    210                215                220

Thr Tyr Tyr Cys Gln Gln His Tyr Thr Thr Pro Pro Thr Phe Gly Gln
225                230                235                240

Gly Thr Lys Val Glu Ile Lys Gly Ser Asp Ile Val Leu Thr Gln Ser
            245                250                255

Pro Asp Ser Leu Ala Val Ser Leu Gly Glu Arg Ala Thr Ile Asn Cys
            260                265                270

Arg Ser Ser Gln Thr Leu Leu Tyr Ser Asn Asn Gln Lys Asn Tyr Leu
            275                280                285

Ala Trp Tyr Gln Lys Lys Pro Gly Gln Pro Pro Lys Leu Leu Ile Ser
    290                295                300

Trp Ala Phe Thr Arg Lys Ser Gly Val Pro Asp Arg Phe Ser Gly Ser
305                310                315                320

Gly Ser Gly Thr Asp Phe Thr Leu Thr Ile Ser Ser Leu Gln Ala Glu
            325                330                335

Asp Val Ala Val Tyr Tyr Cys Gln Gln Tyr Ser Asn Tyr Pro Trp Thr
            340                345                350

Phe Gly Gln Gly Thr Lys Val Glu Ile Lys Arg Thr Val Ala Ala Pro
            355                360                365

Ser Val Phe Ile Phe Pro Pro Ser Asp Glu Gln Leu Lys Ser Gly Thr
    370                375                380

Ala Ser Val Lys Cys Leu Leu Asn Asn Phe Tyr Pro Arg Glu Ala Lys
385                390                395                400

Val Gln Trp Lys Val Asp Asn Ala Leu Gln Ser Gly Asn Ser Gln Glu
            405                410                415

```
Ser Val Thr Glu Gln Asp Ser Lys Asp Ser Thr Tyr Ser Leu Ser Ser
            420             425             430

Thr Leu Thr Leu Ser Lys Ala Asp Tyr Glu Lys His Lys Val Tyr Ala
            435             440             445

Cys Glu Val Thr His Gln Gly Leu Ser Ser Pro Val Thr Lys Ser Phe
    450             455             460

Asn Arg Gly Glu Cys
465
```

<210> 2
<211> 448
<212> PRT
<213> Artificial Sequence

<220>
<223> Antibody fusion construct

<400> 2

```
Gln Val Gln Leu Val Gln Ser Gly Ala Glu Val Lys Lys Pro Gly Ala
1               5               10              15

Ser Val Lys Val Ser Cys Lys Ala Ser Gly Tyr Ser Phe Thr Gly Tyr
            20              25              30

Phe Ile Asn Trp Val Arg Glu Ala Pro Gly Gln Gly Leu Glu Trp Met
            35              40              45

Gly His Ile Ser Ser Ser Tyr Ala Thr Ser Thr Tyr Asn Gln Lys Phe
            50              55              60

Gln Gly Arg Val Thr Phe Thr Val Asp Thr Ser Ser Ser Thr Ala Tyr
65              70              75              80

Met Glu Leu Ser Ser Leu Arg Ser Glu Asp Thr Ala Val Tyr Tyr Cys
                85              90              95

Val Arg Ser Gly Asn Tyr Glu Glu Tyr Ala Met Asp Tyr Trp Gly Gln
            100             105             110

Gly Thr Leu Val Thr Val Ser Ser Ala Ser Thr Lys Gly Pro Ser Val
            115             120             125

Phe Pro Leu Ala Pro Ser Ser Lys Ser Thr Ser Gly Gly Thr Ala Ala
    130             135             140

Leu Gly Cys Leu Val Lys Asp Tyr Phe Pro Glu Pro Val Thr Val Ser
```

|        |     |     |     | 145 |     |     |     |     | 150 |     |     |     |     | 155 |     |     |     |     | 160 |
|--------|-----|-----|-----|-----|-----|-----|-----|-----|-----|-----|-----|-----|-----|-----|-----|-----|-----|-----|-----|

Trp Asn Ser Gly Ala Leu Thr Ser Gly Val His Thr Phe Pro Ala Val
165                     170                     175

Leu Gln Ser Ser Gly Leu Tyr Ser Leu Glu Ser Val Val Thr Val Pro
180                     185                     190

Ser Ser Ser Leu Gly Thr Gln Thr Tyr Ile Cys Asn Val Asn His Lys
195                     200                     205

Pro Ser Asn Thr Lys Val Asp Lys Arg Val Glu Pro Lys Ser Cys Asp
210                     215                     220

Lys Thr His Thr Cys Pro Pro Cys Pro Ala Pro Glu Leu Leu Gly Gly
225                     230                     235                     240

Pro Ser Val Phe Leu Phe Pro Pro Lys Pro Lys Asp Thr Leu Met Ile
245                     250                     255

Ser Arg Thr Pro Glu Val Thr Cys Val Val Val Asp Val Ser His Glu
260                     265                     270

Asp Pro Glu Val Lys Phe Asn Trp Tyr Val Asp Gly Val Glu Val His
275                     280                     285

Asn Ala Lys Thr Lys Pro Arg Glu Glu Gln Tyr Asn Ser Thr Tyr Arg
290                     295                     300

Val Val Ser Val Leu Thr Val Leu His Gln Asp Trp Leu Asn Gly Lys
305                     310                     315                     320

Glu Tyr Lys Cys Lys Val Ser Asn Lys Ala Leu Pro Ala Pro Ile Glu
325                     330                     335

Lys Thr Ile Ser Lys Ala Lys Gly Gln Pro Arg Glu Pro Gln Val Tyr
340                     345                     350

Thr Leu Pro Pro Ser Arg Glu Glu Met Thr Lys Asn Gln Val Ser Leu
355                     360                     365

Thr Cys Leu Val Lys Gly Phe Tyr Pro Ser Asp Ile Ala Val Glu Trp
370                     375                     380

Glu Ser Asn Gly Gln Pro Glu Asn Asn Tyr Lys Thr Thr Pro Pro Val
385                     390                     395                     400

```
Leu Asp Ser Asp Gly Ser Phe Phe Leu Tyr Ser Lys Leu Thr Val Asp
            405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Lys
            435                 440                 445
```

<210> 3
<211> 468
<212> PRT
<213> Artificial Sequence

<220>
<223> Antibody fusion construct

<400> 3

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
            35                  40                  45

Ala Arg Ile Tyr Pro Thr Asn Gly Tyr Thr Arg Tyr Ala Asp Ser Val
            50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Gly Gly Gly Gly Ser Gly Gly Gly
            115                 120                 125

Gly Ser Gly Gly Gly Gly Ser Gly Gly Gly Gly Ser Asp Ile Gln Met
    130                 135                 140

Thr Gln Ser Pro Ser Ser Leu Ser Ala Ser Val Gly Asp Arg Val Thr
145                 150                 155                 160

Ile Thr Cys Arg Ala Ser Gln Asp Val Asn Thr Ala Val Ala Trp Tyr
```

                    165                       170                          175

Gln Gln Lys Pro Gly Lys Ala Pro Lys Leu Leu Ile Tyr Ser Ala Ser
            180                   185                   190

Phe Leu Tyr Ser Gly Val Pro Ser Arg Phe Ser Gly Ser Arg Ser Gly
            195                   200                   205

Thr Asp Phe Thr Leu Thr Ile Ser Ser Leu Gln Pro Glu Asp Phe Ala
210                       215                   220

Thr Tyr Tyr Cys Gln Gln His Tyr Thr Thr Pro Pro Thr Phe Gly Gln
225                   230                   235                   240

Gly Thr Lys Val Glu Ile Lys Gly Ser Asp Ile Val Met Thr Gln Ser
                245                   250                   255

Pro Asp Ser Leu Ala Val Ser Leu Gly Glu Arg Ala Thr Ile Asn Cys
            260                   265                   270

Arg Ala Ser Gln Ser Val Ser Gly Ser Arg Phe Thr Tyr Met His Trp
            275                   280                   285

Tyr Gln Gln Lys Pro Gly Gln Pro Pro Lys Leu Leu Ile Lys Tyr Ala
            290                   295                   300

Ser Ile Leu Glu Ser Gly Val Pro Asp Arg Phe Ser Gly Ser Gly Ser
305                   310                   315                   320

Gly Thr Asp Phe Thr Leu Thr Ile Ser Ser Leu Gln Ala Glu Asp Val
                325                   330                   335

Ala Val Tyr Tyr Cys Gln His Ser Trp Glu Ile Pro Pro Trp Thr Phe
            340                   345                   350

Gly Gln Gly Thr Lys Val Glu Ile Lys Arg Thr Val Ala Ala Pro Ser
            355                   360                   365

Val Phe Ile Phe Pro Pro Ser Asp Glu Gln Leu Lys Ser Gly Thr Ala
            370                   375                   380

Ser Val Lys Cys Leu Leu Asn Asn Phe Tyr Pro Arg Glu Ala Lys Val
385                   390                   395                   400

Gln Trp Lys Val Asp Asn Ala Leu Gln Ser Gly Asn Ser Gln Glu Ser
                405                   410                   415

```
        Val Thr Glu Gln Asp Ser Lys Asp Ser Thr Tyr Ser Leu Ser Ser Thr
                    420             425                 430

        Leu Thr Leu Ser Lys Ala Asp Tyr Glu Lys His Lys Val Tyr Ala Cys
                    435             440                 445

        Glu Val Thr His Gln Gly Leu Ser Ser Pro Val Thr Lys Ser Phe Asn
            450             455                 460

        Arg Gly Glu Cys
        465


        <210>   4
        <211>   446
        <212>   PRT
        <213>   Artificial Sequence

        <220>
        <223>   Antibody fusion construct

        <400>   4

        Glu Val Gln Leu Val Gln Ser Gly Ala Glu Val Lys Lys Pro Gly Ala
        1               5               10                  15

        Ser Val Lys Val Ser Cys Lys Ala Ser Gly Tyr Ser Phe Thr Gly Tyr
                    20              25                  30

        Trp Met Asn Trp Val Arg Gln Ala Pro Gly Gln Gly Leu Glu Trp Ile
                    35              40                  45

        Gly Met Ile His Pro Leu Asp Ala Glu Ile Arg Ala Asn Gln Lys Phe
            50              55                  60

        Arg Asp Arg Val Thr Ile Thr Val Asp Thr Ser Thr Ser Thr Ala Tyr
        65              70                  75                  80

        Leu Glu Leu Ser Ser Leu Arg Ser Glu Asp Thr Ala Val Tyr Tyr Cys
                    85              90                  95

        Ala Arg Gly Thr Tyr Asp Gly Gly Phe Glu Tyr Trp Gly Gln Gly Thr
                    100             105                 110

        Leu Val Thr Val Ser Ser Ala Ser Thr Lys Gly Pro Ser Val Phe Pro
                    115             120                 125

        Leu Ala Pro Ser Ser Lys Ser Thr Ser Gly Gly Thr Ala Ala Leu Gly
            130             135                 140

        Cys Leu Val Lys Asp Tyr Phe Pro Glu Pro Val Thr Val Ser Trp Asn
```

```
                145                    150                    155                    160


            Ser Gly Ala Leu Thr Ser Gly Val His Thr Phe Pro Ala Val Leu Gln
                        165                    170                    175


            Ser Ser Gly Leu Tyr Ser Leu Glu Ser Val Val Thr Val Pro Ser Ser
                        180                    185                    190


            Ser Leu Gly Thr Gln Thr Tyr Ile Cys Asn Val Asn His Lys Pro Ser
                        195                    200                    205


            Asn Thr Lys Val Asp Lys Arg Val Glu Pro Lys Ser Cys Asp Lys Thr
                        210                    215                    220


            His Thr Cys Pro Pro Cys Pro Ala Pro Glu Leu Leu Gly Gly Pro Ser
            225                    230                    235                    240


            Val Phe Leu Phe Pro Pro Lys Pro Lys Asp Thr Leu Met Ile Ser Arg
                        245                    250                    255


            Thr Pro Glu Val Thr Cys Val Val Val Asp Val Ser His Glu Asp Pro
                        260                    265                    270


            Glu Val Lys Phe Asn Trp Tyr Val Asp Gly Val Glu Val His Asn Ala
                        275                    280                    285


            Lys Thr Lys Pro Arg Glu Glu Gln Tyr Asn Ser Thr Tyr Arg Val Val
                        290                    295                    300


            Ser Val Leu Thr Val Leu His Gln Asp Trp Leu Asn Gly Lys Glu Tyr
            305                    310                    315                    320


            Lys Cys Lys Val Ser Asn Lys Ala Leu Pro Ala Pro Ile Glu Lys Thr
                        325                    330                    335


            Ile Ser Lys Ala Lys Gly Gln Pro Arg Glu Pro Gln Val Tyr Thr Leu
                        340                    345                    350


            Pro Pro Ser Arg Glu Glu Met Thr Lys Asn Gln Val Ser Leu Thr Cys
                        355                    360                    365


            Leu Val Lys Gly Phe Tyr Pro Ser Asp Ile Ala Val Glu Trp Glu Ser
                        370                    375                    380


            Asn Gly Gln Pro Glu Asn Asn Tyr Lys Thr Thr Pro Pro Val Leu Asp
            385                    390                    395                    400
```

```
Ser Asp Gly Ser Phe Phe Leu Tyr Ser Lys Leu Thr Val Asp Lys Ser
              405             410             415

Arg Trp Gln Gln Gly Asn Val Phe Ser Cys Ser Val Met His Glu Ala
              420             425             430

Leu His Asn His Tyr Thr Gln Lys Ser Leu Ser Leu Ser Lys
              435             440             445


<210>  5
<211>  223
<212>  PRT
<213>  Artificial Sequence

<220>
<223>  Antibody fusion construct

<400>  5

Gln Val Gln Leu Val Gln Ser Gly Ala Glu Val Lys Lys Pro Gly Ala
1               5               10              15

Ser Val Lys Val Ser Cys Lys Ala Ser Gly Tyr Ser Phe Thr Gly Tyr
              20              25              30

Phe Ile Asn Trp Val Arg Glu Ala Pro Gly Gln Gly Leu Glu Trp Met
              35              40              45

Gly His Ile Ser Ser Ser Tyr Ala Thr Ser Thr Tyr Asn Gln Lys Phe
              50              55              60

Gln Gly Arg Val Thr Phe Thr Val Asp Thr Ser Ser Ser Thr Ala Tyr
65              70              75              80

Met Glu Leu Ser Ser Leu Arg Ser Glu Asp Thr Ala Val Tyr Tyr Cys
              85              90              95

Val Arg Ser Gly Asn Tyr Glu Glu Tyr Ala Met Asp Tyr Trp Gly Gln
              100             105             110

Gly Thr Leu Val Thr Val Ser Ser Ala Ser Thr Lys Gly Pro Ser Val
              115             120             125

Phe Pro Leu Ala Pro Ser Ser Lys Ser Thr Ser Gly Gly Thr Ala Ala
              130             135             140

Leu Gly Cys Leu Val Lys Asp Tyr Phe Pro Glu Pro Val Thr Val Ser
145             150             155             160

Trp Asn Ser Gly Ala Leu Thr Ser Gly Val His Thr Phe Pro Ala Val
```

165 170 175

Leu Gln Ser Ser Gly Leu Tyr Ser Leu Glu Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Arg Val Glu Pro Lys Ser Cys
210 215 220

<210> 6
<211> 221
<212> PRT
<213> Artificial Sequence

<220>
<223> Antibody fusion construct

<400> 6

Glu Val Gln Leu Val Gln Ser Gly Ala Glu Val Lys Lys Pro Gly Ala
1 5 10 15

Ser Val Lys Val Ser Cys Lys Ala Ser Gly Tyr Ser Phe Thr Gly Tyr
20 25 30

Trp Met Asn Trp Val Arg Gln Ala Pro Gly Gln Gly Leu Glu Trp Ile
35 40 45

Gly Met Ile His Pro Leu Asp Ala Glu Ile Arg Ala Asn Gln Lys Phe
50 55 60

Arg Asp Arg Val Thr Ile Thr Val Asp Thr Ser Thr Ser Thr Ala Tyr
65 70 75 80

Leu Glu Leu Ser Ser Leu Arg Ser Glu Asp Thr Ala Val Tyr Tyr Cys
85 90 95

Ala Arg Gly Thr Tyr Asp Gly Gly Phe Glu Tyr Trp Gly Gln Gly Thr
100 105 110

Leu Val Thr Val Ser Ser Ala Ser Thr Lys Gly Pro Ser Val Phe Pro
115 120 125

Leu Ala Pro Ser Ser Lys Ser Thr Ser Gly Gly Thr Ala Ala Leu Gly
130 135 140

Cys Leu Val Lys Asp Tyr Phe Pro Glu Pro Val Thr Val Ser Trp Asn
145 150 155 160

```
Ser Gly Ala Leu Thr Ser Gly Val His Thr Phe Pro Ala Val Leu Gln
            165                 170                 175


Ser Ser Gly Leu Tyr Ser Leu Glu Ser Val Val Thr Val Pro Ser Ser
            180                 185                 190


Ser Leu Gly Thr Gln Thr Tyr Ile Cys Asn Val Asn His Lys Pro Ser
            195                 200                 205


Asn Thr Lys Val Asp Lys Arg Val Glu Pro Lys Ser Cys
    210                 215                 220
```

```
<210>  7
<211>  696
<212>  PRT
<213>  Artificial Sequence

<220>
<223>  Antibody fusion construct

<400>  7
```

```
Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Asn Ile Lys Asp Thr
            20                  25                  30


Tyr Ile His Trp Val Arg Gln Ala Pro Gly Lys Gly Leu Glu Trp Val
            35                  40                  45


Ala Arg Ile Tyr Pro Thr Asn Gly Tyr Thr Arg Tyr Ala Asp Ser Val
    50                  55                  60


Lys Gly Arg Phe Thr Ile Ser Ala Asp Thr Ser Lys Asn Thr Ala Tyr
65                  70                  75                  80


Leu Gln Met Asn Ser Leu Arg Ala Glu Asp Thr Ala Val Tyr Tyr Cys
                85                  90                  95


Ser Arg Trp Gly Gly Asp Gly Phe Tyr Ala Met Asp Tyr Trp Gly Gln
            100                 105                 110


Gly Thr Leu Val Thr Val Ser Ser Gly Gly Gly Gly Ser Gly Gly Gly
            115                 120                 125


Gly Ser Gly Gly Gly Gly Ser Gly Gly Gly Gly Ser Asp Ile Gln Met
    130                 135                 140
```

```
Thr Gln Ser Pro Ser Ser Leu Ser Ala Ser Val Gly Asp Arg Val Thr
145             150             155             160

Ile Thr Cys Arg Ala Ser Gln Asp Val Asn Thr Ala Val Ala Trp Tyr
            165             170             175

Gln Gln Lys Pro Gly Lys Ala Pro Lys Leu Leu Ile Tyr Ser Ala Ser
            180             185             190

Phe Leu Tyr Ser Gly Val Pro Ser Arg Phe Ser Gly Ser Arg Ser Gly
            195             200             205

Thr Asp Phe Thr Leu Thr Ile Ser Ser Leu Gln Pro Glu Asp Phe Ala
210             215             220

Thr Tyr Tyr Cys Gln Gln His Tyr Thr Thr Pro Pro Thr Phe Gly Gln
225             230             235             240

Gly Thr Lys Val Glu Ile Lys Gly Ser Asp Ile Val Leu Thr Gln Ser
            245             250             255

Pro Asp Ser Leu Ala Val Ser Leu Gly Glu Arg Ala Thr Ile Asn Cys
            260             265             270

Arg Ser Ser Gln Thr Leu Leu Tyr Ser Asn Asn Gln Lys Asn Tyr Leu
            275             280             285

Ala Trp Tyr Gln Lys Lys Pro Gly Gln Pro Pro Lys Leu Leu Ile Ser
    290             295             300

Trp Ala Phe Thr Arg Lys Ser Gly Val Pro Asp Arg Phe Ser Gly Ser
305             310             315             320

Gly Ser Gly Thr Asp Phe Thr Leu Thr Ile Ser Ser Leu Gln Ala Glu
            325             330             335

Asp Val Ala Val Tyr Tyr Cys Gln Gln Tyr Ser Asn Tyr Pro Trp Thr
            340             345             350

Phe Gly Gln Gly Thr Lys Val Glu Ile Lys Arg Thr Val Ala Ala Pro
            355             360             365

Ser Val Phe Ile Phe Pro Pro Ser Asp Glu Gln Leu Lys Ser Gly Thr
    370             375             380

Ala Ser Val Lys Cys Leu Leu Asn Asn Phe Tyr Pro Arg Glu Ala Lys
385             390             395             400
```

```
Val Gln Trp Lys Val Asp Asn Ala Leu Gln Ser Gly Asn Ser Gln Glu
            405                 410                 415

Ser Val Thr Glu Gln Asp Ser Lys Asp Ser Thr Tyr Ser Leu Ser Ser
            420                 425                 430

Thr Leu Thr Leu Ser Lys Ala Asp Tyr Glu Lys His Lys Val Tyr Ala
            435                 440                 445

Cys Glu Val Thr His Gln Gly Leu Ser Ser Pro Val Thr Lys Ser Phe
    450                 455                 460

Asn Arg Gly Glu Cys Asp Lys Thr His Thr Cys Pro Pro Cys Pro Ala
465                 470                 475                 480

Pro Glu Leu Leu Gly Gly Pro Ser Val Phe Leu Phe Pro Pro Lys Pro
            485                 490                 495

Lys Asp Thr Leu Met Ile Ser Arg Thr Pro Glu Val Thr Cys Val Val
            500                 505                 510

Val Asp Val Ser His Glu Asp Pro Glu Val Lys Phe Asn Trp Tyr Val
            515                 520                 525

Asp Gly Val Glu Val His Asn Ala Lys Thr Lys Pro Arg Glu Glu Gln
            530                 535                 540

Tyr Asn Ser Thr Tyr Arg Val Val Ser Val Leu Thr Val Leu His Gln
545                 550                 555                 560

Asp Trp Leu Asn Gly Lys Glu Tyr Lys Cys Lys Val Ser Asn Lys Ala
            565                 570                 575

Leu Pro Ala Pro Ile Glu Lys Thr Ile Ser Lys Ala Lys Gly Gln Pro
            580                 585                 590

Arg Glu Pro Gln Val Tyr Thr Leu Pro Pro Ser Arg Lys Glu Met Thr
            595                 600                 605

Lys Asn Gln Val Ser Leu Thr Cys Leu Val Lys Gly Phe Tyr Pro Ser
            610                 615                 620

Asp Ile Ala Val Glu Trp Glu Ser Asn Gly Gln Pro Glu Asn Asn Tyr
625                 630                 635                 640

Lys Thr Thr Pro Pro Val Leu Lys Ser Asp Gly Ser Phe Phe Leu Tyr
```

```
                  645                      650                         655

        Ser Lys Leu Thr Val Asp Lys Ser Arg Trp Gln Gln Gly Asn Val Phe
                    660                 665                 670

        Ser Cys Ser Val Met His Glu Ala Leu His Asn His Tyr Thr Gln Lys
                    675                 680                 685

        Ser Leu Ser Leu Ser Pro Gly Lys
            690                 695


        <210>  8
        <211>  450
        <212>  PRT
        <213>  Artificial Sequence

        <220>
        <223>  Antibody fusion construct

        <400>  8

        Gln Val Gln Leu Val Gln Ser Gly Ala Glu Val Lys Lys Pro Gly Ala
        1               5                   10                  15

        Ser Val Lys Val Ser Cys Lys Ala Ser Gly Tyr Ser Phe Thr Gly Tyr
                    20                  25                  30

        Phe Ile Asn Trp Val Arg Glu Ala Pro Gly Gln Gly Leu Glu Trp Met
                    35                  40                  45

        Gly His Ile Ser Ser Ser Tyr Ala Thr Ser Thr Tyr Asn Gln Lys Phe
                50                  55                  60

        Gln Gly Arg Val Thr Phe Thr Val Asp Thr Ser Ser Ser Thr Ala Tyr
        65                  70                  75                  80

        Met Glu Leu Ser Ser Leu Arg Ser Glu Asp Thr Ala Val Tyr Tyr Cys
                    85                  90                  95

        Val Arg Ser Gly Asn Tyr Glu Glu Tyr Ala Met Asp Tyr Trp Gly Gln
                    100                 105                 110

        Gly Thr Leu Val Thr Val Ser Ser Ala Ser Thr Lys Gly Pro Ser Val
                    115                 120                 125

        Phe Pro Leu Ala Pro Ser Ser Lys Ser Thr Ser Gly Gly Thr Ala Ala
                130                 135                 140

        Leu Gly Cys Leu Val Lys Asp Tyr Phe Pro Glu Pro Val Thr Val Ser
        145                 150                 155                 160
```

```
Trp Asn Ser Gly Ala Leu Thr Ser Gly Val His Thr Phe Pro Ala Val
            165                 170                 175

Leu Gln Ser Ser Gly Leu Tyr Ser Leu Glu Ser Val Val Thr Val Pro
            180                 185                 190

Ser Ser Ser Leu Gly Thr Gln Thr Tyr Ile Cys Asn Val Asn His Lys
            195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
            245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
            275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
            325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
            355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Asp Thr Thr Pro Pro Val
385                 390                 395                 400

Leu Asp Ser Asp Gly Ser Phe Phe Leu Tyr Ser Asp Leu Thr Val Asp
```

```
                  405                    410                    415


      Lys Ser Arg Trp Gln Gln Gly Asn Val Phe Ser Cys Ser Val Met His
              420                    425                    430


      Glu Ala Leu His Asn His Tyr Thr Gln Lys Ser Leu Ser Leu Ser Pro
              435                    440                    445


      Gly Lys
          450



      <210>  9
      <211>  695
      <212>  PRT
      <213>  Artificial Sequence

      <220>
      <223>  Antibody fusion construct

      <400>  9

      Glu Val Gln Leu Val Glu Ser Gly Gly Gly Leu Val Gln Pro Gly Gly
      1               5                   10                  15


      Ser Leu Arg Leu Ser Cys Ala Ala Ser Gly Phe Asn Ile Lys Asp Thr
              20                  25                  30


      Tyr Ile His Trp Val Arg Gln Ala Pro Gly Lys Gly Leu Glu Trp Val
              35                  40                  45


      Ala Arg Ile Tyr Pro Thr Asn Gly Tyr Thr Arg Tyr Ala Asp Ser Val
              50                  55                  60


      Lys Gly Arg Phe Thr Ile Ser Ala Asp Thr Ser Lys Asn Thr Ala Tyr
      65                  70                  75                  80


      Leu Gln Met Asn Ser Leu Arg Ala Glu Asp Thr Ala Val Tyr Tyr Cys
                      85                  90                  95


      Ser Arg Trp Gly Gly Asp Gly Phe Tyr Ala Met Asp Tyr Trp Gly Gln
                  100                 105                 110


      Gly Thr Leu Val Thr Val Ser Ser Gly Gly Gly Gly Ser Gly Gly Gly
              115                 120                 125


      Gly Ser Gly Gly Gly Gly Ser Gly Gly Gly Gly Ser Asp Ile Gln Met
          130                 135                 140


      Thr Gln Ser Pro Ser Ser Leu Ser Ala Ser Val Gly Asp Arg Val Thr
      145                 150                 155                 160
```

```
Ile Thr Cys Arg Ala Ser Gln Asp Val Asn Thr Ala Val Ala Trp Tyr
            165             170             175

Gln Gln Lys Pro Gly Lys Ala Pro Lys Leu Leu Ile Tyr Ser Ala Ser
            180             185             190

Phe Leu Tyr Ser Gly Val Pro Ser Arg Phe Ser Gly Ser Arg Ser Gly
            195             200             205

Thr Asp Phe Thr Leu Thr Ile Ser Ser Leu Gln Pro Glu Asp Phe Ala
210             215             220

Thr Tyr Tyr Cys Gln Gln His Tyr Thr Thr Pro Pro Thr Phe Gly Gln
225             230             235             240

Gly Thr Lys Val Glu Ile Lys Gly Ser Asp Ile Val Met Thr Gln Ser
            245             250             255

Pro Asp Ser Leu Ala Val Ser Leu Gly Glu Arg Ala Thr Ile Asn Cys
            260             265             270

Arg Ala Ser Gln Ser Val Ser Gly Ser Arg Phe Thr Tyr Met His Trp
            275             280             285

Tyr Gln Gln Lys Pro Gly Gln Pro Pro Lys Leu Leu Ile Lys Tyr Ala
290             295             300

Ser Ile Leu Glu Ser Gly Val Pro Asp Arg Phe Ser Gly Ser Gly Ser
305             310             315             320

Gly Thr Asp Phe Thr Leu Thr Ile Ser Ser Leu Gln Ala Glu Asp Val
            325             330             335

Ala Val Tyr Tyr Cys Gln His Ser Trp Glu Ile Pro Pro Trp Thr Phe
            340             345             350

Gly Gln Gly Thr Lys Val Glu Ile Lys Arg Thr Val Ala Ala Pro Ser
            355             360             365

Val Phe Ile Phe Pro Pro Ser Asp Glu Gln Leu Lys Ser Gly Thr Ala
            370             375             380

Ser Val Lys Cys Leu Leu Asn Asn Phe Tyr Pro Arg Glu Ala Lys Val
385             390             395             400

Gln Trp Lys Val Asp Asn Ala Leu Gln Ser Gly Asn Ser Gln Glu Ser
```

```
                    405                      410                      415

        Val Thr Glu Gln Asp Ser Lys Asp Ser Thr Tyr Ser Leu Ser Ser Thr
                    420              425              430

        Leu Thr Leu Ser Lys Ala Asp Tyr Glu Lys His Lys Val Tyr Ala Cys
                    435              440              445

        Glu Val Thr His Gln Gly Leu Ser Ser Pro Val Thr Lys Ser Phe Asn
                    450              455              460

        Arg Gly Glu Cys Asp Lys Thr His Thr Cys Pro Pro Cys Pro Ala Pro
        465              470              475              480

        Glu Leu Leu Gly Gly Pro Ser Val Phe Leu Phe Pro Pro Lys Pro Lys
                        485              490              495

        Asp Thr Leu Met Ile Ser Arg Thr Pro Glu Val Thr Cys Val Val Val
                    500              505              510

        Asp Val Ser His Glu Asp Pro Glu Val Lys Phe Asn Trp Tyr Val Asp
                    515              520              525

        Gly Val Glu Val His Asn Ala Lys Thr Lys Pro Arg Glu Glu Gln Tyr
            530              535              540

        Asn Ser Thr Tyr Arg Val Val Ser Val Leu Thr Val Leu His Gln Asp
        545              550              555              560

        Trp Leu Asn Gly Lys Glu Tyr Lys Cys Lys Val Ser Asn Lys Ala Leu
                        565              570              575

        Pro Ala Pro Ile Glu Lys Thr Ile Ser Lys Ala Lys Gly Gln Pro Arg
                    580              585              590

        Glu Pro Gln Val Tyr Thr Leu Pro Pro Ser Arg Lys Glu Met Thr Lys
                    595              600              605

        Asn Gln Val Ser Leu Thr Cys Leu Val Lys Gly Phe Tyr Pro Ser Asp
            610              615              620

        Ile Ala Val Glu Trp Glu Ser Asn Gly Gln Pro Glu Asn Asn Tyr Lys
        625              630              635              640

        Thr Thr Pro Pro Val Leu Lys Ser Asp Gly Ser Phe Phe Leu Tyr Ser
                        645              650              655
```

```
Lys Leu Thr Val Asp Lys Ser Arg Trp Gln Gln Gly Asn Val Phe Ser
            660                 665             670


Cys Ser Val Met His Glu Ala Leu His Asn His Tyr Thr Gln Lys Ser
            675                 680             685


Leu Ser Leu Ser Pro Gly Lys
    690                 695
```

```
<210>  10
<211>  448
<212>  PRT
<213>  Artificial Sequence

<220>
<223>  Antibody fusion construct

<400>  10
```

```
Glu Val Gln Leu Val Gln Ser Gly Ala Glu Val Lys Lys Pro Gly Ala
1               5                   10                  15


Ser Val Lys Val Ser Cys Lys Ala Ser Gly Tyr Ser Phe Thr Gly Tyr
            20                  25                  30


Trp Met Asn Trp Val Arg Gln Ala Pro Gly Gln Gly Leu Glu Trp Ile
            35                  40                  45


Gly Met Ile His Pro Leu Asp Ala Glu Ile Arg Ala Asn Gln Lys Phe
    50                  55                  60


Arg Asp Arg Val Thr Ile Thr Val Asp Thr Ser Thr Ser Thr Ala Tyr
65                  70                  75                  80


Leu Glu Leu Ser Ser Leu Arg Ser Glu Asp Thr Ala Val Tyr Tyr Cys
                85                  90                  95


Ala Arg Gly Thr Tyr Asp Gly Gly Phe Glu Tyr Trp Gly Gln Gly Thr
            100                 105                 110


Leu Val Thr Val Ser Ser Ala Ser Thr Lys Gly Pro Ser Val Phe Pro
            115                 120                 125


Leu Ala Pro Ser Ser Lys Ser Thr Ser Gly Gly Thr Ala Ala Leu Gly
            130                 135                 140


Cys Leu Val Lys Asp Tyr Phe Pro Glu Pro Val Thr Val Ser Trp Asn
145                 150                 155                 160


Ser Gly Ala Leu Thr Ser Gly Val His Thr Phe Pro Ala Val Leu Gln
```

34

|     |     | 165 |     |     |     |     |     | 170 |     |     |     |     |     | 175 |     |
| --- | --- | --- | --- | --- | --- | --- | --- | --- | --- | --- | --- | --- | --- | --- | --- |

Ser Ser Gly Leu Tyr Ser Leu Glu Ser Val Val Thr Val Pro Ser Ser
180 185 190

Ser Leu Gly Thr Gln Thr Tyr Ile Cys Asn Val Asn His Lys Pro Ser
195 200 205

Asn Thr Lys Val Asp Lys Arg Val Glu Pro Lys Ser Cys Asp Lys Thr
210 215 220

His Thr Cys Pro Pro Cys Pro Ala Pro Glu Leu Leu Gly Gly Pro Ser
225 230 235 240

Val Phe Leu Phe Pro Pro Lys Pro Lys Asp Thr Leu Met Ile Ser Arg
245 250 255

Thr Pro Glu Val Thr Cys Val Val Val Asp Val Ser His Glu Asp Pro
260 265 270

Glu Val Lys Phe Asn Trp Tyr Val Asp Gly Val Glu Val His Asn Ala
275 280 285

Lys Thr Lys Pro Arg Glu Glu Gln Tyr Asn Ser Thr Tyr Arg Val Val
290 295 300

Ser Val Leu Thr Val Leu His Gln Asp Trp Leu Asn Gly Lys Glu Tyr
305 310 315 320

Lys Cys Lys Val Ser Asn Lys Ala Leu Pro Ala Pro Ile Glu Lys Thr
325 330 335

Ile Ser Lys Ala Lys Gly Gln Pro Arg Glu Pro Gln Val Tyr Thr Leu
340 345 350

Pro Pro Ser Arg Glu Glu Met Thr Lys Asn Gln Val Ser Leu Thr Cys
355 360 365

Leu Val Lys Gly Phe Tyr Pro Ser Asp Ile Ala Val Glu Trp Glu Ser
370 375 380

Asn Gly Gln Pro Glu Asn Asn Tyr Asp Thr Thr Pro Pro Val Leu Asp
385 390 395 400

Ser Asp Gly Ser Phe Phe Leu Tyr Ser Asp Leu Thr Val Asp Lys Ser
405 410 415

Arg Trp Gln Gln Gly Asn Val Phe Ser Cys Ser Val Met His Glu Ala
        420                 425                 430

Leu His Asn His Tyr Thr Gln Lys Ser Leu Ser Leu Ser Pro Gly Lys
        435                 440                 445


<210>  11
<211>  220
<212>  PRT
<213>  Artificial Sequence

<220>
<223>  Antibody fusion construct

<400>  11

Asp Ile Val Leu Thr Gln Ser Pro Asp Ser Leu Ala Val Ser Leu Gly
1               5                   10                  15

Glu Arg Ala Thr Ile Asn Cys Arg Ser Ser Gln Thr Leu Leu Tyr Ser
        20                  25                  30

Asn Asn Gln Lys Asn Tyr Leu Ala Trp Tyr Gln Lys Lys Pro Gly Gln
        35                  40                  45

Pro Pro Lys Leu Leu Ile Ser Trp Ala Phe Thr Arg Lys Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Ser Asn Tyr Pro Trp Thr Phe Gly Gln Gly Thr Lys Val Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Lys Cys Leu Leu Asn Asn
        130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
            165                 170                 175

Ser Thr Tyr Ser Leu Ser Ser Thr Leu Thr Leu Ser Lys Ala Asp Tyr

36

                    180                        185                        190

        Glu Lys His Lys Val Tyr Ala Cys Glu Val Thr His Gln Gly Leu Ser
                195                    200                    205

        Ser Pro Val Thr Lys Ser Phe Asn Arg Gly Glu Cys
            210                    215                    220

<210>   12
<211>   697
<212>   PRT
<213>   Artificial Sequence

<220>
<223>   Antibody fusion construct

<400>   12

        Glu Val Gln Leu Val Glu Ser Gly Gly Gly Leu Val Gln Pro Gly Gly
        1               5                   10                  15

        Ser Leu Arg Leu Ser Cys Ala Ala Ser Gly Phe Asn Ile Lys Asp Thr
                20                  25                  30

        Tyr Ile His Trp Val Arg Gln Ala Pro Gly Lys Gly Leu Glu Trp Val
                35                  40                  45

        Ala Arg Ile Tyr Pro Thr Asn Gly Tyr Thr Arg Tyr Ala Asp Ser Val
                50                  55                  60

        Lys Gly Arg Phe Thr Ile Ser Ala Asp Thr Ser Lys Asn Thr Ala Tyr
        65                  70                  75                  80

        Leu Gln Met Asn Ser Leu Arg Ala Glu Asp Thr Ala Val Tyr Tyr Cys
                        85                  90                  95

        Ser Arg Trp Gly Gly Asp Gly Phe Tyr Ala Met Asp Tyr Trp Gly Gln
                100                 105                 110

        Gly Thr Leu Val Thr Val Ser Ser Gly Gly Gly Gly Ser Gly Gly Gly
                115                 120                 125

        Gly Ser Gly Gly Gly Gly Ser Gly Gly Gly Gly Ser Asp Ile Gln Met
                130                 135                 140

        Thr Gln Ser Pro Ser Ser Leu Ser Ala Ser Val Gly Asp Arg Val Thr
        145                 150                 155                 160

        Ile Thr Cys Arg Ala Ser Gln Asp Val Asn Thr Ala Val Ala Trp Tyr
                        165                 170                 175

Gln Gln Lys Pro Gly Lys Ala Pro Lys Leu Leu Ile Tyr Ser Ala Ser
            180                 185                 190

Phe Leu Tyr Ser Gly Val Pro Ser Arg Phe Ser Gly Ser Arg Ser Gly
            195                 200                 205

Thr Asp Phe Thr Leu Thr Ile Ser Ser Leu Gln Pro Glu Asp Phe Ala
    210                 215                 220

Thr Tyr Tyr Cys Gln Gln His Tyr Thr Thr Pro Pro Thr Phe Gly Gln
225                 230                 235                 240

Gly Thr Lys Val Glu Ile Lys Gly Ser Gln Val Gln Leu Val Gln Ser
                245                 250                 255

Gly Ala Glu Val Lys Lys Pro Gly Ala Ser Val Lys Val Ser Cys Lys
            260                 265                 270

Ala Ser Gly Tyr Ser Phe Thr Gly Tyr Phe Ile Asn Trp Val Arg Glu
            275                 280                 285

Ala Pro Gly Gln Gly Leu Glu Trp Met Gly His Ile Ser Ser Ser Tyr
            290                 295                 300

Ala Thr Ser Thr Tyr Asn Gln Lys Phe Gln Gly Arg Val Thr Phe Thr
305                 310                 315                 320

Val Asp Thr Ser Ser Ser Thr Ala Tyr Met Glu Leu Ser Ser Leu Arg
                325                 330                 335

Ser Glu Asp Thr Ala Val Tyr Tyr Cys Val Arg Ser Gly Asn Tyr Glu
            340                 345                 350

Glu Tyr Ala Met Asp Tyr Trp Gly Gln Gly Thr Leu Val Thr Val Ser
            355                 360                 365

Ser Ala Ser Thr Lys Gly Pro Ser Val Phe Pro Leu Ala Pro Ser Ser
    370                 375                 380

Lys Ser Thr Ser Gly Gly Thr Ala Ala Leu Gly Cys Leu Val Lys Asp
385                 390                 395                 400

Tyr Phe Pro Glu Pro Val Thr Val Ser Trp Asn Ser Gly Ala Leu Thr
                405                 410                 415

Ser Gly Val His Thr Phe Pro Ala Val Leu Gln Ser Ser Gly Leu Tyr

|  |  |  | 420 |  |  |  | 425 |  |  |  | 430 |  |  |  |
|---|---|---|---|---|---|---|---|---|---|---|---|---|---|---|

Ser Leu Glu Ser Val Val Thr Val Pro Ser Ser Ser Leu Gly Thr Gln
      435             440             445

Thr Tyr Ile Cys Asn Val Asn His Lys Pro Ser Asn Thr Lys Val Asp
    450             455             460

Lys Arg Val Glu Pro Lys Ser Cys Asp Lys Thr His Thr Cys Pro Pro
465             470          475            480

Cys Pro Ala Pro Glu Leu Leu Gly Gly Pro Ser Val Phe Leu Phe Pro
          485          490            495

Pro Lys Pro Lys Asp Thr Leu Met Ile Ser Arg Thr Pro Glu Val Thr
        500          505          510

Cys Val Val Val Asp Val Ser His Glu Asp Pro Glu Val Lys Phe Asn
    515             520            525

Trp Tyr Val Asp Gly Val Glu Val His Asn Ala Lys Thr Lys Pro Arg
    530             535          540

Glu Glu Gln Tyr Asn Ser Thr Tyr Arg Val Val Ser Val Leu Thr Val
545             550          555            560

Leu His Gln Asp Trp Leu Asn Gly Lys Glu Tyr Lys Cys Lys Val Ser
        565          570          575

Asn Lys Ala Leu Pro Ala Pro Ile Glu Lys Thr Ile Ser Lys Ala Lys
        580          585          590

Gly Gln Pro Arg Glu Pro Gln Val Tyr Thr Leu Pro Pro Ser Arg Glu
        595          600          605

Glu Met Thr Lys Asn Gln Val Ser Leu Thr Cys Leu Val Lys Gly Phe
    610             615          620

Tyr Pro Ser Asp Ile Ala Val Glu Trp Glu Ser Asn Gly Gln Pro Glu
625             630          635            640

Asn Asn Tyr Lys Thr Thr Pro Pro Val Leu Asp Ser Asp Gly Ser Phe
        645          650          655

Phe Leu Tyr Ser Lys Leu Thr Val Asp Lys Ser Arg Trp Gln Gln Gly
        660          665          670

```
Asn Val Phe Ser Cys Ser Val Met His Glu Ala Leu His Asn His Tyr
        675                 680             685

Thr Gln Lys Ser Leu Ser Leu Ser Lys
    690                 695
```

<210> 13
<211> 472
<212> PRT
<213> Artificial Sequence

<220>
<223> Antibody fusion construct

<400> 13

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
        50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Gly Gly Gly Gly Ser Gly Gly Gly
            115                 120                 125

Gly Ser Gly Gly Gly Gly Ser Gly Gly Gly Gly Ser Asp Ile Gln Met
    130                 135                 140

Thr Gln Ser Pro Ser Ser Leu Ser Ala Ser Val Gly Asp Arg Val Thr
145                 150                 155                 160

Ile Thr Cys Arg Ala Ser Gln Asp Val Asn Thr Ala Val Ala Trp Tyr
            165                 170                 175

Gln Gln Lys Pro Gly Lys Ala Pro Lys Leu Leu Ile Tyr Ser Ala Ser
```

             180                    185               190

```
Phe Leu Tyr Ser Gly Val Pro Ser Arg Phe Ser Gly Ser Arg Ser Gly
        195                 200                 205

Thr Asp Phe Thr Leu Thr Ile Ser Ser Leu Gln Pro Glu Asp Phe Ala
        210                 215                 220

Thr Tyr Tyr Cys Gln Gln His Tyr Thr Thr Pro Pro Thr Phe Gly Gln
225                 230                 235                 240

Gly Thr Lys Val Glu Ile Lys Gly Ser Gln Val Gln Leu Val Gln Ser
                245                 250                 255

Gly Ala Glu Val Lys Lys Pro Gly Ala Ser Val Lys Val Ser Cys Lys
                260                 265                 270

Ala Ser Gly Tyr Ser Phe Thr Gly Tyr Phe Ile Asn Trp Val Arg Glu
                275                 280                 285

Ala Pro Gly Gln Gly Leu Glu Trp Met Gly His Ile Ser Ser Ser Tyr
        290                 295                 300

Ala Thr Ser Thr Tyr Asn Gln Lys Phe Gln Gly Arg Val Thr Phe Thr
305                 310                 315                 320

Val Asp Thr Ser Ser Ser Thr Ala Tyr Met Glu Leu Ser Ser Leu Arg
                325                 330                 335

Ser Glu Asp Thr Ala Val Tyr Tyr Cys Val Arg Ser Gly Asn Tyr Glu
                340                 345                 350

Glu Tyr Ala Met Asp Tyr Trp Gly Gln Gly Thr Leu Val Thr Val Ser
        355                 360                 365

Ser Ala Ser Thr Lys Gly Pro Ser Val Phe Pro Leu Ala Pro Ser Ser
        370                 375                 380

Lys Ser Thr Ser Gly Gly Thr Ala Ala Leu Gly Cys Leu Val Lys Asp
385                 390                 395                 400

Tyr Phe Pro Glu Pro Val Thr Val Ser Trp Asn Ser Gly Ala Leu Thr
                405                 410                 415

Ser Gly Val His Thr Phe Pro Ala Val Leu Gln Ser Ser Gly Leu Tyr
                420                 425                 430
```

Ser Leu Glu Ser Val Val Thr Val Pro Ser Ser Ser Leu Gly Thr Gln
        435                     440                 445

Thr Tyr Ile Cys Asn Val Asn His Lys Pro Ser Asn Thr Lys Val Asp
        450                     455                 460

Lys Arg Val Glu Pro Lys Ser Cys
465                 470


<210> 14
<211> 107
<212> PRT
<213> Artificial Sequence

<220>
<223> Antibody light chain

<400> 14

Asp Ile Gln Met Thr Gln Ser Pro Ser Ser Leu Ser Ala Ser Val Gly
1               5               10                  15

Asp Arg Val Thr Ile Thr Cys Arg Ala Ser Gln Asp Val Asn Thr Ala
        20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105


<210> 15
<211> 120
<212> PRT
<213> Artificial Sequence

<220>
<223> Antibody heavy chain

<400> 15

Glu Val Gln Leu Val Glu Ser Gly Gly Gly Leu Val Gln Pro Gly Gly
1               5               10                  15


42

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
            35                  40                  45

Ala Arg Ile Tyr Pro Thr Asn Gly Tyr Thr Arg Tyr Ala Asp Ser Val
            50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
            115                 120

<210> 16
<211> 20
<212> PRT
<213> Artificial Sequence

<220>
<223> scFv linker

<400> 16

Gly Gly Gly Gly Ser Gly Gly Gly Gly Ser Gly Gly Gly Gly Ser Gly
1                5                   10                  15

Gly Gly Gly Ser
            20

<210> 17
<211> 2
<212> PRT
<213> Artificial Sequence

<220>
<223> linker

<400> 17

Gly Ser
1

<210> 18
<211> 220

<212>    PRT
<213>    Artificial Sequence

<220>
<223>    Antibody light chain

<400>    18

Asp Ile Val Leu Thr Gln Ser Pro Asp Ser Leu Ala Val Ser Leu Gly
1               5                   10                  15

Glu Arg Ala Thr Ile Asn Cys Arg Ser Ser Gln Thr Leu Leu Tyr Ser
            20                  25                  30

Asn Asn Gln Lys Asn Tyr Leu Ala Trp Tyr Gln Lys Lys Pro Gly Gln
        35                  40                  45

Pro Pro Lys Leu Leu Ile Ser Trp Ala Phe Thr Arg Lys Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Ser Asn Tyr Pro Trp Thr Phe Gly Gln Gly Thr Lys Val Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Lys Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210> 19
<211> 448
<212> PRT
<213> Artificial Sequence

<220>
<223> Antibody heavy chain

<400> 19

```
Gln Val Gln Leu Val Gln Ser Gly Ala Glu Val Lys Lys Pro Gly Ala
1               5                   10                  15


Ser Val Lys Val Ser Cys Lys Ala Ser Gly Tyr Ser Phe Thr Gly Tyr
                20                  25                  30


Phe Ile Asn Trp Val Arg Glu Ala Pro Gly Gln Gly Leu Glu Trp Met
        35                  40                  45


Gly His Ile Ser Ser Ser Tyr Ala Thr Ser Thr Tyr Asn Gln Lys Phe
    50                  55                  60


Gln Gly Arg Val Thr Phe Thr Val Asp Thr Ser Ser Ser Thr Ala Tyr
65                  70                  75                  80


Met Glu Leu Ser Ser Leu Arg Ser Glu Asp Thr Ala Val Tyr Tyr Cys
                85                  90                  95


Val Arg Ser Gly Asn Tyr Glu Glu Tyr Ala Met Asp Tyr Trp Gly Gln
                100                 105                 110


Gly Thr Leu Val Thr Val Ser Ser Ala Ser Thr Lys Gly Pro Ser Val
        115                 120                 125


Phe Pro Leu Ala Pro Ser Ser Lys Ser Thr Ser Gly Gly Thr Ala Ala
    130                 135                 140


Leu Gly Cys Leu Val Lys Asp Tyr Phe Pro Glu Pro Val Thr Val Ser
145                 150                 155                 160


Trp Asn Ser Gly Ala Leu Thr Ser Gly Val His Thr Phe Pro Ala Val
                165                 170                 175


Leu Gln Ser Ser Gly Leu Tyr Ser Leu Glu Ser Val Val Thr Val Pro
                180                 185                 190


Ser Ser Ser Leu Gly Thr Gln Thr Tyr Ile Cys Asn Val Asn His Lys
        195                 200                 205
```

```
Pro Ser Asn Thr Lys Val Asp Lys Arg Val Glu Pro Lys Ser Cys Asp
    210                 215             220

Lys Thr His Thr Cys Pro Pro Cys Pro Ala Pro Glu Leu Leu Gly Gly
    225             230             235                 240

Pro Ser Val Phe Leu Phe Pro Pro Lys Pro Lys Asp Thr Leu Met Ile
                245             250                 255

Ser Arg Thr Pro Glu Val Thr Cys Val Val Val Asp Val Ser His Glu
            260             265             270

Asp Pro Glu Val Lys Phe Asn Trp Tyr Val Asp Gly Val Glu Val His
        275             280             285

Asn Ala Lys Thr Lys Pro Arg Glu Glu Gln Tyr Asn Ser Thr Tyr Arg
    290             295             300

Val Val Ser Val Leu Thr Val Leu His Gln Asp Trp Leu Asn Gly Lys
305             310             315                 320

Glu Tyr Lys Cys Lys Val Ser Asn Lys Ala Leu Pro Ala Pro Ile Glu
            325             330             335

Lys Thr Ile Ser Lys Ala Lys Gly Gln Pro Arg Glu Pro Gln Val Tyr
            340             345             350

Thr Leu Pro Pro Ser Arg Glu Glu Met Thr Lys Asn Gln Val Ser Leu
        355             360             365

Thr Cys Leu Val Lys Gly Phe Tyr Pro Ser Asp Ile Ala Val Glu Trp
    370             375             380

Glu Ser Asn Gly Gln Pro Glu Asn Asn Tyr Lys Thr Thr Pro Pro Val
385             390             395                 400

Leu Asp Ser Asp Gly Ser Phe Phe Leu Tyr Ser Lys Leu Thr Val Asp
            405             410             415

Lys Ser Arg Trp Gln Gln Gly Asn Val Phe Ser Cys Ser Val Met His
        420             425             430

Glu Ala Leu His Asn His Tyr Thr Gln Lys Ser Leu Ser Leu Ser Lys
    435             440             445


<210>   20
<211>   223
<212>   PRT
```

<213>    Artificial Sequence

<220>
<223>    Antibody heavy chain

<400>    20

```
Gln Val Gln Leu Val Gln Ser Gly Ala Glu Val Lys Lys Pro Gly Ala
1                   5                   10                  15

Ser Val Lys Val Ser Cys Lys Ala Ser Gly Tyr Ser Phe Thr Gly Tyr
                20                  25                  30

Phe Ile Asn Trp Val Arg Glu Ala Pro Gly Gln Gly Leu Glu Trp Met
            35                  40                  45

Gly His Ile Ser Ser Ser Tyr Ala Thr Ser Thr Tyr Asn Gln Lys Phe
        50                  55                  60

Gln Gly Arg Val Thr Phe Thr Val Asp Thr Ser Ser Ser Thr Ala Tyr
65                  70                  75                  80

Met Glu Leu Ser Ser Leu Arg Ser Glu Asp Thr Ala Val Tyr Tyr Cys
                85                  90                  95

Val Arg Ser Gly Asn Tyr Glu Glu Tyr Ala Met Asp Tyr Trp Gly Gln
                100                 105                 110

Gly Thr Leu Val Thr Val Ser Ser Ala Ser Thr Lys Gly Pro Ser Val
            115                 120                 125

Phe Pro Leu Ala Pro Ser Ser Lys Ser Thr Ser Gly Gly Thr Ala Ala
        130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Glu Ser Val Val Thr Val Pro
                180                 185                 190

Ser Ser Ser Leu Gly Thr Gln Thr Tyr Ile Cys Asn Val Asn His Lys
            195                 200                 205

Pro Ser Asn Thr Lys Val Asp Lys Arg Val Glu Pro Lys Ser Cys
        210                 215                 220
```

```
<210>  21
<211>  10
<212>  PRT
<213>  Artificial Sequence

<220>
<223>  Antibody fragment

<400>  21

Gly Phe Asn Ile Lys Asp Thr Tyr Ile His
1               5                   10


<210>  22
<211>  17
<212>  PRT
<213>  Artificial Sequence

<220>
<223>  Antibody fragment

<400>  22

Arg Ile Tyr Pro Thr Asn Gly Tyr Thr Arg Tyr Ala Asp Ser Val Lys
1               5                   10                  15

Gly


<210>  23
<211>  11
<212>  PRT
<213>  Artificial Sequence

<220>
<223>  Antibody fragment

<400>  23

Trp Gly Gly Asp Gly Phe Tyr Ala Met Asp Tyr
1               5                   10


<210>  24
<211>  11
<212>  PRT
<213>  Artificial Sequence

<220>
<223>  Antibody fragment

<400>  24

Arg Ala Ser Gln Asp Val Asn Thr Ala Val Ala
1               5                   10


<210>  25
<211>  7
<212>  PRT
```

```
<213>   Artificial Sequence

<220>
<223>   Antibody fragment

<400>   25

Ser Ala Ser Phe Leu Tyr Ser
1               5


<210>   26
<211>   9
<212>   PRT
<213>   Artificial Sequence

<220>
<223>   Antibody fragment

<400>   26

Gln Gln His Tyr Thr Thr Pro Pro Thr
1               5


<210>   27
<211>   10
<212>   PRT
<213>   Artificial Sequence

<220>
<223>   Antibody fragment

<400>   27

Gly Tyr Ser Phe Thr Gly Tyr Phe Ile Asn
1               5                   10


<210>   28
<211>   17
<212>   PRT
<213>   Artificial Sequence

<220>
<223>   Antibody fragment

<400>   28

His Ile Ser Ser Ser Tyr Ala Thr Ser Thr Tyr Asn Gln Lys Phe Gln
1               5                   10                  15


Gly


<210>   29
<211>   11
<212>   PRT
<213>   Artificial Sequence

<220>
```

<223> Antibody fragment

<400> 29

Ser Gly Asn Tyr Glu Glu Tyr Ala Met Asp Tyr
1               5                   10

<210> 30
<211> 17
<212> PRT
<213> Artificial Sequence

<220>
<223> Antibody construct

<400> 30

Arg Ser Ser Gln Thr Leu Leu Tyr Ser Asn Asn Gln Lys Asn Tyr Leu
1               5                   10                  15

Ala

<210> 31
<211> 7
<212> PRT
<213> Artificial Sequence

<220>
<223> Antibody fragment

<400> 31

Trp Ala Phe Thr Arg Lys Ser
1               5

<210> 32
<211> 9
<212> PRT
<213> Artificial Sequence

<220>
<223> Antibody fragment

<400> 32

Gln Gln Tyr Ser Asn Tyr Pro Trp Thr
1               5

<210> 33
<211> 10
<212> PRT
<213> Artificial Sequence

<220>
<223> Antibody fragment

<400> 33

Gly Tyr Ser Phe Thr Gly Tyr Trp Met Asn
1               5                   10

<210>   34
<211>   16
<212>   PRT
<213>   Artificial Sequence

<220>
<223>   Antibody fragment

<400>   34

Met Ile His Pro Leu Asp Ala Glu Ile Arg Ala Asn Gln Lys Phe Arg
1               5                   10              15

<210>   35
<211>   9
<212>   PRT
<213>   Artificial Sequence

<220>
<223>   Antibody fragment

<400>   35

Gly Thr Tyr Asp Gly Gly Phe Glu Tyr
1               5

<210>   36
<211>   15
<212>   PRT
<213>   Artificial Sequence

<220>
<223>   Antibody fragment

<400>   36

Arg Ala Ser Gln Ser Val Ser Gly Ser Arg Phe Thr Tyr Met His
1               5                   10              15

<210>   37
<211>   7
<212>   PRT
<213>   Artificial Sequence

<220>
<223>   Antibody fragment

<400>   37

Tyr Ala Ser Ile Leu Glu Ser
1               5

<210>   38
<211>   10

<212> PRT
<213> Artificial Sequence

<220>
<223> Antibody fragment

<400> 38

Gln His Ser Trp Glu Ile Pro Pro Trp Thr
1               5                   10


<210> 39
<211> 220
<212> PRT
<213> Artificial Sequence

<220>
<223> Antibody light chain

<400> 39

Asp Ile Val Leu Thr Gln Ser Pro Asp Ser Leu Ala Val Ser Leu Gly
1               5                   10                  15


Glu Arg Ala Thr Ile Asn Cys Arg Ser Ser Gln Thr Leu Leu Tyr Ser
            20                  25                  30


Asn Asn Gln Lys Asn Tyr Leu Ala Trp Tyr Gln Gln Lys Pro Gly Gln
            35                  40                  45


Pro Pro Lys Leu Leu Ile Ser Trp Ala Phe Thr Arg Lys Ser Gly Val
            50                  55                  60


Pro Asp Arg Phe Ser Gly Ser Gly Ser Gly Thr Asp Phe Thr Leu Thr
65                  70                  75                  80


Ile Ser Ser Leu Gln Ala Glu Asp Val Ala Val Tyr Tyr Cys Gln Gln
                85                  90                  95


Tyr Ser Asn Tyr Pro Trp Thr Phe Gly Gln Gly Thr Lys Val Glu Ile
                100                 105                 110


Lys Arg Thr Val Ala Ala Pro Ser Val Phe Ile Phe Pro Pro Ser Asp
            115                 120                 125


Glu Gln Leu Lys Ser Gly Thr Ala Ser Val Val Cys Leu Leu Asn Asn
            130                 135                 140


Phe Tyr Pro Arg Glu Ala Lys Val Gln Trp Lys Val Asp Asn Ala Leu
145                 150                 155                 160


Gln Ser Gly Asn Ser Gln Glu Ser Val Thr Glu Gln Asp Ser Lys Asp

                165                     170                     175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
            195                 200                 205

Ser Pro Val Thr Lys Ser Phe Asn Arg Gly Glu Cys
    210                 215                 220

<210>  40
<211>  223
<212>  PRT
<213>  Artificial Sequence

<220>
<223>  Antibody heavy chain

<400>  40

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Gly Tyr
            20                  25                  30

Phe Ile Asn Trp Val Arg Gln Ala Pro Gly Gln Gly Leu Glu Trp Met
            35                  40                  45

Gly His Ile Ser Ser Ser Tyr Ala Thr Ser Thr Tyr Asn Gln Lys Phe
            50                  55                  60

Gln Gly Arg Val Thr Phe Thr Val Asp Thr Ser Ser Ser Thr Ala Tyr
65                  70                  75                  80

Met Glu Leu Ser Ser Leu Arg Ser Glu Asp Thr Ala Val Tyr Tyr Cys
            85                  90                  95

Val Arg Ser Gly Asn Tyr Glu Glu Tyr Ala Met Asp Tyr Trp Gly Gln
            100                 105                 110

Gly Thr Leu Val Thr Val Ser Ser Ala Ser Thr Lys Gly Pro Ser Val
            115                 120                 125

Phe Pro Leu Ala Pro Ser Ser Lys Ser Thr Ser Gly Gly Thr Ala Ala
            130                 135                 140

Leu Gly Cys Leu Val Lys Asp Tyr Phe Pro Glu Pro Val Thr Val Ser
145                 150                 155                 160

53

```
Trp Asn Ser Gly Ala Leu Thr Ser Gly Val His Thr Phe Pro Ala Val
                165                 170                 175


Leu Gln Ser Ser Gly Leu Tyr Ser Leu Ser Ser Val Val Thr Val Pro
                180                 185                 190


Ser Ser Ser Leu Gly Thr Gln Thr Tyr Ile Cys Asn Val Asn His Lys
                195                 200                 205


Pro Ser Asn Thr Lys Val Asp Lys Arg Val Glu Pro Lys Ser Cys
    210                 215                 220
```

```
<210>  41
<211>  220
<212>  PRT
<213>  Artificial Sequence

<220>
<223>  Antibody light chain

<400>  41
```

```
Asp Ile Val Leu Thr Gln Ser Pro Asp Ser Leu Ala Val Ser Leu Gly
1                   5                   10                  15


Glu Arg Ala Thr Ile Asn Cys Arg Ser Ser Gln Pro Leu Glu Tyr Ser
                20                  25                  30


Asn Asn Gln Trp Asn Tyr Leu Ala Trp Tyr Gln Lys Lys Pro Gly Gln
                35                  40                  45


Pro Pro Lys Leu Leu Ile Ser Trp Ala Phe Thr Arg Lys Ser Gly Val
    50                  55                  60


Pro Asp Arg Phe Ser Gly Ser Gly Ser Gly Thr Asp Phe Thr Leu Thr
65                  70                  75                  80


Ile Ser Ser Leu Gln Ala Glu Asp Val Ala Val Tyr Tyr Cys Gly Gln
                85                  90                  95


Tyr Ser Asp Tyr Pro Asn Thr Phe Gly Gln Gly Thr Lys Val Glu Ile
                100                 105                 110


Lys Arg Thr Val Ala Ala Pro Ser Val Phe Ile Phe Pro Pro Ser Asp
                115                 120                 125


Glu Gln Leu Lys Ser Gly Thr Ala Ser Val Lys Cys Leu Leu Asn Asn
    130                 135                 140
```

```
Phe Tyr Pro Arg Glu Ala Lys Val Gln Trp Lys Val Asp Asn Ala Leu
145             150             155             160

Gln Ser Gly Asn Ser Gln Glu Ser Val Thr Glu Gln Asp Ser Lys Asp
            165             170             175

Ser Thr Tyr Ser Leu Ser Ser Thr Leu Thr Leu Ser Lys Ala Asp Tyr
            180             185             190

Glu Lys His Lys Val Tyr Ala Cys Glu Val Thr His Gln Gly Leu Ser
            195             200             205

Ser Pro Val Thr Lys Ser Phe Asn Arg Gly Glu Cys
    210             215             220


<210>  42
<211>  448
<212>  PRT
<213>  Artificial Sequence

<220>
<223>  Antibody heavy chain

<400>  42

Gln Val Gln Leu Val Gln Ser Gly Ala Glu Val Lys Lys Pro Gly Ala
1           5               10              15

Ser Val Lys Val Ser Cys Lys Ala Ser Gly Tyr Pro Phe Thr Gln Tyr
            20              25              30

Phe Ile His Trp Val Arg Glu Ala Pro Gly Gln Gly Leu Glu Trp Met
            35              40              45

Gly His Ile Ser Ser Ser Tyr Ala Thr Val Asp Tyr Asn Gln Lys Phe
            50              55              60

Gln Gly Arg Val Thr Phe Thr Val Asp Thr Ser Ser Ser Thr Ala Tyr
65              70              75              80

Met Glu Leu Ser Ser Leu Arg Ser Glu Asp Thr Ala Val Tyr Tyr Cys
                85              90              95

Val Arg Ser Gly Asn Tyr Glu Glu Tyr Ala Met Asp Tyr Trp Gly Gln
            100             105             110

Gly Thr Leu Val Thr Val Ser Ser Ala Ser Thr Lys Gly Pro Ser Val
            115             120             125
```

```
Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
    145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Glu Ser Val Val Thr Val Pro
                180                 185                 190

Ser Ser Ser Leu Gly Thr Gln Thr Tyr Ile Cys Asn Val Asn His Lys
                195                 200                 205

Pro Ser Asn Thr Lys Val Asp Lys Arg Val Glu Pro Lys Ser Cys Asp
    210                 215                 220

Lys Thr His Thr Cys Pro Pro Cys Pro Ala Pro Glu Leu Leu Gly Gly
    225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
                260                 265                 270

Asp Pro Glu Val Lys Phe Asn Trp Tyr Val Asp Gly Val Glu Val His
    275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
    305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
                340                 345                 350

Thr Leu Pro Pro Ser Arg Glu Glu Met Thr Lys Asn Gln Val Ser Leu
    355                 360                 365

Thr Cys Leu Val Lys Gly Phe Tyr Pro Ser Asp Ile Ala Val Glu Trp
    370                 375                 380
```

Glu Ser Asn Gly Gln Pro Glu Asn Asn Tyr Lys Thr Thr Pro Pro Val
385             390             395             400

Leu Asp Ser Asp Gly Ser Phe Phe Leu Tyr Ser Lys Leu Thr Val Asp
            405             410             415

Lys Ser Arg Trp Gln Gln Gly Asn Val Phe Ser Cys Ser Val Met His
            420             425             430

Glu Ala Leu His Asn His Tyr Thr Gln Lys Ser Leu Ser Leu Ser Lys
        435             440             445

<210>   43
<211>   107
<212>   PRT
<213>   Artificial Sequence

<220>
<223>   Antibody light chain

<400>   43

Asp Ile Gln Met Thr Gln Ser Pro Ser Ser Leu Ser Ala Ser Val Gly
1                   5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
        50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys
            100                 105

<210>   44
<211>   120
<212>   PRT
<213>   Artificial Sequence

<220>
<223>   Antibody heavy chain

57

<400> 44

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Ala Tyr Thr Arg Tyr Ala Asp Ser Val
        50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Thr Gly Phe Tyr Ala Met Asp Tyr Trp Gly Gln
            100                 105                 110

Gly Thr Leu Val Thr Val Ser Ser
            115                 120

<210> 45
<211> 217
<212> PRT
<213> Artificial Sequence

<220>
<223> Antibody fragment

<400> 45

Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
        50                  55                  60

Gln Tyr Asn Ser Thr Tyr Arg Val Val Ser Val Leu Thr Val Leu His
65                  70                  75                  80

```
Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
               100                 105                 110

Pro Arg Glu Pro Gln Val Tyr Thr Leu Pro Pro Ser Arg Glu Glu Met
           115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
               165                 170                 175

Tyr Ser Lys Leu Thr Val Asp Lys Ser Arg Trp Gln Gln Gly Asn Val
           180                 185                 190

Phe Ser Cys Ser Val Met His Glu Ala Leu His Asn His Tyr Thr Gln
           195                 200                 205

Lys Ser Leu Ser Leu Ser Pro Gly Lys
    210                 215
```

```
<210>  46
<211>  217
<212>  PRT
<213>  Artificial Sequence

<220>
<223>  Antibody fragment

<400>  46
```

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
            35                  40                  45

Val Asp Gly Val Glu Val His Asn Ala Lys Thr Lys Pro Arg Glu Glu
    50                  55                  60
```

```
Gln Tyr Asn Ser Thr Tyr Arg Val Val Ser Val Leu Thr Val Leu His
65              70              75              80

Gln Asp Trp Leu Asn Gly Lys Glu Tyr Lys Cys Lys Val Ser Asn Lys
            85              90              95

Ala Leu Pro Ala Pro Ile Glu Lys Thr Ile Ser Lys Ala Lys Gly Gln
        100             105             110

Pro Arg Glu Pro Gln Val Tyr Thr Leu Pro Pro Ser Arg Glu Glu Met
        115             120             125

Thr Lys Asn Gln Val Ser Leu Tyr Cys Leu Val Lys Gly Phe Tyr Pro
        130             135             140

Ser Asp Ile Ala Val Glu Trp Glu Ser Asn Gly Gln Pro Glu Asn Asn
145             150             155             160

Tyr Lys Thr Thr Pro Pro Val Leu Asp Ser Asp Gly Ser Phe Phe Leu
            165             170             175

Tyr Ser Lys Leu Thr Val Asp Lys Ser Arg Trp Gln Gln Gly Asn Val
        180             185             190

Phe Ser Cys Ser Val Met His Glu Ala Leu His Asn His Tyr Thr Gln
        195             200             205

Lys Ser Leu Ser Leu Ser Pro Gly Lys
    210             215
```

```
<210>   47
<211>   217
<212>   PRT
<213>   Artificial Sequence

<220>
<223>   Antibody fragment

<400>   47
```

```
Ala Pro Glu Leu Leu Gly Gly Pro Ser Val Phe Leu Phe Pro Pro Lys
1               5               10              15

Pro Lys Asp Thr Leu Met Ile Ser Arg Thr Pro Glu Val Thr Cys Val
            20              25              30

Val Val Asp Val Ser His Glu Asp Pro Glu Val Lys Phe Asn Trp Tyr
        35              40              45

Val Asp Gly Val Glu Val His Asn Ala Lys Thr Lys Pro Arg Glu Glu
```

```
            50                        55                        60

        Gln Tyr Asn Ser Thr Tyr Arg Val Val Ser Val Leu Thr Val Leu His
        65                  70                  75                  80


        Gln Asp Trp Leu Asn Gly Lys Glu Tyr Lys Cys Lys Val Ser Asn Lys
                        85                  90                  95


        Ala Leu Pro Ala Pro Ile Glu Lys Thr Ile Ser Lys Ala Lys Gly Gln
                        100                 105                 110


        Pro Arg Glu Pro Gln Val Tyr Thr Leu Pro Pro Ser Arg Glu Glu Met
                    115                 120                 125


        Thr Lys Asn Gln Val Ser Leu Thr Cys Leu Val Lys Gly Phe Tyr Pro
                    130                 135                 140


        Ser Asp Ile Ala Val Glu Trp Glu Ser Asn Gly Gln Pro Glu Asn Asn
        145                 150                 155                 160


        Tyr Lys Thr Thr Pro Pro Val Leu Asp Ser Asp Gly Ser Phe Phe Leu
                        165                 170                 175


        Thr Ser Lys Leu Thr Val Asp Lys Ser Arg Trp Gln Gln Gly Asn Val
                    180                 185                 190


        Phe Ser Cys Ser Val Met His Glu Ala Leu His Asn His Tyr Thr Gln
                    195                 200                 205


        Lys Ser Leu Ser Leu Ser Pro Gly Lys
            210                 215


        <210>  48
        <211>  217
        <212>  PRT
        <213>  Artificial Sequence

        <220>
        <223>  Antibody fragment

        <400>  48

        Ala Pro Glu Leu Leu Gly Gly Pro Ser Val Phe Leu Phe Pro Pro Lys
        1                   5                   10                  15


        Pro Lys Asp Thr Leu Met Ile Ser Arg Thr Pro Glu Val Thr Cys Val
                        20                  25                  30


        Val Val Asp Val Ser His Glu Asp Pro Glu Val Lys Phe Asn Trp Tyr
                    35                  40                  45
```

```
Val Asp Gly Val Glu Val His Asn Ala Lys Thr Lys Pro Arg Glu Glu
    50              55              60

Gln Tyr Asn Ser Thr Tyr Arg Val Val Ser Val Leu Thr Val Leu His
65              70              75              80

Gln Asp Trp Leu Asn Gly Lys Glu Tyr Lys Cys Lys Val Ser Asn Lys
            85              90              95

Ala Leu Pro Ala Pro Ile Glu Lys Thr Ile Ser Lys Ala Lys Gly Gln
            100             105             110

Pro Arg Glu Pro Gln Val Tyr Thr Leu Pro Pro Cys Arg Glu Glu Met
        115             120             125

Thr Lys Asn Gln Val Ser Leu Trp Cys Leu Val Lys Gly Phe Tyr Pro
    130             135             140

Ser Asp Ile Ala Val Glu Trp Glu Ser Asn Gly Gln Pro Glu Asn Asn
145             150             155             160

Tyr Lys Thr Thr Pro Pro Val Leu Asp Ser Asp Gly Ser Phe Phe Leu
            165             170             175

Tyr Ser Lys Leu Thr Val Asp Lys Ser Arg Trp Gln Gln Gly Asn Val
            180             185             190

Phe Ser Cys Ser Val Met His Glu Ala Leu His Asn His Tyr Thr Gln
        195             200             205

Lys Ser Leu Ser Leu Ser Pro Gly Lys
    210             215
```

<210> 49
<211> 217
<212> PRT
<213> Artificial Sequence

<220>
<223> Antibody fragment

<400> 49

```
Ala Pro Glu Leu Leu Gly Gly Pro Ser Val Phe Leu Phe Pro Pro Lys
1               5               10              15

Pro Lys Asp Thr Leu Met Ile Ser Arg Thr Pro Glu Val Thr Cys Val
            20              25              30
```

```
Val Val Asp Val Ser His Glu Asp Pro Glu Val Lys Phe Asn Trp Tyr
        35              40              45

Val Asp Gly Val Glu Val His Asn Ala Lys Thr Lys Pro Arg Glu Glu
        50              55              60

Gln Tyr Asn Ser Thr Tyr Arg Val Val Ser Val Leu Thr Val Leu His
65              70              75              80

Gln Asp Trp Leu Asn Gly Lys Glu Tyr Lys Cys Lys Val Ser Asn Lys
            85              90              95

Ala Leu Pro Ala Pro Ile Glu Lys Thr Ile Ser Lys Ala Lys Gly Gln
        100             105             110

Pro Arg Glu Pro Gln Val Cys Thr Leu Pro Pro Ser Arg Glu Glu Met
        115             120             125

Thr Lys Asn Gln Val Ser Leu Ser Cys Ala Val Lys Gly Phe Tyr Pro
        130             135             140

Ser Asp Ile Ala Val Glu Trp Glu Ser Asn Gly Gln Pro Glu Asn Asn
145             150             155             160

Tyr Lys Thr Thr Pro Pro Val Leu Asp Ser Asp Gly Ser Phe Phe Leu
            165             170             175

Val Ser Lys Leu Thr Val Asp Lys Ser Arg Trp Gln Gln Gly Asn Val
        180             185             190

Phe Ser Cys Ser Val Met His Glu Ala Leu His Asn His Tyr Thr Gln
        195             200             205

Lys Ser Leu Ser Leu Ser Pro Gly Lys
    210             215
```

<210> 50
<211> 220
<212> PRT
<213> Artificial Sequence

<220>
<223> Antibody light chain

<400> 50

```
Asp Ile Gln Leu Thr Gln Ser Pro Ser Ser Leu Ser Ala Ser Val Gly
1               5               10              15
```

```
Asp Arg Val Thr Ile Thr Cys Arg Ser Ser Gln Thr Leu Leu Tyr Ser
              20                  25                  30

Asn Asn Gln Lys Asn Tyr Leu Ala Trp Tyr Gln Gln Lys Pro Gly Lys
              35                  40                  45

Ala Pro Lys Leu Leu Ile Ser Trp Ala Phe Thr Arg Lys Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Gln Gln
              85                  90                  95

Tyr Ser Asn Tyr Pro Trp Thr Phe Gly Gln Gly Thr Lys Val Glu Ile
              100                 105                 110

Lys Arg Thr Val Ala Ala Pro Ser Val Phe Ile Phe Pro Pro Ser Asp
              115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
              165                 170                 175

Ser Thr Tyr Ser Leu Ser Ser Thr Leu Thr Leu Ser Lys Ala Asp Tyr
              180                 185                 190

Glu Lys His Lys Val Tyr Ala Cys Glu Val Thr His Gln Gly Leu Ser
              195                 200                 205

Ser Pro Val Thr Lys Ser Phe Asn Arg Gly Glu Cys
    210                 215                 220
```

<210> 51
<211> 223
<212> PRT
<213> Artificial Sequence

<220>
<223> Antibody heavy chain

<400> 51

```
Glu Val Gln Leu Val Gln Ser Gly Pro Glu Leu Val Gln Pro Gly Gly
```

EP 3 733 714 A1

|   |   |   |   |   |   |   |   |   |   |   |   |   |   |   |
|---|---|---|---|---|---|---|---|---|---|---|---|---|---|---|
| 1 |   |   |   | 5 |   |   |   |   | 10 |   |   |   |   | 15 |

Ser Val Arg Ile Ser Cys Ala Ala Ser Gly Tyr Ser Phe Thr Gly Tyr
        20              25                  30

Phe Ile Asn Trp Val Lys Gln Ala Pro Gly Lys Gly Leu Glu Trp Ile
        35              40                  45

Ser His Ile Ser Ser Ser Tyr Ala Thr Ser Thr Tyr Asn Gln Ser Phe
        50              55                  60

Lys Gly Arg Ala Thr Phe Ser Val Asp Thr Ser Ser Ser Thr Ala Tyr
65              70              75                  80

Met Gln Leu Asn Ser Leu Arg Ala Glu Asp Thr Ala Val Tyr Tyr Cys
                85              90                  95

Val Arg Ser Gly Asn Tyr Glu Glu Tyr Ala Met Asp Tyr Trp Gly Gln
            100             105             110

Gly Thr Leu Val Thr Val Ser Ser Ala Ser Thr Lys Gly Pro Ser Val
        115             120             125

Phe Pro Leu Ala Pro Ser Ser Lys Ser Thr Ser Gly Gly Thr Ala Ala
        130             135             140

Leu Gly Cys Leu Val Lys Asp Tyr Phe Pro Glu Pro Val Thr Val Ser
145             150             155             160

Trp Asn Ser Gly Ala Leu Thr Ser Gly Val His Thr Phe Pro Ala Val
            165             170             175

Leu Gln Ser Ser Gly Leu Tyr Ser Leu Ser Ser Val Val Thr Val Pro
            180             185             190

Ser Ser Ser Leu Gly Thr Gln Thr Tyr Ile Cys Asn Val Asn His Lys
        195             200             205

Pro Ser Asn Thr Lys Val Asp Lys Arg Val Glu Pro Lys Ser Cys
        210             215             220

<210>   52
<211>   222
<212>   PRT
<213>   Artificial Sequence

<220>
<223>   Antibody heavy chain

65

<400> 52

Val Gln Leu Val Glu Ser Gly Gly Gly Leu Val Gln Pro Gly Gly Ser
1               5                   10                  15

Leu Arg Leu Ser Cys Ala Ala Ser Gly Tyr Ser Phe Thr Gly Tyr Phe
            20                  25                  30

Ile Asn Trp Val Arg Gln Ala Pro Gly Lys Gly Leu Glu Trp Val Ser
        35                  40                  45

His Ile Ser Ser Ser Tyr Ala Thr Ser Thr Tyr Asn Gln Ser Val Lys
    50                  55                  60

Gly Arg Phe Thr Phe Ser Val Asp Thr Ser Ser Ser Thr Ala Tyr Leu
65                  70                  75                  80

Gln Met Asn Ser Leu Arg Ala Glu Asp Thr Ala Val Tyr Tyr Cys Val
            85                  90                  95

Arg Ser Gly Asn Tyr Glu Glu Tyr Ala Met Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
            165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys
    210                 215                 220

<210> 53
<211> 120
<212> PRT
<213> Artificial Sequence

<220>
<223>  Antibody heavy chain

<400>   53

Gln Val Gln Leu Val Gln Ser Gly Ala Glu Val Lys Lys Pro Gly Ala
1                   5                   10                  15

Ser Val Lys Val Ser Cys Lys Ala Ser Gly Phe Asn Ile Lys Asp Thr
            20                  25                  30

Tyr Ile His Trp Val Arg Gln Ala Pro Gly Gln Gly Leu Glu Gln Met
            35                  40                  45

Gly Arg Ile Tyr Pro Thr Asn Gly Tyr Thr Arg Tyr Asp Pro Lys Phe
        50                  55                  60

Gln Gly Arg Val Thr Ile Thr Ala Asp Thr Ser Ser Asn Thr Ala Tyr
65                  70                  75                  80

Met Glu Leu Ser Ser Leu Arg Ser Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser
        115                 120


<210>   54
<211>   120
<212>   PRT
<213>   Artificial Sequence

<220>
<223>  Antibody heavy chain

<400>   54

Glu Val Gln Leu Val Gln Ser Gly Pro Glu Leu Val Gln Pro Gly Gly
1                   5                   10                  15

Ser Leu Arg Leu Ser Cys Ala Ala Ser Gly Phe Asn Ile Lys Asp Thr
            20                  25                  30

Tyr Ile His Trp Val Lys Gln Ala Pro Gly Lys Gly Leu Glu Trp Ile
            35                  40                  45

Ser Arg Ile Tyr Pro Thr Asn Gly Tyr Thr Arg Tyr Asp Pro Ser Phe
        50                  55                  60

```
Lys Gly Arg Ala Thr Ile Ser Ala Asp Thr Ser Ser Asn Thr Ala Tyr
65               70               75                   80


Leu Gln Val Asn Ser Leu Arg Ala Glu Asp Thr Ala Val Tyr Tyr Cys
                85                   90                   95


Ser Arg Trp Gly Gly Asp Gly Phe Tyr Ala Met Asp Tyr Trp Gly Gln
            100              105              110


Gly Thr Leu Val Thr Val Ser Ser
        115              120


<210>   55
<211>   5
<212>   PRT
<213>   Artificial Sequence

<220>
<223>   linker

<400>   55

Gly Gly Gly Gly Gly
1               5


<210>   56
<211>   6
<212>   PRT
<213>   Artificial Sequence

<220>
<223>   linker

<400>   56

Gly Ser Gly Ser Gly Ser
1               5


<210>   57
<211>   11
<212>   PRT
<213>   Artificial Sequence

<220>
<223>   linker

<400>   57

Gly Ser Gly Gly Gly Thr Gly Gly Gly Ser Gly
1               5                   10


<210>   58
<211>   3
<212>   PRT
<213>   Artificial Sequence
```

```
<220>
<223>  linker

<400>  58

Pro Pro Pro
1


<210>  59
<211>  21
<212>  PRT
<213>  Artificial Sequence

<220>
<223>  linker

<400>  59

Ala Ser Pro Ala Ala Pro Ala Pro Ala Ser Pro Ala Ala Pro Ala Pro
1               5                   10                  15


Ser Ala Pro Ala Ala
            20


<210>  60
<211>  21
<212>  PRT
<213>  Artificial Sequence

<220>
<223>  linker

<400>  60

Ala Ser Ala Ala Ala Pro Ala Ala Ala Ser Ala Ala Ala Ser Ala Pro
1               5                   10                  15


Ser Ala Ala Ala Ala
            20


<210>  61
<211>  25
<212>  PRT
<213>  Artificial Sequence

<220>
<223>  linker

<400>  61

Ala Ala Ser Pro Ala Ala Pro Ser Ala Pro Pro Ala Ala Ala Ser Pro
1               5                   10                  15


Ala Ala Pro Ser Ala Pro Pro Ala Ala
            20                  25
```

```
<210>   62
<211>   6
<212>   PRT
<213>   Artificial Sequence

<220>
<223>   linker

<400>   62

Ala Ser Pro Ala Ser Ala
1               5


<210>   63
<211>   9
<212>   PRT
<213>   Artificial Sequence

<220>
<223>   linker

<400>   63

Ala Ser Pro Ala Ser Pro Ala Ser Ala
1               5


<210>   64
<211>   5
<212>   PRT
<213>   Artificial Sequence

<220>
<223>   linker

<400>   64

Pro Ala Gly Ser Pro
1               5


<210>   65
<211>   5
<212>   PRT
<213>   Artificial Sequence

<220>
<223>   linker

<400>   65

Ser Thr Glu Pro Ser
1               5


<210>   66
<211>   5
<212>   PRT
<213>   Artificial Sequence

<220>
<223>   linker
```

<400> 66

Ser Thr Glu Glu Gly
1               5

<210> 67
<211> 5
<212> PRT
<213> Artificial Sequence

<220>
<223> linker

<400> 67

Gly Ser Ala Pro Gly
1               5

<210> 68
<211> 5
<212> PRT
<213> Artificial Sequence

<220>
<223> linker

<400> 68

Gly Ala Ser Thr Pro
1               5

<210> 69
<211> 5
<212> PRT
<213> Artificial Sequence

<220>
<223> linker

<400> 69

Gly Pro Ser Ala Thr
1               5

<210> 70
<211> 220
<212> PRT
<213> Artificial Sequence

<220>
<223> Antibody light chain

<400> 70

Asp Ile Val Met Thr Gln Thr Pro Leu Ser Leu Ser Val Thr Pro Gly
1               5                   10                  15

```
Gln Pro Ala Ser Ile Ser Cys Lys Ser Ser Gln Ser Val Phe Phe Arg
            20              25              30

Ser Asn Asn Lys Asn Ile Leu Ala Trp Tyr Leu Gln Lys Pro Gly Gln
            35              40              45

Pro Pro Gln Leu Leu Ile Tyr Trp Ala Ser Ser Arg Glu Ser Gly Val
    50              55              60

Pro Asp Arg Phe Ser Gly Ser Gly Ser Gly Thr Asp Phe Thr Leu Lys
65              70              75              80

Ile Ser Arg Val Glu Ala Glu Asp Val Gly Val Tyr Tyr Cys Gln Gln
            85              90              95

Tyr Phe Gly Ser Pro Phe Thr Phe Gly Pro Gly Thr Lys Val Asp Ile
            100             105             110

Lys Arg Thr Val Ala Ala Pro Ser Val Phe Ile Phe Pro Pro Ser Asp
        115             120             125

Glu Gln Leu Lys Ser Gly Thr Ala Ser Val Val Cys Leu Leu Asn Asn
    130             135             140

Phe Tyr Pro Arg Glu Ala Lys Val Gln Trp Lys Val Asp Asn Ala Leu
145             150             155             160

Gln Ser Gly Asn Ser Gln Glu Ser Val Thr Glu Gln Asp Ser Lys Asp
            165             170             175

Ser Thr Tyr Ser Leu Ser Ser Thr Leu Thr Leu Ser Lys Ala Asp Tyr
            180             185             190

Glu Lys His Lys Val Tyr Ala Cys Glu Val Thr His Gln Gly Leu Ser
        195             200             205

Ser Pro Val Thr Lys Ser Phe Asn Arg Gly Glu Cys
    210             215             220


<210>  71
<211>  449
<212>  PRT
<213>  Artificial Sequence

<220>
<223>  Antibody heavy chain

<400>  71

Glu Val Gln Leu Val Glu Ser Gly Gly Gly Leu Val Lys Pro Gly Gly
```

72

|     |     |     |     | 1   |     |     |     | 5   |     |     |     | 10  |     |     |     | 15  |
|-----|-----|-----|-----|-----|-----|-----|-----|-----|-----|-----|-----|-----|-----|-----|-----|-----|

Ser Leu Arg Leu Ser Cys Ala Ala Ser Gly Phe Thr Phe Ser Ser Tyr
20              25              30

Ser Met Ser Trp Val Arg Gln Ala Pro Gly Lys Gly Leu Glu Trp Val
35              40              45

Ser Ser Ile Ser Ser Ser Ser Ser Tyr Ile Tyr Tyr Ala Asp Ser Val
50              55              60

Lys Gly Arg Phe Thr Ile Ser Arg Asp Asn Ala Lys Asn Ser Leu Tyr
65              70              75              80

Leu Gln Met Asn Ser Leu Arg Ala Glu Asp Thr Ala Val Tyr Tyr Cys
85              90              95

Ala Arg Gly Gly Asp Ala Tyr Asn Tyr Tyr Tyr Phe Asp Tyr Trp Gly
100             105             110

Gln Gly Thr Leu Val Thr Val Ser Ser Ala Ser Thr Lys Gly Pro Ser
115             120             125

Val Phe Pro Leu Ala Pro Ser Ser Lys Ser Thr Ser Gly Gly Thr Ala
130             135             140

Ala Leu Gly Cys Leu Val Lys Asp Tyr Phe Pro Glu Pro Val Thr Val
145             150             155             160

Ser Trp Asn Ser Gly Ala Leu Thr Ser Gly Val His Thr Phe Pro Ala
165             170             175

Val Leu Gln Ser Ser Gly Leu Tyr Ser Leu Ser Ser Val Val Thr Val
180             185             190

Pro Ser Ser Ser Leu Gly Thr Gln Thr Tyr Ile Cys Asn Val Asn His
195             200             205

Lys Pro Ser Asn Thr Lys Val Asp Lys Arg Val Glu Pro Lys Ser Cys
210             215             220

Asp Lys Thr His Thr Cys Pro Pro Cys Pro Ala Pro Glu Leu Leu Gly
225             230             235             240

Gly Pro Ser Val Phe Leu Phe Pro Pro Lys Pro Lys Asp Thr Leu Met
245             250             255

73

```
Ile Ser Arg Thr Pro Glu Val Thr Cys Val Val Val Asp Val Ser His
            260                 265             270

Glu Asp Pro Glu Val Lys Phe Asn Trp Tyr Val Asp Gly Val Glu Val
            275                 280             285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
            355                 360                 365

Leu Thr Cys Leu Val Lys Gly Phe Tyr Pro Ser Asp Ile Ala Val Glu
            370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
                420                 425                 430

His Glu Ala Leu His Asn His Tyr Thr Gln Lys Ser Leu Ser Leu Ser
                435                 440                 445

Lys
```

```
<210>  72
<211>  217
<212>  PRT
<213>  Artificial Sequence

<220>
<223>  Antibody light chain

<400>  72
```

```
Gln Ser Ala Leu Thr Gln Pro Ala Ser Val Ser Gly Ser Pro Gly Gln
```

```
         1                5                     10                    15

         Ser Ile Thr Ile Ser Cys Thr Gly Thr Ser Ser Asp Val Gly Gly Tyr
                     20                25                30

         Asn Tyr Val Ser Trp Tyr Gln Gln His Pro Gly Lys Ala Pro Lys Leu
                     35                40                45

         Met Ile Tyr Asp Val Ser Lys Arg Pro Ser Gly Val Ser Asn Arg Phe
                     50                55                60

         Ser Gly Ser Lys Ser Gly Asn Thr Ala Ser Leu Thr Ile Ser Gly Leu
         65                70                75                80

         Gln Ala Glu Asp Glu Ala Asp Tyr Tyr Cys Ser Ser Tyr Thr Ser Ser
                     85                90                95

         Ser Thr Leu Val Phe Gly Gly Gly Thr Lys Leu Thr Val Leu Gly Thr
                     100               105               110

         Val Ala Ala Pro Ser Val Phe Ile Phe Pro Pro Ser Asp Glu Gln Leu
                     115               120               125

         Lys Ser Gly Thr Ala Ser Val Val Cys Leu Leu Asn Asn Phe Tyr Pro
                     130               135               140

         Arg Glu Ala Lys Val Gln Trp Lys Val Asp Asn Ala Leu Gln Ser Gly
         145               150               155               160

         Asn Ser Gln Glu Ser Val Thr Glu Gln Asp Ser Lys Asp Ser Thr Tyr
                     165               170               175

         Ser Leu Ser Ser Thr Leu Thr Leu Ser Lys Ala Asp Tyr Glu Lys His
                     180               185               190

         Lys Val Tyr Ala Cys Glu Val Thr His Gln Gly Leu Ser Ser Pro Val
                     195               200               205

         Thr Lys Ser Phe Asn Arg Gly Glu Cys
                     210               215
```

<210> 73
<211> 445
<212> PRT
<213> Artificial Sequence

<220>
<223> Antibody heavy chain

<400> 73

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Glu Gly Asp Gly Ala Phe Asp Tyr Trp Gly Gln Gly Thr Leu
            100                 105                 110

Val Thr Val Ser Ser Ala Ser Thr Lys Gly Pro Ser Val Phe Pro Leu
            115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
    195                 200                 205

Thr Lys Val Asp Lys Lys Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr

76

245                          250                          255

Pro Glu Val Thr Cys Val Val Val Asp Val Ser His Glu Asp Pro Glu
        260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
        290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
        325                 330                 335

Ser Lys Ala Lys Gly Gln Pro Arg Glu Pro Gln Val Tyr Thr Leu Pro
        340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
        370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
        405                 410                 415

Trp Gln Gln Gly Asn Val Phe Ser Cys Ser Val Met His Glu Ala Leu
        420                 425                 430

His Asn His Tyr Thr Gln Lys Ser Leu Ser Leu Ser Lys
        435                 440                 445

<210> 74
<211> 214
<212> PRT
<213> Artificial Sequence

<220>
<223> antibody light chain

<400> 74

Asp Ile Gln Met Thr Gln Ser Pro Ser Ser Leu Ser Ala Ser Val Gly
1               5               10                  15

```
Asp Arg Val Thr Ile Thr Cys Arg Ala Ser Gln Ser Ile Ser Ser Tyr
            20                  25              30

Leu Asn Trp Tyr Gln Gln Lys Pro Gly Lys Ala Pro Lys Leu Leu Ile
            35                  40              45

Tyr Ala Ala Ser Ser Leu Gln Ser Gly Val Pro Ser Arg Phe Ser Gly
        50              55                  60

Ser Gly Ser Gly Thr Asp Phe Thr Leu Thr Ile Ser Ser Leu Gln Pro
65              70                  75                      80

Glu Asp Phe Ala Thr Tyr Tyr Cys Gln Gln Ser Tyr Ser Thr Pro Pro
                85                  90                  95

Thr Phe Gly Gln Gly Thr Lys Val Glu Ile Lys Arg Thr Val Ala Ala
            100                 105             110

Pro Ser Val Phe Ile Phe Pro Pro Ser Asp Glu Gln Leu Lys Ser Gly
        115                 120                 125

Thr Ala Ser Val Lys Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185             190

Ala Cys Glu Val Thr His Gln Gly Leu Ser Ser Pro Val Thr Lys Ser
        195                 200                 205

Phe Asn Arg Gly Glu Cys
        210


<210>   75
<211>   449
<212>   PRT
<213>   Artificial Sequence

<220>
<223>   Antibody heavy chain

<400>   75
```

Gln Val Gln Leu Val Gln Ser Gly Ala Glu Val Lys Lys Pro Gly Ala
1               5               10              15

Ser Val Lys Leu Ser Cys Lys Ala Ser Gly Tyr Thr Phe Thr Ala Tyr
            20              25              30

Tyr Ile Asn Trp Val Arg Gln Ala Pro Gly Gln Gly Leu Glu Trp Ile
        35              40              45

Gly Arg Ile Tyr Pro Gly Ser Gly Tyr Thr Ser Tyr Ala Gln Lys Phe
    50              55              60

Gln Gly Arg Ala Thr Leu Thr Ala Asp Glu Ser Thr Ser Thr Ala Tyr
65              70              75              80

Met Glu Leu Ser Ser Leu Arg Ser Glu Asp Thr Ala Val Tyr Phe Cys
            85              90              95

Ala Arg Pro Pro Val Tyr Tyr Asp Ser Ala Trp Phe Ala Tyr Trp Gly
        100             105             110

Gln Gly Thr Leu Val Thr Val Ser Ser Ala Ser Thr Lys Gly Pro Ser
        115             120             125

Val Phe Pro Leu Ala Pro Ser Ser Lys Ser Thr Ser Gly Gly Thr Ala
    130             135             140

Ala Leu Gly Cys Leu Val Lys Asp Tyr Phe Pro Glu Pro Val Thr Val
145             150             155             160

Ser Trp Asn Ser Gly Ala Leu Thr Ser Gly Val His Thr Phe Pro Ala
            165             170             175

Val Leu Gln Ser Ser Gly Leu Tyr Ser Leu Glu Ser Val Val Thr Val
        180             185             190

Pro Ser Ser Ser Leu Gly Thr Gln Thr Tyr Ile Cys Asn Val Asn His
    195             200             205

Lys Pro Ser Asn Thr Lys Val Asp Lys Arg Val Glu Pro Lys Ser Cys
    210             215             220

Asp Lys Thr His Thr Cys Pro Pro Cys Pro Ala Pro Glu Leu Leu Gly
225             230             235             240

Gly Pro Ser Val Phe Leu Phe Pro Pro Lys Pro Lys Asp Thr Leu Met
            245             250             255

```
Ile Ser Arg Thr Pro Glu Val Thr Cys Val Val Val Asp Val Ser His
            260             265             270

Glu Asp Pro Glu Val Lys Phe Asn Trp Tyr Val Asp Gly Val Glu Val
            275             280             285

His Asn Ala Lys Thr Lys Pro Arg Glu Glu Gln Tyr Asn Ser Thr Tyr
    290             295             300

Arg Val Val Ser Val Leu Thr Val Leu His Gln Asp Trp Leu Asn Gly
305             310             315             320

Lys Glu Tyr Lys Cys Lys Val Ser Asn Lys Ala Leu Pro Ala Pro Ile
                325             330             335

Glu Lys Thr Ile Ser Lys Ala Lys Gly Gln Pro Arg Glu Pro Gln Val
            340             345             350

Tyr Thr Leu Pro Pro Ser Arg Glu Glu Met Thr Lys Asn Gln Val Ser
            355             360             365

Leu Thr Cys Leu Val Lys Gly Phe Tyr Pro Ser Asp Ile Ala Val Glu
    370             375             380

Trp Glu Ser Asn Gly Gln Pro Glu Asn Asn Tyr Lys Thr Thr Pro Pro
385             390             395             400

Val Leu Asp Ser Asp Gly Ser Phe Phe Leu Tyr Ser Lys Leu Thr Val
            405             410             415

Asp Lys Ser Arg Trp Gln Gln Gly Asn Val Phe Ser Cys Ser Val Met
            420             425             430

His Glu Ala Leu His Asn His Tyr Thr Gln Lys Ser Leu Ser Leu Ser
    435             440             445

Lys
```

```
<210>  76
<211>  220
<212>  PRT
<213>  Artificial Sequence

<220>
<223>  Antibody light chain

<400>  76
```

```
Asp Ile Val Leu Thr Gln Ser Pro Asp Ser Leu Ala Val Ser Leu Gly
1               5                   10                  15

Glu Arg Ala Thr Ile Asn Cys Arg Ser Ser Gln Thr Leu Leu Tyr Ser
            20                  25                  30

Asn Asn Gln Lys Asn Tyr Leu Ala Trp Tyr Gln Lys Lys Pro Gly Gln
        35                  40                  45

Pro Pro Lys Leu Leu Ile Ser Trp Ala Phe Thr Arg Lys Ser Gly Val
        50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Ser Asn Tyr Pro Trp Thr Phe Gly Gln Gly Thr Lys Val Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Lys Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
            165                 170                 175

Ser Thr Tyr Ser Leu Ser Ser Thr Leu Thr Leu Ser Lys Ala Asp Tyr
        180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220
```

```
<210>   77
<211>   448
<212>   PRT
<213>   Artificial Sequence

<220>
```

<223> Antibody heavy chain

<400> 77

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Gly Tyr
            20                  25                  30

Phe Ile Asn Trp Val Arg Glu Ala Pro Gly Gln Gly Leu Glu Trp Met
            35                  40                  45

Gly His Ile Ser Ser Ser Tyr Ala Thr Ser Thr Tyr Asn Gln Lys Phe
        50                  55                  60

Gln Gly Arg Val Thr Phe Thr Val Asp Thr Ser Ser Ser Thr Ala Tyr
65                  70                  75                  80

Met Glu Leu Ser Ser Leu Arg Ser Glu Asp Thr Ala Val Tyr Tyr Cys
                85                  90                  95

Val Arg Ser Gly Asn Tyr Glu Glu Tyr Ala Met Asp Tyr Trp Gly Gln
            100                 105                 110

Gly Thr Leu Val Thr Val Ser Ser Ala Ser Thr Lys Gly Pro Ser Val
            115                 120                 125

Phe Pro Leu Ala Pro Ser Ser Lys Ser Thr Ser Gly Gly Thr Ala Ala
        130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Glu Ser Val Val Thr Val Pro
            180                 185                 190

Ser Ser Ser Leu Gly Thr Gln Thr Tyr Ile Cys Asn Val Asn His Lys
            195                 200                 205

Pro Ser Asn Thr Lys Val Asp Lys Arg Val Glu Pro Lys Ser Cys Asp
        210                 215                 220

Lys Thr His Thr Cys Pro Pro Cys Pro Ala Pro Glu Leu Leu Gly Gly
225                 230                 235                 240

```
Pro Ser Val Phe Leu Phe Pro Pro Lys Pro Lys Asp Thr Leu Met Ile
                245             250             255

Ser Arg Thr Pro Glu Val Thr Cys Val Val Val Asp Val Ser His Glu
            260             265             270

Asp Pro Glu Val Lys Phe Asn Trp Tyr Val Asp Gly Val Glu Val His
        275             280             285

Asn Ala Lys Thr Lys Pro Arg Glu Glu Gln Tyr Asn Ser Thr Tyr Arg
    290             295             300

Val Val Ser Val Leu Thr Val Leu His Gln Asp Trp Leu Asn Gly Lys
305             310             315             320

Glu Tyr Lys Cys Lys Val Ser Asn Lys Ala Leu Pro Ala Pro Ile Glu
            325             330             335

Lys Thr Ile Ser Lys Ala Lys Gly Gln Pro Arg Glu Pro Gln Val Tyr
            340             345             350

Thr Leu Pro Pro Ser Arg Glu Glu Met Thr Lys Asn Gln Val Ser Leu
        355             360             365

Thr Cys Leu Val Lys Gly Phe Tyr Pro Ser Asp Ile Ala Val Glu Trp
    370             375             380

Glu Ser Asn Gly Gln Pro Glu Asn Asn Tyr Lys Thr Thr Pro Pro Val
385             390             395             400

Leu Asp Ser Asp Gly Ser Phe Phe Leu Tyr Ser Lys Leu Thr Val Asp
            405             410             415

Lys Ser Arg Trp Gln Gln Gly Asn Val Phe Ser Cys Ser Val Met His
            420             425             430

Glu Ala Leu His Asn His Tyr Thr Gln Lys Ser Leu Ser Leu Ser Lys
        435             440             445


<210>  78
<211>  219
<212>  PRT
<213>  Artificial Sequence

<220>
<223>  Antibody light chain

<400>  78
```

```
Asp Ile Val Met Thr Gln Ser Pro Asp Ser Leu Ala Val Ser Leu Gly
1                   5                   10                  15

Glu Arg Ala Thr Ile Asn Cys Arg Ala Ser Gln Ser Val Ser Gly Ser
                20                  25                  30

Arg Phe Thr Tyr Met His Trp Tyr Gln Gln Lys Pro Gly Gln Pro Pro
        35                  40                  45

Lys Leu Leu Ile Lys Tyr Ala Ser Ile Leu Glu Ser Gly Val Pro Asp
        50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln His Ser Trp
                85                  90                  95

Glu Ile Pro Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
        130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
        180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215
```

```
<210>  79
<211>  446
<212>  PRT
<213>  Artificial Sequence

<220>
<223>  Antibody heavy chain
```

84

<400> 79

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Ser Phe Thr Gly Tyr
        20                  25                  30

Trp Met Asn Trp Val Arg Gln Ala Pro Gly Gln Gly Leu Glu Trp Ile
        35                  40                  45

Gly Met Ile His Pro Leu Asp Ala Glu Ile Arg Ala Asn Gln Lys Phe
        50                  55                  60

Arg Asp Arg Val Thr Ile Thr Val Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Arg Gly Thr Tyr Asp Gly Gly Phe Glu Tyr Trp Gly Gln Gly Thr
            100                 105                 110

Leu Val Thr Val Ser Ser Ala Ser Thr Lys Gly Pro Ser Val Phe Pro
            115                 120                 125

Leu Ala Pro Ser Ser Lys Ser Thr Ser Gly Gly Thr Ala Ala Leu Gly
            130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
            195                 200                 205

Asn Thr Lys Val Asp Lys Arg Val Glu Pro Lys Ser Cys Asp Lys Thr
210                 215                 220

His Thr Cys Pro Pro Cys Pro Ala Pro Glu Leu Leu Gly Gly Pro Ser
225                 230                 235                 240

```
Val Phe Leu Phe Pro Pro Lys Pro Lys Asp Thr Leu Met Ile Ser Arg
            245             250                 255

Thr Pro Glu Val Thr Cys Val Val Val Asp Val Ser His Glu Asp Pro
            260             265                 270

Glu Val Lys Phe Asn Trp Tyr Val Asp Gly Val Glu Val His Asn Ala
            275             280                 285

Lys Thr Lys Pro Arg Glu Glu Gln Tyr Asn Ser Thr Tyr Arg Val Val
    290             295                 300

Ser Val Leu Thr Val Leu His Gln Asp Trp Leu Asn Gly Lys Glu Tyr
305             310                 315                 320

Lys Cys Lys Val Ser Asn Lys Ala Leu Pro Ala Pro Ile Glu Lys Thr
            325             330                 335

Ile Ser Lys Ala Lys Gly Gln Pro Arg Glu Pro Gln Val Tyr Thr Leu
            340             345                 350

Pro Pro Ser Arg Glu Glu Met Thr Lys Asn Gln Val Ser Leu Thr Cys
            355             360                 365

Leu Val Lys Gly Phe Tyr Pro Ser Asp Ile Ala Val Glu Trp Glu Ser
    370             375                 380

Asn Gly Gln Pro Glu Asn Asn Tyr Lys Thr Thr Pro Pro Val Leu Asp
385             390                 395                 400

Ser Asp Gly Ser Phe Phe Leu Tyr Ser Lys Leu Thr Val Asp Lys Ser
            405             410                 415

Arg Trp Gln Gln Gly Asn Val Phe Ser Cys Ser Val Met His Glu Ala
            420             425                 430

Leu His Asn His Tyr Thr Gln Lys Ser Leu Ser Leu Ser Lys
            435             440                 445
```

```
<210>  80
<211>  448
<212>  PRT
<213>  Artificial Sequence

<220>
<223>  Antibody heavy chain

<400>  80
```

```
Glu Val Gln Leu Val Glu Ser Gly Gly Gly Leu Val Gln Pro Gly Gly
```

```
      1                 5                         10                              15

      Ser Leu Arg Leu Ser Cys Ala Ala Ser Gly Phe Asn Ile Lys Asp Thr
              20              25                      30

      Tyr Ile His Trp Val Arg Gln Ala Pro Gly Lys Gly Leu Glu Trp Val
              35              40                      45

      Ala Arg Ile Tyr Pro Thr Asn Gly Tyr Thr Arg Tyr Ala Asp Ser Val
          50              55                      60

      Lys Gly Arg Phe Thr Ile Ser Ala Asp Thr Ser Lys Asn Thr Ala Tyr
      65              70                      75                      80

      Leu Gln Met Asn Ser Leu Arg Ala Glu Asp Thr Ala Val Tyr Tyr Cys
                  85                      90                      95

      Ser Arg Trp Gly Gly Asp Gly Phe Tyr Ala Met Asp Tyr Trp Gly Gln
                  100                     105                     110

      Gly Thr Leu Val Thr Val Ser Ser Ala Ser Thr Lys Gly Pro Ser Val
              115                     120                     125

      Phe Pro Leu Ala Pro Ser Ser Lys Ser Thr Ser Gly Gly Thr Ala Ala
          130                     135                     140

      Leu Gly Cys Leu Val Lys Asp Tyr Phe Pro Glu Pro Val Thr Val Ser
      145                     150                     155                     160

      Trp Asn Ser Gly Ala Leu Thr Ser Gly Val His Thr Phe Pro Ala Val
                  165                     170                     175

      Leu Gln Ser Ser Gly Leu Tyr Ser Leu Ser Ser Val Val Thr Val Pro
                  180                     185                     190

      Ser Ser Ser Leu Gly Thr Gln Thr Tyr Ile Cys Asn Val Asn His Lys
              195                     200                     205

      Pro Ser Asn Thr Lys Val Asp Lys Arg Val Glu Pro Lys Ser Cys Asp
          210                     215                     220

      Lys Thr His Thr Cys Pro Pro Cys Pro Ala Pro Glu Leu Leu Gly Gly
      225                     230                     235                     240

      Pro Ser Val Phe Leu Phe Pro Pro Lys Pro Lys Asp Thr Leu Met Ile
                  245                     250                     255
```

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Lys
435 440 445

<210> 81
<211> 214
<212> PRT
<213> Artificial Sequence

<220>
<223> Antibody light chain

<400> 81

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala

```
                    20                        25                          30

        Val Ala Trp Tyr Gln Gln Lys Pro Gly Lys Ala Pro Lys Leu Leu Ile
                35                    40                    45

        Tyr Ser Ala Ser Phe Leu Tyr Ser Gly Val Pro Ser Arg Phe Ser Gly
                50                    55                    60

        Ser Arg Ser Gly Thr Asp Phe Thr Leu Thr Ile Ser Ser Leu Gln Pro
        65                    70                    75                    80

        Glu Asp Phe Ala Thr Tyr Tyr Cys Gln Gln His Tyr Thr Thr Pro Pro
                        85                    90                    95

        Thr Phe Gly Gln Gly Thr Lys Val Glu Ile Lys Arg Thr Val Ala Ala
                    100                   105                   110

        Pro Ser Val Phe Ile Phe Pro Pro Ser Asp Glu Gln Leu Lys Ser Gly
                    115                   120                   125

        Thr Ala Ser Val Val Cys Leu Leu Asn Asn Phe Tyr Pro Arg Glu Ala
                130                   135                   140

        Lys Val Gln Trp Lys Val Asp Asn Ala Leu Gln Ser Gly Asn Ser Gln
        145                   150                   155                   160

        Glu Ser Val Thr Glu Gln Asp Ser Lys Asp Ser Thr Tyr Ser Leu Ser
                    165                   170                   175

        Ser Thr Leu Thr Leu Ser Lys Ala Asp Tyr Glu Lys His Lys Val Tyr
                    180                   185                   190

        Ala Cys Glu Val Thr His Gln Gly Leu Ser Ser Pro Val Thr Lys Ser
                195                   200                   205

        Phe Asn Arg Gly Glu Cys
                210

        <210>   82
        <211>   5
        <212>   PRT
        <213>   Artificial Sequence

        <220>
        <223>   linker

        <400>   82

        Gly Gly Gly Gly Ser
        1                 5
```

<210> 83
<211> 10
<212> PRT
<213> Artificial Sequence

<220>
<223> linker

<400> 83

Gly Gly Gly Gly Ser Gly Gly Gly Gly Ser
1               5                   10

<210> 84
<211> 15
<212> PRT
<213> Artificial Sequence

<220>
<223> linker

<400> 84

Gly Gly Gly Gly Ser Gly Gly Gly Gly Ser Gly Gly Gly Gly Ser
1               5                   10                  15

<210> 85
<211> 20
<212> PRT
<213> Artificial Sequence

<220>
<223> linker

<400> 85

Gly Gly Gly Gly Ser Gly Gly Gly Gly Ser Gly Gly Gly Gly Ser Gly
1               5                   10                  15

Gly Gly Gly Ser
            20

<210> 86
<211> 25
<212> PRT
<213> Artificial Sequence

<220>
<223> linker

<400> 86

Gly Gly Gly Gly Ser Gly Gly Gly Gly Ser Gly Gly Gly Gly Ser Gly
1               5                   10                  15

Gly Gly Gly Ser Gly Gly Gly Gly Ser

20                          25

<210> 87
<211> 7
<212> PRT
<213> Artificial Sequence

<220>
<223> linker

<400> 87

Gly Ala Ala Gly Ala Ala Gly
1                   5


<210> 88
<211> 6
<212> PRT
<213> Artificial Sequence

<220>
<223> linker

<400> 88

Gly Gly Thr Gly Gly Thr
1                   5


<210> 89
<211> 5
<212> PRT
<213> Artificial Sequence

<220>
<223> linker

<400> 89

Ser Thr Ser Thr Ser
1                   5


<210> 90
<211> 5
<212> PRT
<213> Artificial Sequence

<220>
<223> linker

<400> 90

Pro Ala Pro Ala Pro
1                   5


<210> 91
<211> 7
<212> PRT
<213> Artificial Sequence

<220>

<220>
<223>  linker

<400>  91

Ser Ala Ala Ser Ala Ala Ser
1                   5


<210>  92
<211>  447
<212>  PRT
<213>  Artificial Sequence

<220>
<223>  Antibody light chain

<400>  92

Asp Ile Val Leu Thr Gln Ser Pro Asp Ser Leu Ala Val Ser Leu Gly
1               5                   10                  15


Glu Arg Ala Thr Ile Asn Cys Arg Ser Ser Gln Thr Leu Leu Tyr Ser
            20                  25                  30


Asn Asn Gln Lys Asn Tyr Leu Ala Trp Tyr Gln Lys Lys Pro Gly Gln
            35                  40                  45


Pro Pro Lys Leu Leu Ile Ser Trp Ala Phe Thr Arg Lys Ser Gly Val
        50                  55                  60


Pro Asp Arg Phe Ser Gly Ser Gly Ser Gly Thr Asp Phe Thr Leu Thr
65                  70                  75                  80


Ile Ser Ser Leu Gln Ala Glu Asp Val Ala Val Tyr Tyr Cys Gln Gln
                85                  90                  95


Tyr Ser Asn Tyr Pro Trp Thr Phe Gly Gln Gly Thr Lys Val Glu Ile
            100                 105                 110


Lys Arg Thr Val Ala Ala Pro Ser Val Phe Ile Phe Pro Pro Ser Asp
        115                 120                 125


Glu Gln Leu Lys Ser Gly Thr Ala Ser Val Lys Cys Leu Leu Asn Asn
        130                 135                 140


Phe Tyr Pro Arg Glu Ala Lys Val Gln Trp Lys Val Asp Asn Ala Leu
145                 150                 155                 160


Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
            195                 200                 205

Ser Pro Val Thr Lys Ser Phe Asn Arg Gly Glu Ser Asp Lys Thr His
            210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
            275                 280                 285

Thr Lys Pro Arg Glu Glu Gln Tyr Asn Ser Thr Tyr Arg Val Val Ser
            290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
            355                 360                 365

Val Lys Gly Phe Tyr Pro Ser Asp Ile Ala Val Glu Trp Glu Ser Asn
            370                 375                 380

Gly Gln Pro Glu Asn Asn Tyr Asp Thr Thr Pro Pro Val Leu Asp Ser
385                 390                 395                 400

Asp Gly Ser Phe Phe Leu Tyr Ser Asp Leu Thr Val Asp Lys Ser Arg
                405                 410                 415

Trp Gln Gln Gly Asn Val Phe Ser Cys Ser Val Met His Glu Ala Leu
            420                 425                 430

```
His Asn His Tyr Thr Gln Lys Ser Leu Ser Leu Ser Pro Gly Lys
        435                 440                 445


<210>   93
<211>   699
<212>   PRT
<213>   Artificial Sequence

<220>
<223>   Antibody heavy chain

<400>   93

Glu Val Gln Leu Val Glu Ser Gly Gly Gly Leu Val Gln Pro Gly Gly
1                   5                   10                  15


Ser Leu Arg Leu Ser Cys Ala Ala Ser Gly Phe Asn Ile Lys Asp Thr
            20                  25                  30


Tyr Ile His Trp Val Arg Gln Ala Pro Gly Lys Gly Leu Glu Trp Val
            35                  40                  45


Ala Arg Ile Tyr Pro Thr Asn Gly Tyr Thr Arg Tyr Ala Asp Ser Val
        50                  55                  60


Lys Gly Arg Phe Thr Ile Ser Ala Asp Thr Ser Lys Asn Thr Ala Tyr
65                  70                  75                  80


Leu Gln Met Asn Ser Leu Arg Ala Glu Asp Thr Ala Val Tyr Tyr Cys
                85                  90                  95


Ser Arg Trp Gly Gly Asp Gly Phe Tyr Ala Met Asp Tyr Trp Gly Gln
            100                 105                 110


Gly Thr Leu Val Thr Val Ser Ser Gly Gly Gly Gly Ser Gly Gly Gly
            115                 120                 125


Gly Ser Gly Gly Gly Gly Ser Gly Gly Gly Gly Ser Asp Ile Gln Met
        130                 135                 140


Thr Gln Ser Pro Ser Ser Leu Ser Ala Ser Val Gly Asp Arg Val Thr
145                 150                 155                 160


Ile Thr Cys Arg Ala Ser Gln Asp Val Asn Thr Ala Val Ala Trp Tyr
            165                 170                 175


Gln Gln Lys Pro Gly Lys Ala Pro Lys Leu Leu Ile Tyr Ser Ala Ser
            180                 185                 190
```

Phe Leu Tyr Ser Gly Val Pro Ser Arg Phe Ser Gly Ser Arg Ser Gly
195 200 205

Thr Asp Phe Thr Leu Thr Ile Ser Ser Leu Gln Pro Glu Asp Phe Ala
210 215 220

Thr Tyr Tyr Cys Gln Gln His Tyr Thr Thr Pro Pro Thr Phe Gly Gln
225 230 235 240

Gly Thr Lys Val Glu Ile Lys Gly Ser Gln Val Gln Leu Val Gln Ser
245 250 255

Gly Ala Glu Val Lys Lys Pro Gly Ala Ser Val Lys Val Ser Cys Lys
260 265 270

Ala Ser Gly Tyr Ser Phe Thr Gly Tyr Phe Ile Asn Trp Val Arg Glu
275 280 285

Ala Pro Gly Gln Gly Leu Glu Trp Met Gly His Ile Ser Ser Ser Tyr
290 295 300

Ala Thr Ser Thr Tyr Asn Gln Lys Phe Gln Gly Arg Val Thr Phe Thr
305 310 315 320

Val Asp Thr Ser Ser Ser Thr Ala Tyr Met Glu Leu Ser Ser Leu Arg
325 330 335

Ser Glu Asp Thr Ala Val Tyr Tyr Cys Val Arg Ser Gly Asn Tyr Glu
340 345 350

Glu Tyr Ala Met Asp Tyr Trp Gly Gln Gly Thr Leu Val Thr Val Ser
355 360 365

Ser Ala Ser Thr Lys Gly Pro Ser Val Phe Pro Leu Ala Pro Ser Ser
370 375 380

Lys Ser Thr Ser Gly Gly Thr Ala Ala Leu Gly Cys Leu Val Lys Asp
385 390 395 400

Tyr Phe Pro Glu Pro Val Thr Val Ser Trp Asn Ser Gly Ala Leu Thr
405 410 415

Ser Gly Val His Thr Phe Pro Ala Val Leu Gln Ser Ser Gly Leu Tyr
420 425 430

Ser Leu Glu Ser Val Val Thr Val Pro Ser Ser Ser Leu Gly Thr Gln
435 440 445

```
Thr Tyr Ile Cys Asn Val Asn His Lys Pro Ser Asn Thr Lys Val Asp
    450                 455                 460

Lys Arg Val Glu Pro Lys Ser Cys Asp Lys Thr His Thr Cys Pro Pro
465                 470                 475                 480

Cys Pro Ala Pro Glu Leu Leu Gly Gly Pro Ser Val Phe Leu Phe Pro
                485                 490                 495

Pro Lys Pro Lys Asp Thr Leu Met Ile Ser Arg Thr Pro Glu Val Thr
                500                 505                 510

Cys Val Val Val Asp Val Ser His Glu Asp Pro Glu Val Lys Phe Asn
                515                 520                 525

Trp Tyr Val Asp Gly Val Glu Val His Asn Ala Lys Thr Lys Pro Arg
    530                 535                 540

Glu Glu Gln Tyr Asn Ser Thr Tyr Arg Val Val Ser Val Leu Thr Val
545                 550                 555                 560

Leu His Gln Asp Trp Leu Asn Gly Lys Glu Tyr Lys Cys Lys Val Ser
                565                 570                 575

Asn Lys Ala Leu Pro Ala Pro Ile Glu Lys Thr Ile Ser Lys Ala Lys
                580                 585                 590

Gly Gln Pro Arg Glu Pro Gln Val Tyr Thr Leu Pro Pro Ser Arg Lys
                595                 600                 605

Glu Met Thr Lys Asn Gln Val Ser Leu Thr Cys Leu Val Lys Gly Phe
    610                 615                 620

Tyr Pro Ser Asp Ile Ala Val Glu Trp Glu Ser Asn Gly Gln Pro Glu
625                 630                 635                 640

Asn Asn Tyr Lys Thr Thr Pro Pro Val Leu Lys Ser Asp Gly Ser Phe
                645                 650                 655

Phe Leu Tyr Ser Lys Leu Thr Val Asp Lys Ser Arg Trp Gln Gln Gly
                660                 665                 670

Asn Val Phe Ser Cys Ser Val Met His Glu Ala Leu His Asn His Tyr
                675                 680                 685

Thr Gln Lys Ser Leu Ser Leu Ser Pro Gly Lys
```

690                              695